(19) Europäisches Patentamt
European Patent Office
Office européen des brevets

(11) EP 4 222 153 B1

(12) **EUROPEAN PATENT SPECIFICATION**

(45) Date of publication and mention of the grant of the patent:
**29.10.2025 Bulletin 2025/44**

(21) Application number: **21793858.8**

(22) Date of filing: **28.09.2021**

(51) International Patent Classification (IPC):
***C07D 487/14*** *(2006.01)* ***C09K 11/06*** *(2006.01)*
***H10K 85/60*** *(2023.01)* ***H10K 101/20*** *(2023.01)*
***H10K 101/40*** *(2023.01)* ***H10K 101/00*** *(2023.01)*

(52) Cooperative Patent Classification (CPC):
**C07D 487/14; C09K 11/06; H10K 85/657; H10K 85/6572;** C09K 2211/1007; C09K 2211/1014; C09K 2211/1029; C09K 2211/1074; H10K 50/11; H10K 2101/20; H10K 2101/40; H10K 2101/90; Y02E 10/549

(86) International application number:
**PCT/EP2021/076611**

(87) International publication number:
**WO 2022/069453 (07.04.2022 Gazette 2022/14)**

(54) **ORGANIC MOLECULES FOR OPTOELECTRONIC DEVICES**
ORGANISCHE MOLEKÜLE FÜR OPTOELEKTRONISCHE VORRICHTUNGEN
MOLÉCULES ORGANIQUES POUR DISPOSITIFS OPTOÉLECTRONIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.10.2020 EP 20199796**

(43) Date of publication of application:
**09.08.2023 Bulletin 2023/32**

(73) Proprietor: **Samsung Display Co., Ltd.**
**Yongin-si, Gyeonggi-do 17113 (KR)**

(72) Inventor: **PASHAZADEH, Ramin**
**76646 Bruchsal (DE)**

(74) Representative: **Gulde & Partner**
**Patent- und Rechtsanwaltskanzlei mbB**
**Berliner Freiheit 2**
**10785 Berlin (DE)**

(56) References cited:
**CN-A- 108 727 394 KR-A- 20180 008 283**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 222 153 B1

## Description

The invention relates to organic light-emitting molecules and their use in organic light-emitting diodes (OLEDs) and in other optoelectronic devices.

The object of the present invention is to provide molecules which are suitable for use in optoelectronic devices.

This object is achieved by the invention which provides a new class of organic molecules.

The organic molecules of the invention are purely organic molecules, i.e. they do not contain any metal ions in contrast to metal complexes known for the use in optoelectronic devices. The organic molecules of the invention, however, may include metalloids, in particular B, Si, Sn, Se, and/or Ge.

For example, CN 108727394 A describes a bipolar main body organic electroluminescent material which is applied to the field of organic electroluminescent materials and is suitable in triplet state energy level and the like, and can serve as blue and green light main body materials. KR 20180008283 A discloses compounds which are used in an organic electronic device including an organic light emitting device, thereby being capable of lowering driving voltage of an organic electronic device, improving light efficiency and improving lifespan properties of the device.

The organic molecules of the invention exhibit emission maxima in the blue, sky-blue, green or yellow spectral range. The organic molecules exhibit in particular emission maxima between 420 nm and 520 nm, preferably between 440 nm and 495 nm, more preferably between 450 nm and 470 nm or the organic molecules exhibit in particular emission maxima between 490 and 600 nm, more preferably between 510 and 560 nm, and even more preferably between 520 and 540 nm. The photoluminescence quantum yields of the organic molecules according to the invention are, in particular, 50 % or more. The use of the molecules according to the invention in an optoelectronic device, for example an organic light-emitting diode (OLED), leads to higher efficiencies or higher color purity, expressed by the full width at half maximum (FWHM) of emission, of the device. Corresponding OLEDs have a higher stability than OLEDs with known emitter materials and comparable color.

The organic light-emitting molecules according to the invention is a structure of formula Ia or Ib:

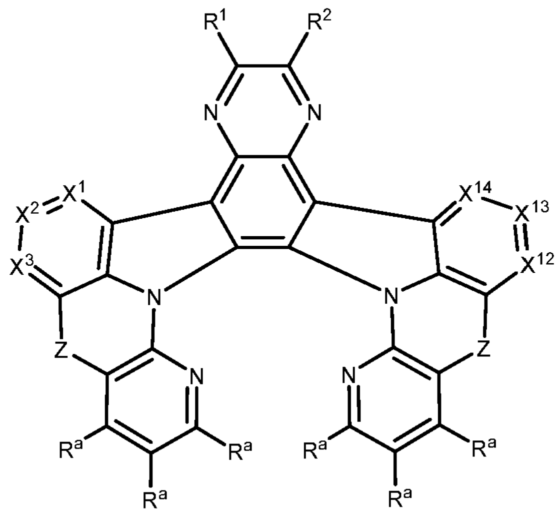

,

Formula Ia

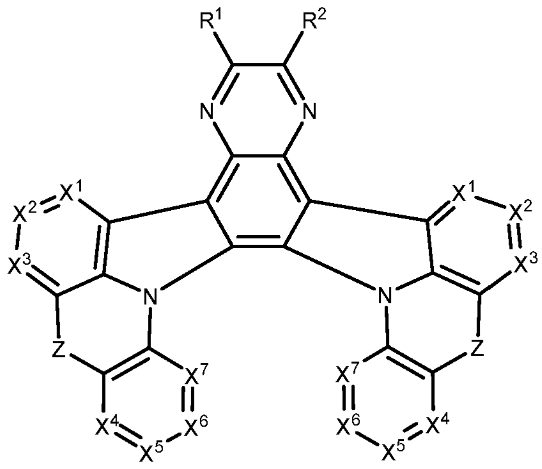

,

Formula Ib

wherein

$X^1$, $X^2$, $X^3$, $X^4$, $X^5$, $X^6$, $X^7$, $X^8$, $X^9$, $X^{10}$, $X^{11}$, $X^{12}$, $X^{13}$, and $X^{14}$ is $CR^a$;
Z is a direct bond;
$R^1$ and $R^2$ is at each occurrence independently from another selected from the group consisting of hydrogen, deuterium, $N(R^5)_2$, $OR^5$, $Si(R^5)_3$, $B(OR^5)_2$, $OSO_2R^5$, $CF_3$, CN, F, Br, I, $C_1$-$C_{40}$-alkyl,

which is optionally substituted with one or more substituents $R^5$ and
wherein one or more non-adjacent $CH_2$-groups are optionally substituted by $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-alkoxy,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)2$, C=O, C=S, C=Se, $C=NR^5$, $P(=O)(R^5)$, SO, $SO_2$, $NR^5$, O, S or $CONR^5$;

$C_1$-$C_{40}$-thioalkoxy,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)2$, C=O, C=S, C=Se, $C=NR^5$, $P(=O)(R^5)$, SO, $SO_2$, $NR^5$, O, S or $CONR^5$;

$C_2$-$C_{40}$-alkenyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)2$, C=O, C=S, C=Se, $C=NR^5$, $P(=O)(R^5)$, SO, $SO_2$, $NR^5$, O, S or $CONR^5$;

$C_2$-$C_{40}$-alkynyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)2$, C=O, C=S, C=Se, $C=NR^5$, $P(=O)(R^5)$, SO, $SO_2$, $NR^5$, O, S or $CONR^5$;

$C_6$-$C_{60}$-aryl,

which is optionally substituted with one or more substituents $R^5$; and $C_3$-$C_{57}$-heteroaryl,
which is optionally substituted with one or more substituents $R^5$; and

a mono- or polycyclic, aliphatic, aromatic and/or benzo-fused ring system formed by ring-closure with one or more of the other substituents selected from the group consisting of $R^1$, $R^2$ and $R^5$;
$R^2$ is at each occurrence independently from another selected from the group consisting of hydrogen, deuterium,
$N(R^5)2$,
$OR^5$,
$Si(R^5)_3$,
$B(OR^5)_2$,
$OSO_2R^5$,
$CF_3$,
CN,
F,
Br,
I,
$C_1$-$C_{40}$-alkyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)2$, C=O, C=S, C=Se, $C=NR^5$, $P(=O)(R^5)$, SO, $SO_2$, $NR^5$, O, S or $CONR^5$;

$C_1$-$C_{40}$-alkoxy,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)2$, C=O, C=S, C=Se, $C=NR^5$, $P(=O)(R^5)$, SO, $SO_2$, $NR^5$, O, S or $CONR^5$;

$C_1$-$C_{40}$-thioalkoxy,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)2$, C=O, C=S, C=Se, $C=NR^5$, $P(=O)(R^5)$, SO, $SO_2$, $NR^5$, O, S or $CONR^5$;

$C_2$-$C_{40}$-alkenyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)2$, C=O, C=S, C=Se, $C=NR^5$, $P(=O)(R^5)$, SO, $SO_2$, $NR^5$, O, S or $CONR^5$;

$C_2$-$C_{40}$-alkynyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)2$, C=O, C=S, C=Se, $C=NR^5$, $P(=O)(R^5)$, SO, $SO_2$, $NR^5$, O, S or $CONR^5$;

$C_6$-$C_{60}$-aryl,

which is optionally substituted with one or more substituents $R^5$; and $C_3$-$C_{57}$-heteroaryl,
which is optionally substituted with one or more substituents $R^5$;

$R^5$ is at each occurrence independently from another selected from the group consisting of hydrogen, deuterium, $N(R^6)_2$, $OR^6$, $Si(R^6)_3$, $B(OR^6)_2$, $OSO_2R^6$, $CF_3$, CN, F, Br, I,
$C_1$-$C_{40}$-alkyl,

which is optionally substituted with one or more substituents $R^6$ and
wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_1$-$C_{40}$-alkoxy,

which is optionally substituted with one or more substituents $R^6$ and
wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_1$-$C_{40}$-thioalkoxy,

which is optionally substituted with one or more substituents $R^6$ and
wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_2$-$C_{40}$-alkenyl,

which is optionally substituted with one or more substituents $R^6$ and
wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_2$-$C_{40}$-alkynyl,

which is optionally substituted with one or more substituents $R^6$ and
wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_6$-$C_{60}$-aryl,

which is optionally substituted with one or more substituents $R^6$; and $C_3$-$C_{57}$-heteroaryl,
which is optionally substituted with one or more substituents $R^6$;

$R^6$ is at each occurrence independently from another selected from the group consisting of hydrogen, deuterium, OPh (Ph = phenyl), $CF_3$, CN, F,
$C_1$-$C_5$-alkyl,
wherein one or more hydrogen atoms are optionally, independently from each other substituted by deuterium, CN, $CF_3$, or F;
$C_1$-$C_5$-alkoxy,
wherein one or more hydrogen atoms are optionally, independently from each other substituted by deuterium, CN, $CF_3$, or F;
$C_1$-$C_5$-thioalkoxy,
wherein one or more hydrogen atoms are optionally, independently from each other substituted by deuterium, CN, $CF_3$, or F;
$C_2$-$C_5$-alkenyl,
wherein one or more hydrogen atoms are optionally, independently from each other substituted by deuterium, CN, $CF_3$, or F;
$C_2$-$C_5$-alkynyl,
wherein one or more hydrogen atoms are optionally, independently from each other substituted by deuterium, CN, $CF_3$, or F;
$C_6$-$C_{18}$-aryl,

which is optionally substituted with one or more $C_1$-$C_5$-alkyl substituents; $C_3$-$C_{17}$-heteroaryl,
which is optionally substituted with one or more $C_1$-$C_5$-alkyl substituents; $N(C_6$-$C_{18}$-aryl$)_2$;

$N(C_3$-$C_{17}$-heteroaryl$)_2$,
and $N(C_3$-$C_{17}$-heteroaryl$)(C_6$-$C_{18}$-aryl);
wherein the substituents $R^1$, $R^2$, $R^a$, or $R^5$ independently from each other optionally form a mono- or polycyclic, aliphatic, aromatic and/or benzo-fused ring system with one or more substituents $R^1$, $R^2$, $R^a$, or $R^5$.

Selected examples of organic molecules according to the invention are shown in the following:

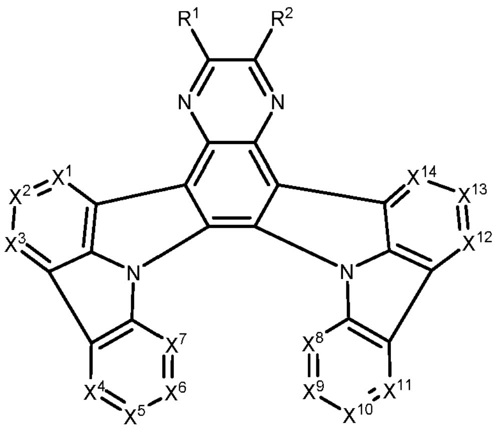

In one embodiment of the invention, the organic molecules according to the invention comprise or consist of a structure selected from the group consisting of formula IIa or formula IIb,:

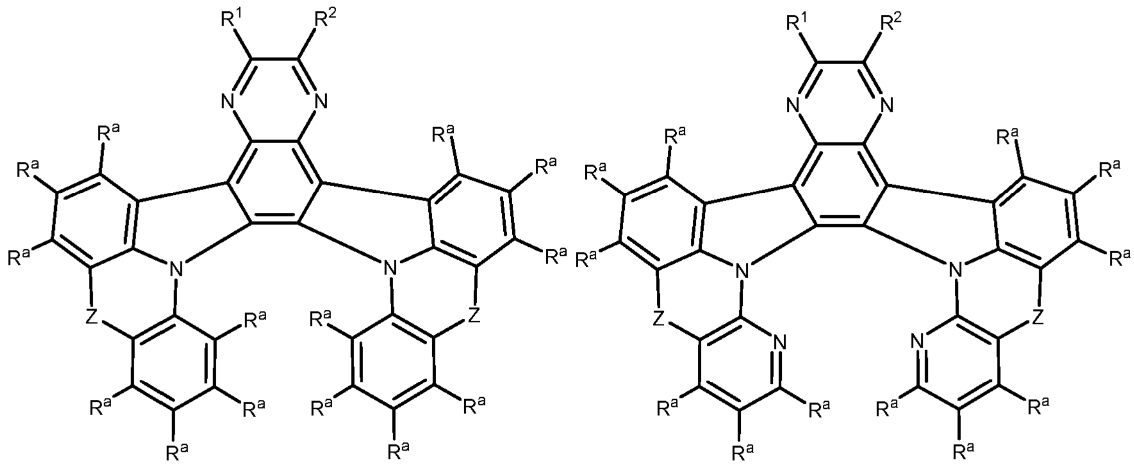

Formula IIa Formula IIb

In a preferred embodiment, the organic molecules according to the invention comprise or consist of a structure according to formula IIa.

In a preferred embodiment of the invention, the organic molecules according to the invention comprise or consist of a structure selected from the group consisting of formula IIa-1 or formula IIb-1:

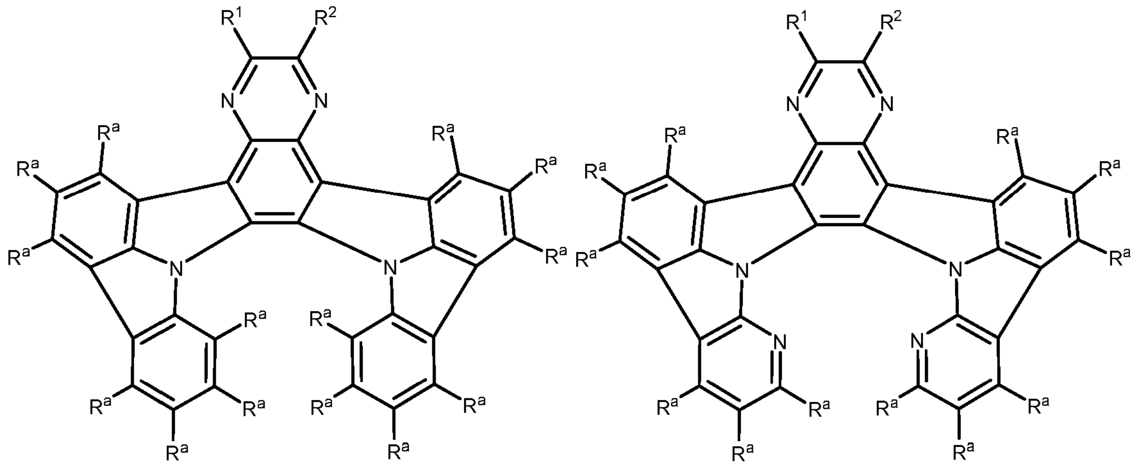

Formula IIa-1 Formula IIb-1

In one embodiment of the invention, at least one mono- or polycyclic, aliphatic, aromatic and/or benzofused ring

system is formed by $R^a$, or $R^5$ substituents together with one or more further $R^a$, or $R^5$ substituents. Examples for such structures are shown below:

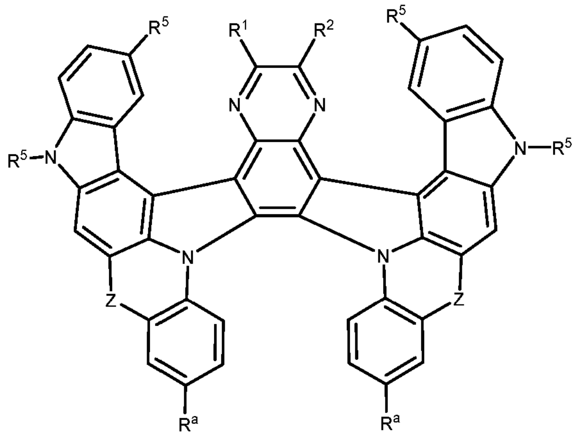
,
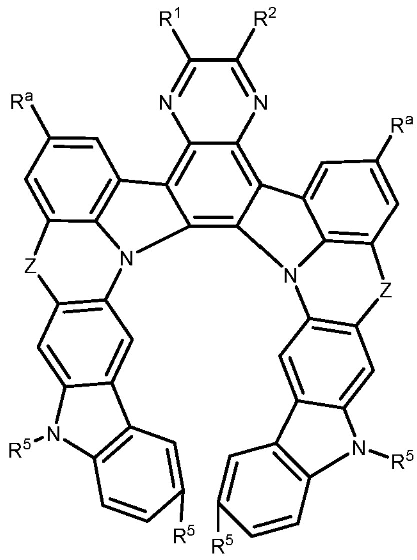
,
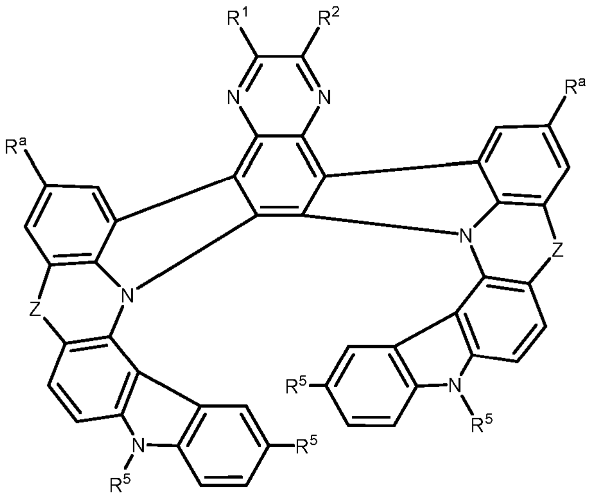
,
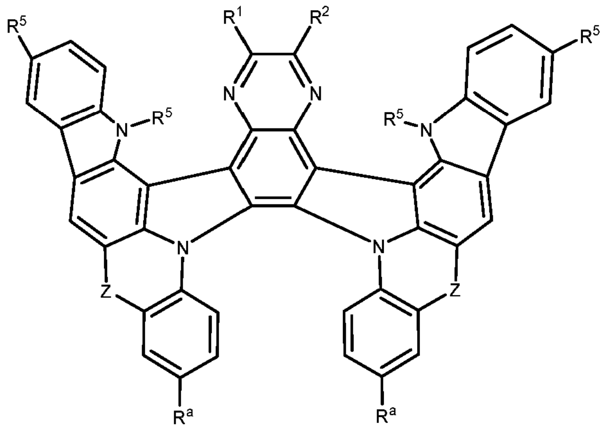
,

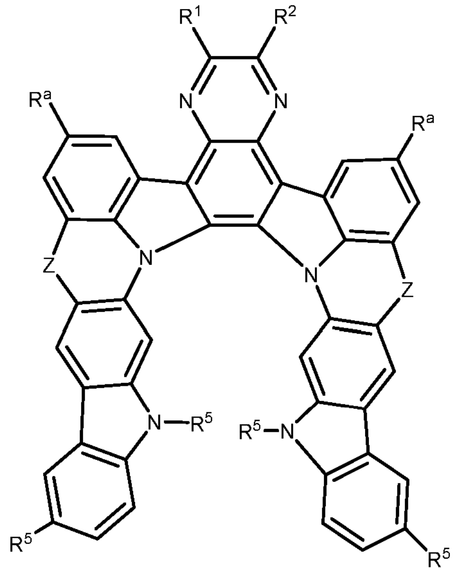,

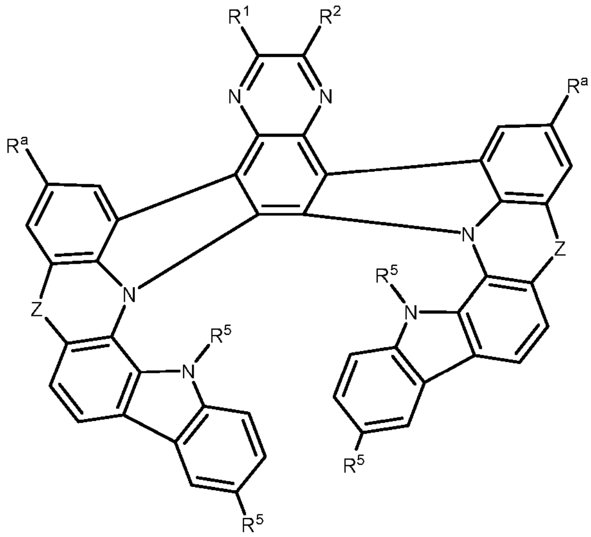,

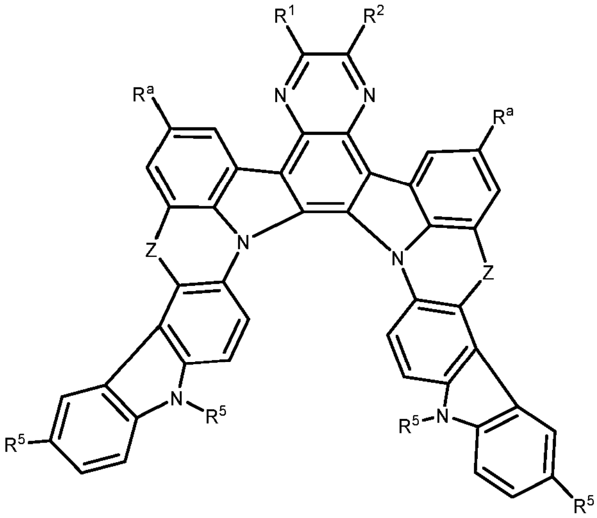,

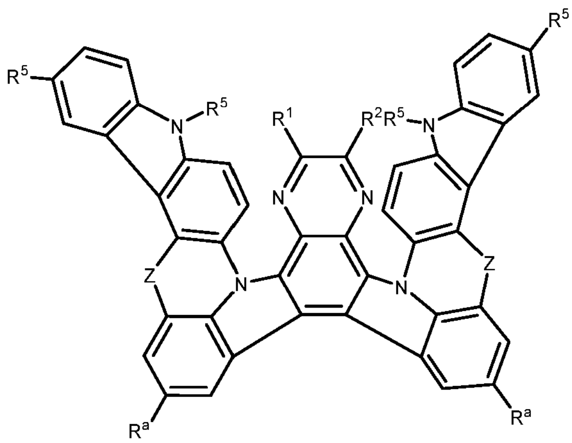,

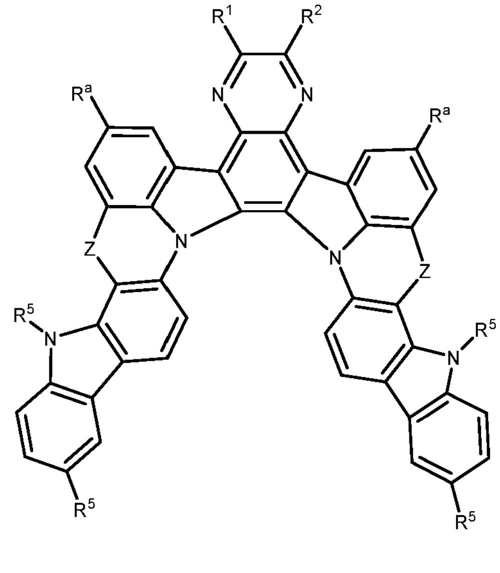,

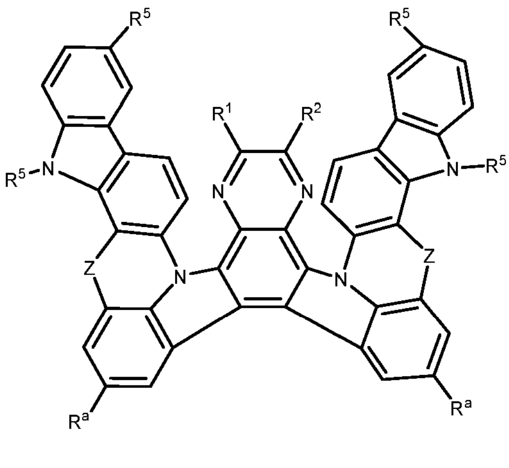,

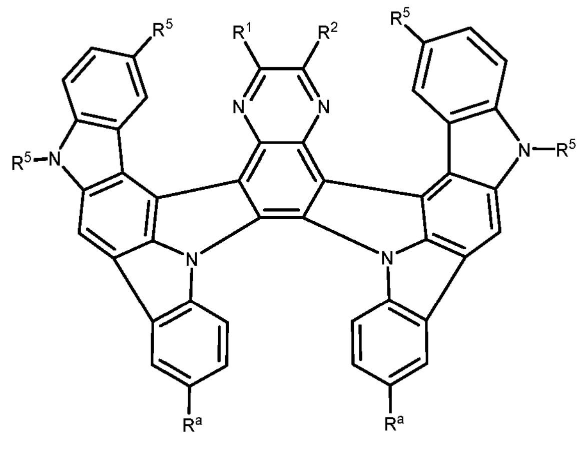
,
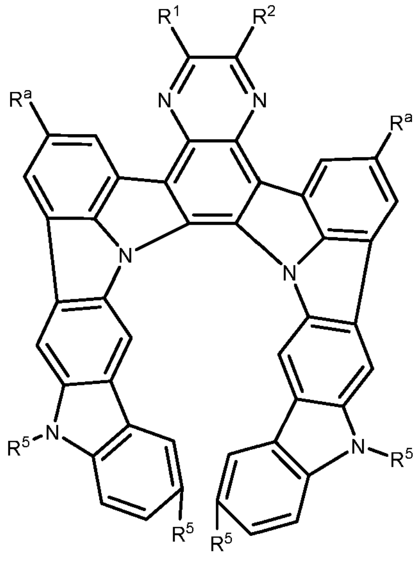
,

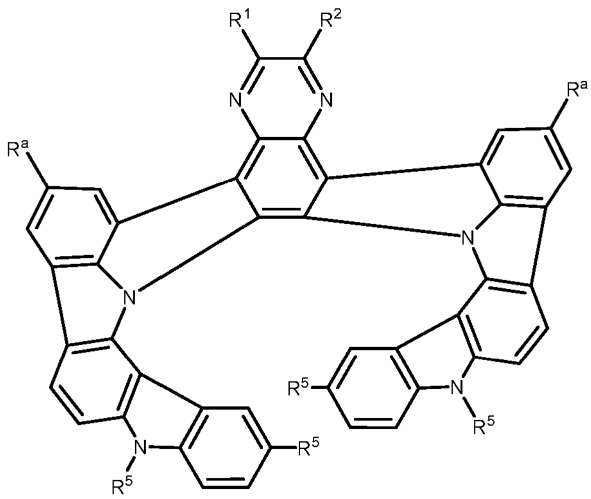
,
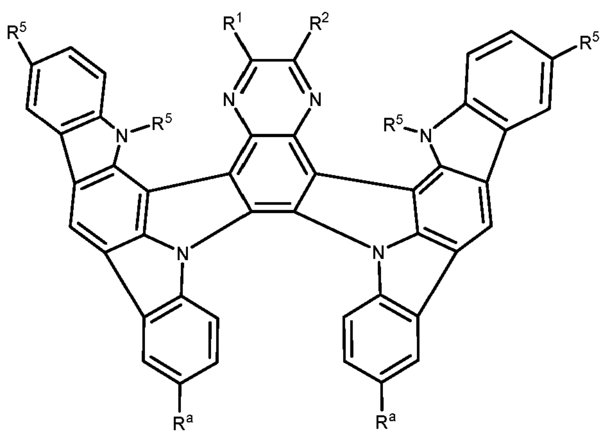
,

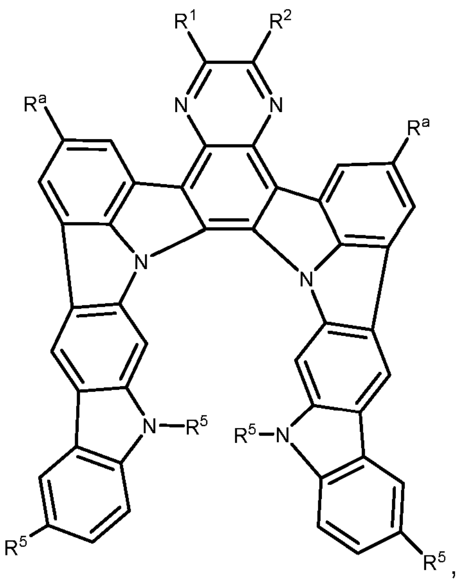
,
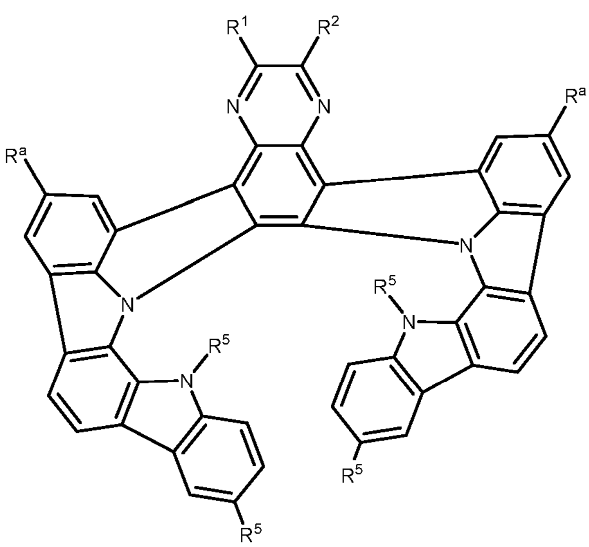
,

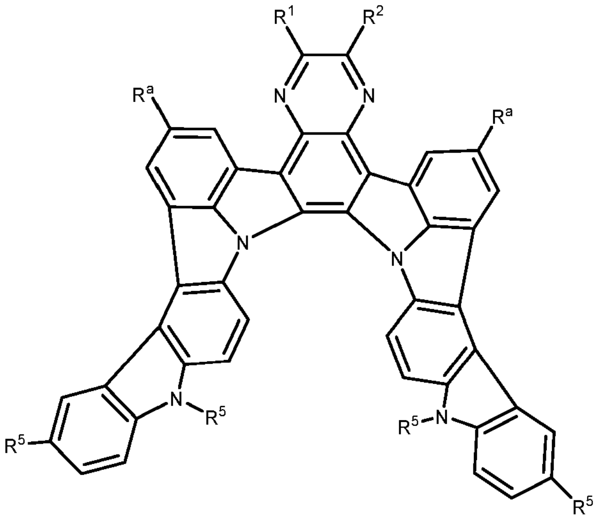
,
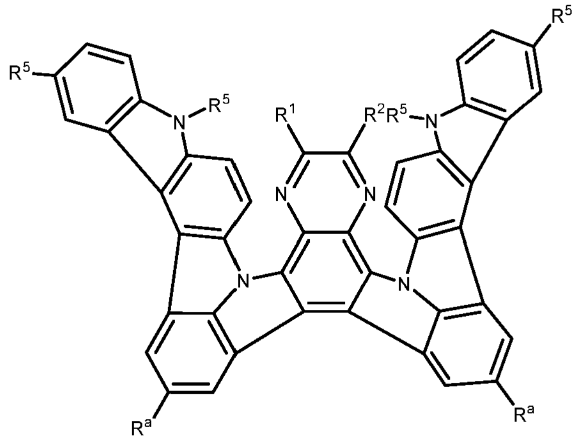
,

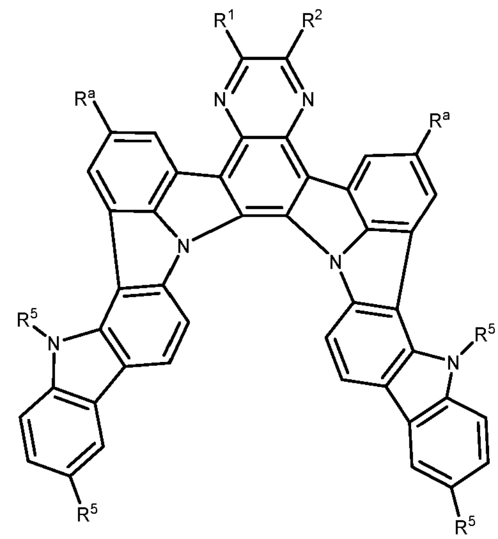
,
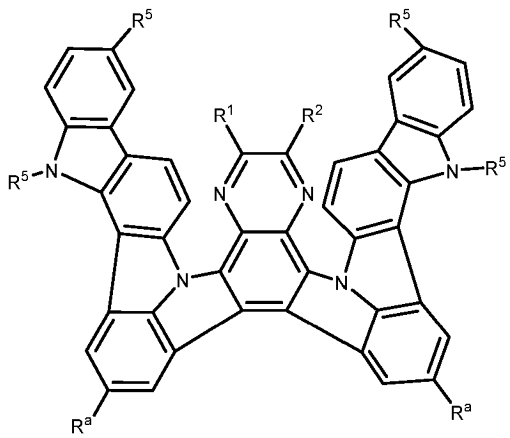
,

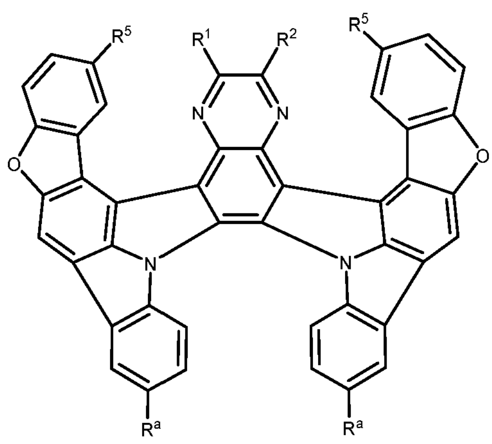
,
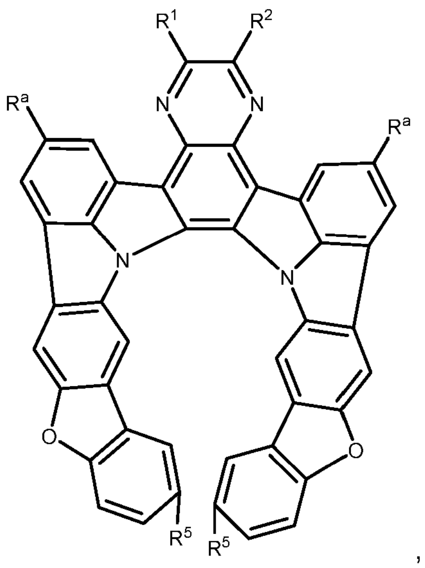
,

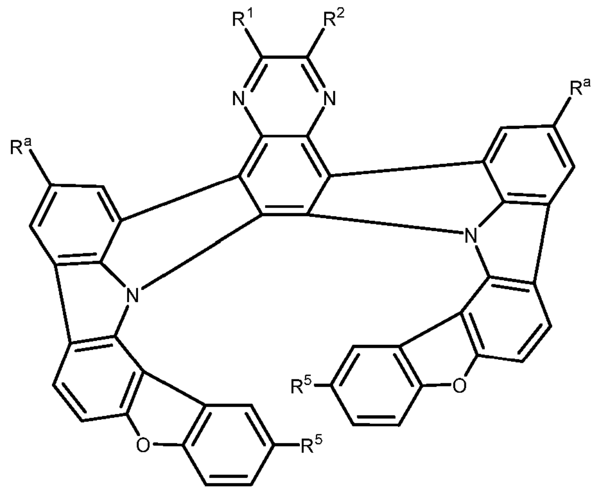
,
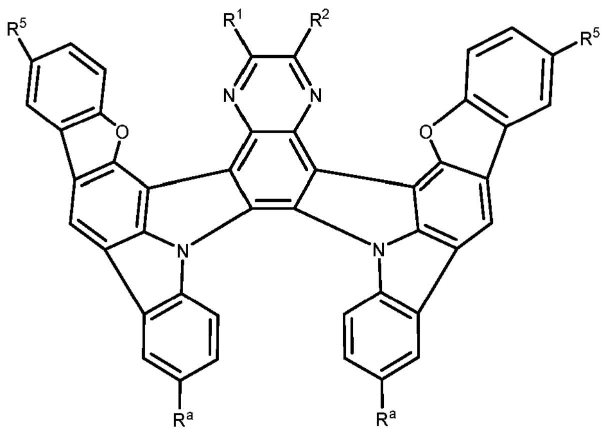
,

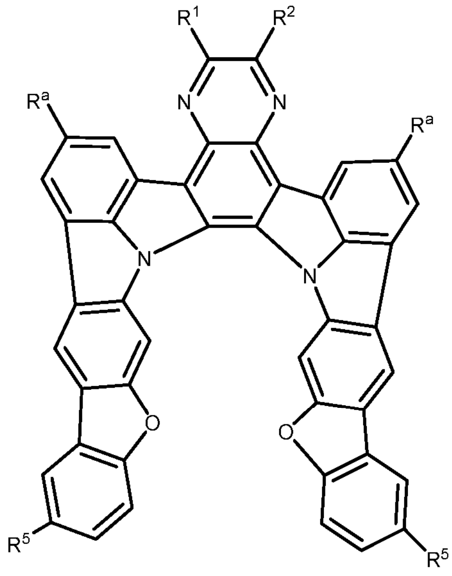
,
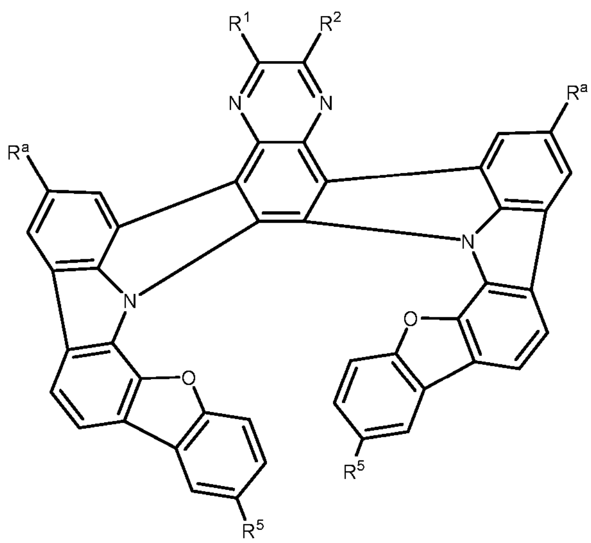
,

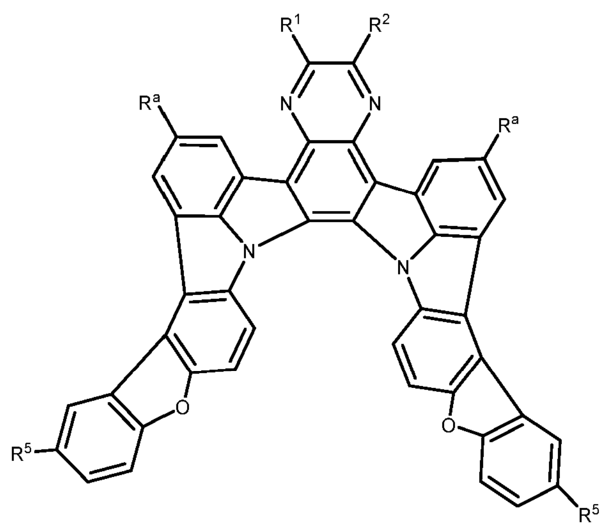
,
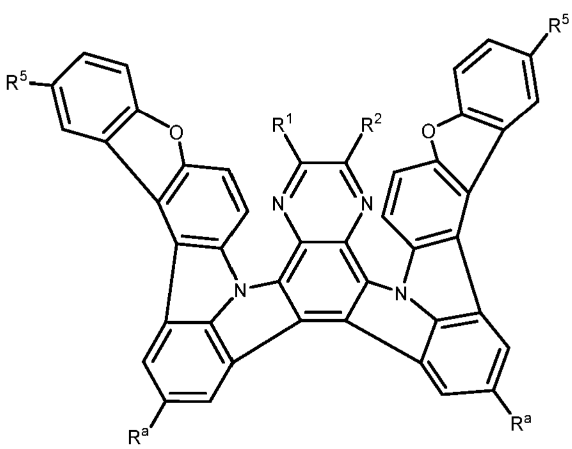
,

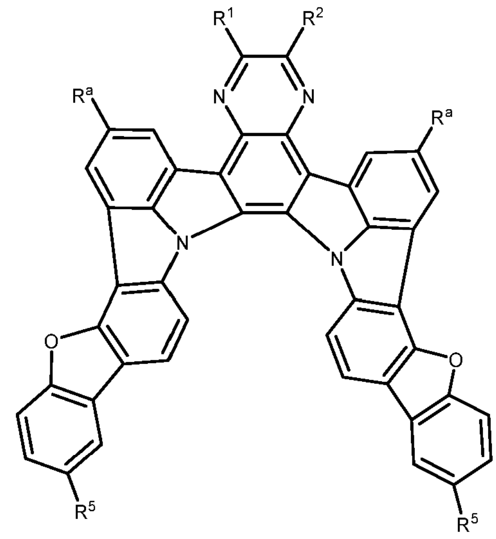
,
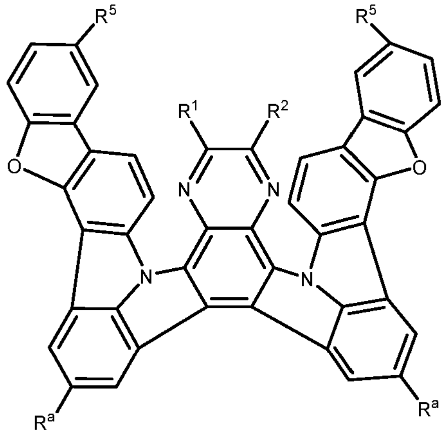
,

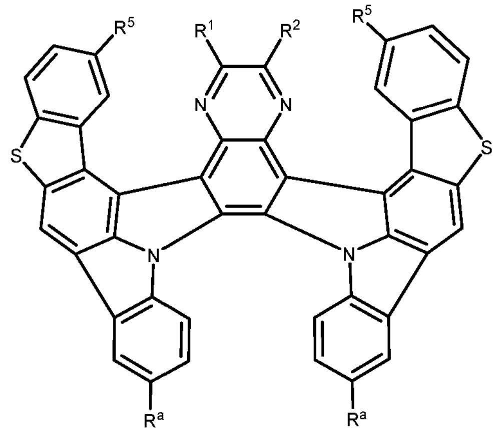
,
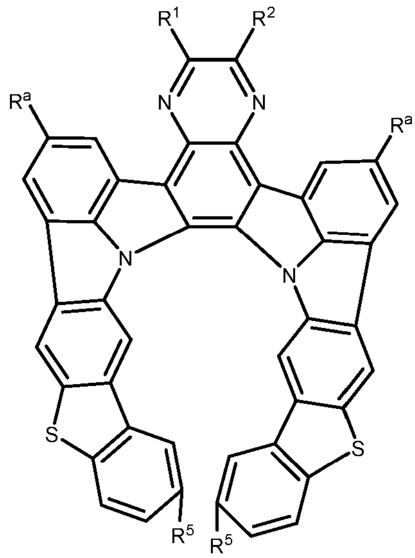
,

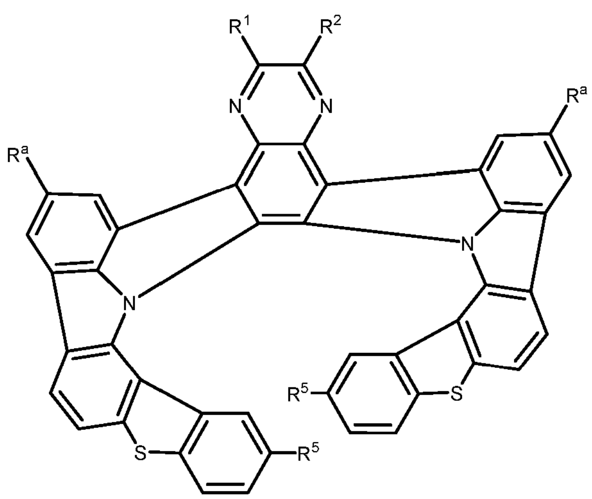
,
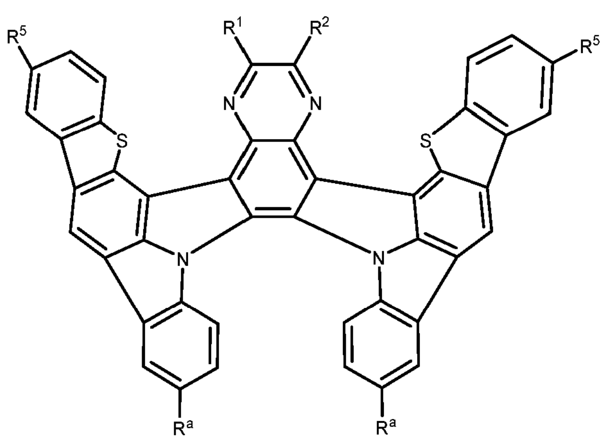
,

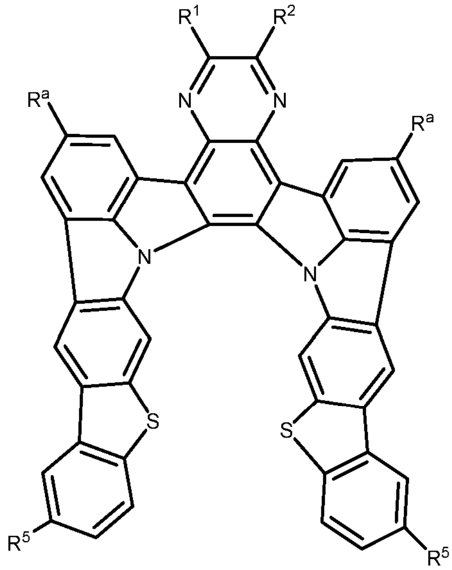
,
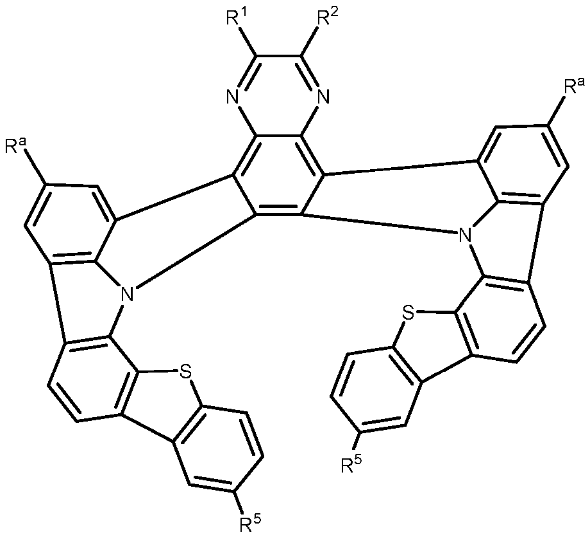
,
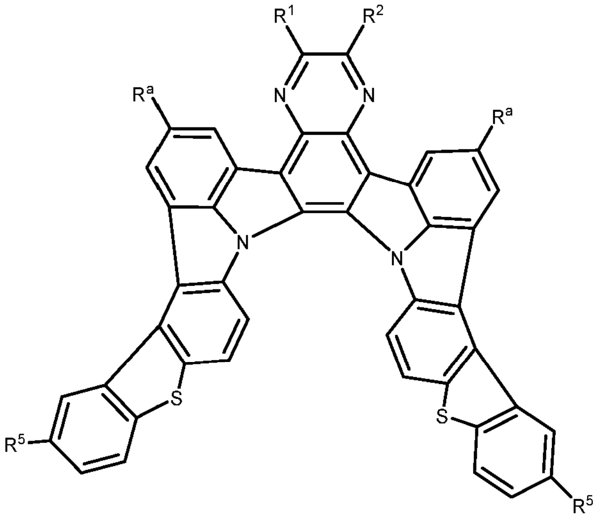
,
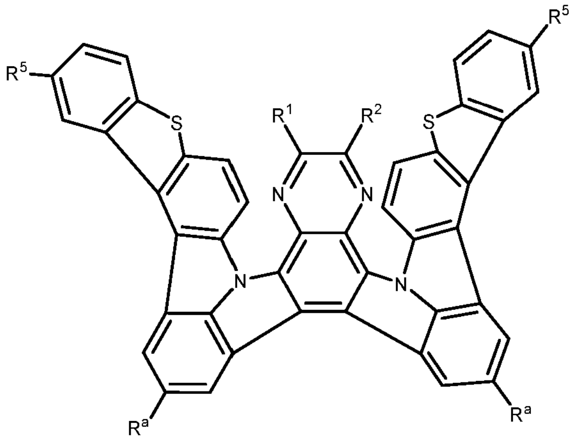
,
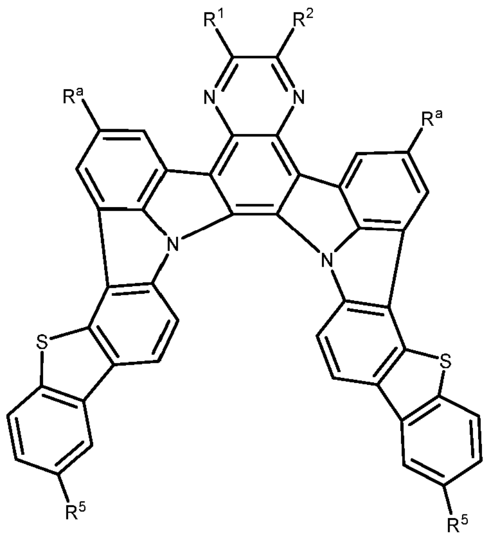
,
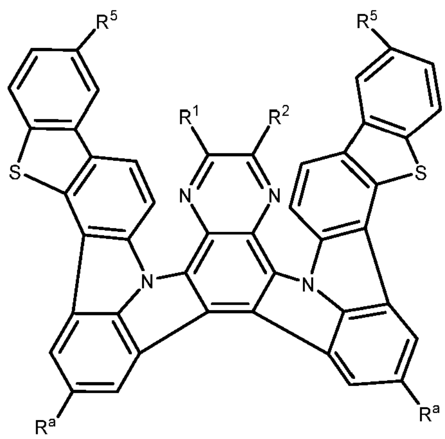
.

In a preferred embodiment of the invention, $R^1$ and $R^2$ is independently from each other selected from the group consisting of

phenyl, which is optionally substituted with one or more substituents $R^5$; and pyridine, which is optionally substituted with one or more substituents $R^5$.

In further embodiments of the invention, the organic molecules comprise or consist of a structure selected from the group consisting of formula IIIa, formula IIIb, formula IIIc, formula IIId, formula IIIe, formula IIIf, formula IIIg, formula IIIh, and formula IIIi:

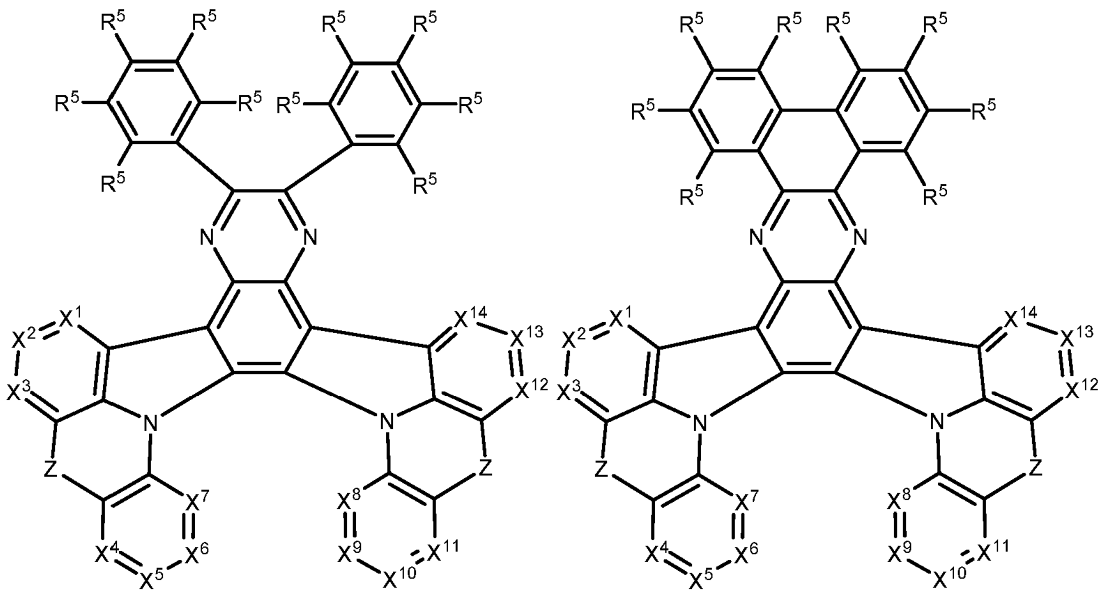

Formula IIIa

Formula IIIb

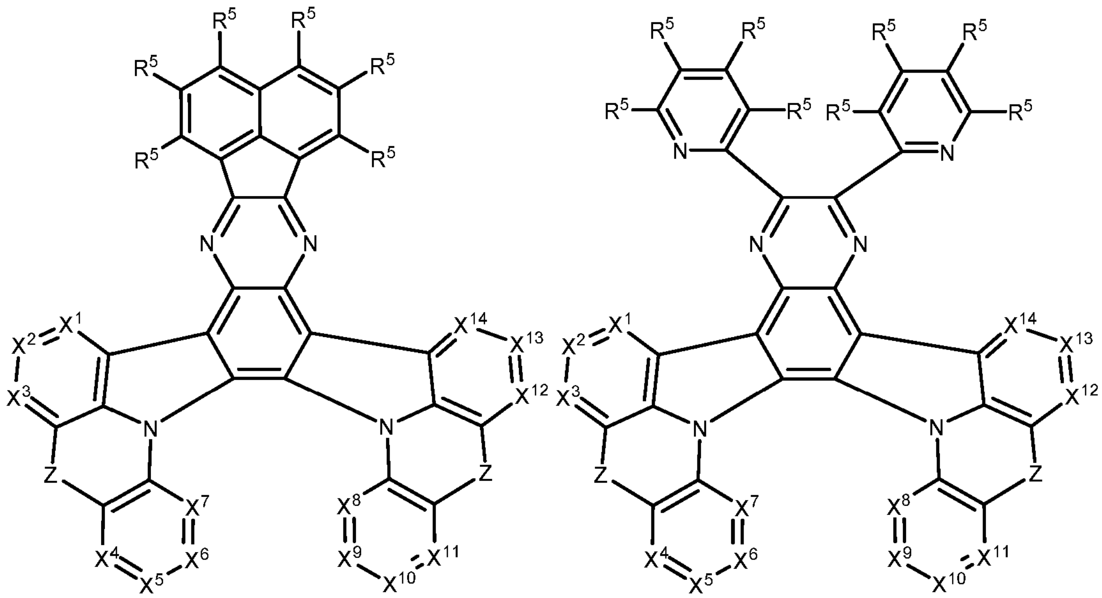

Formula IIIc

Formula IIId

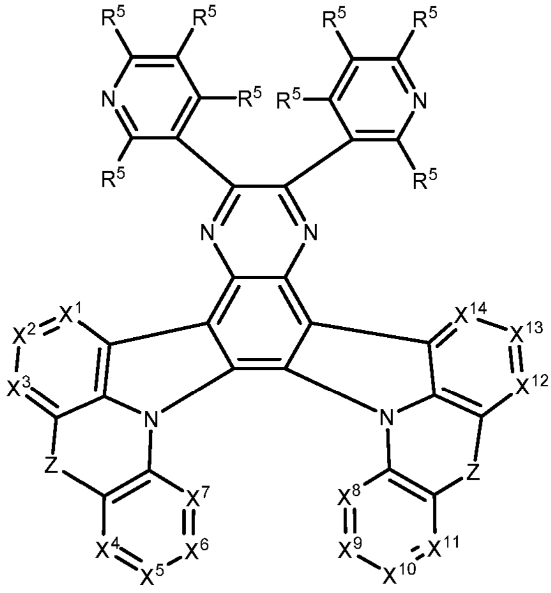

Formula IIIe

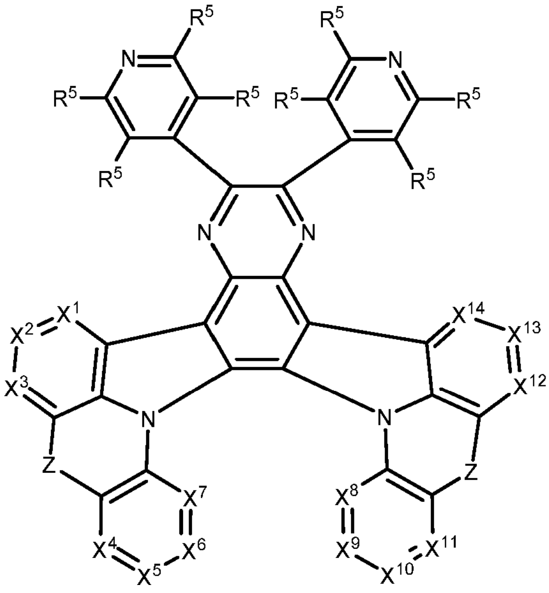

Formula IIIf

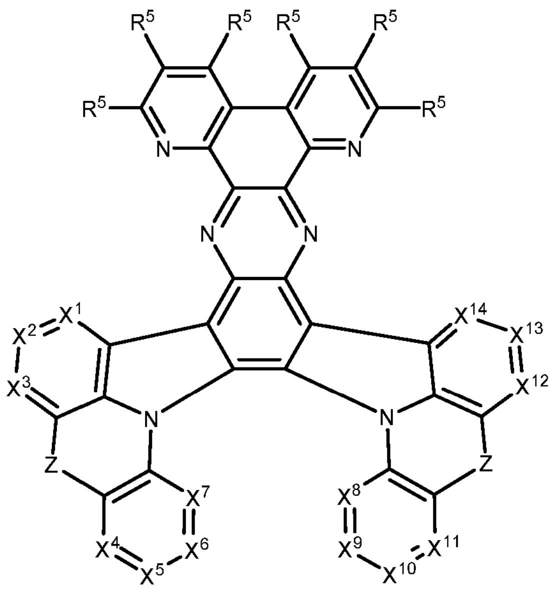

Formula IIIg

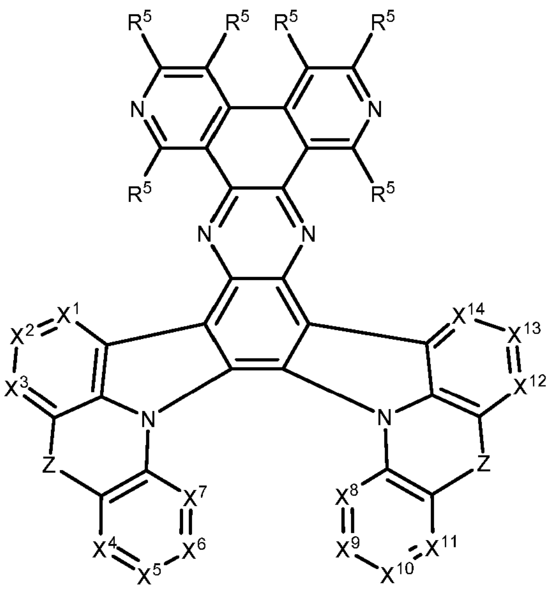

Formula IIIh

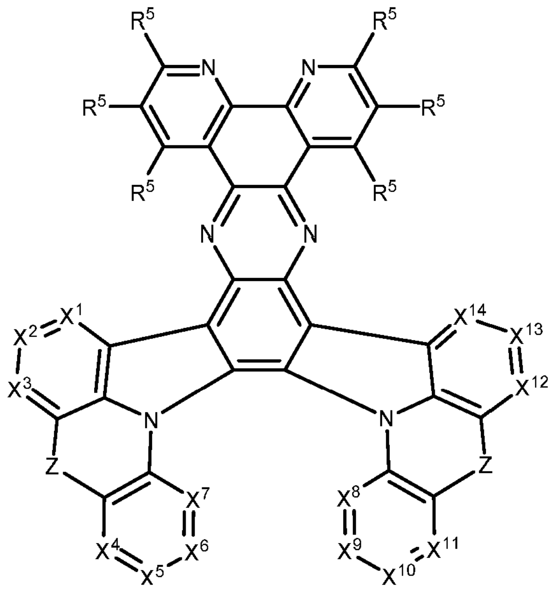

Formula IIIi

In the embodiments of the invention, Z is a direct bond at each occurrence.

In further embodiments of the invention, the organic molecules comprise or consist of a structure selected from the group consisting of formula IVa, formula IVb, formula IVc, formula IVd, formula IVe, formula IVf, formula IVg, formula IVh, and formula IVi:

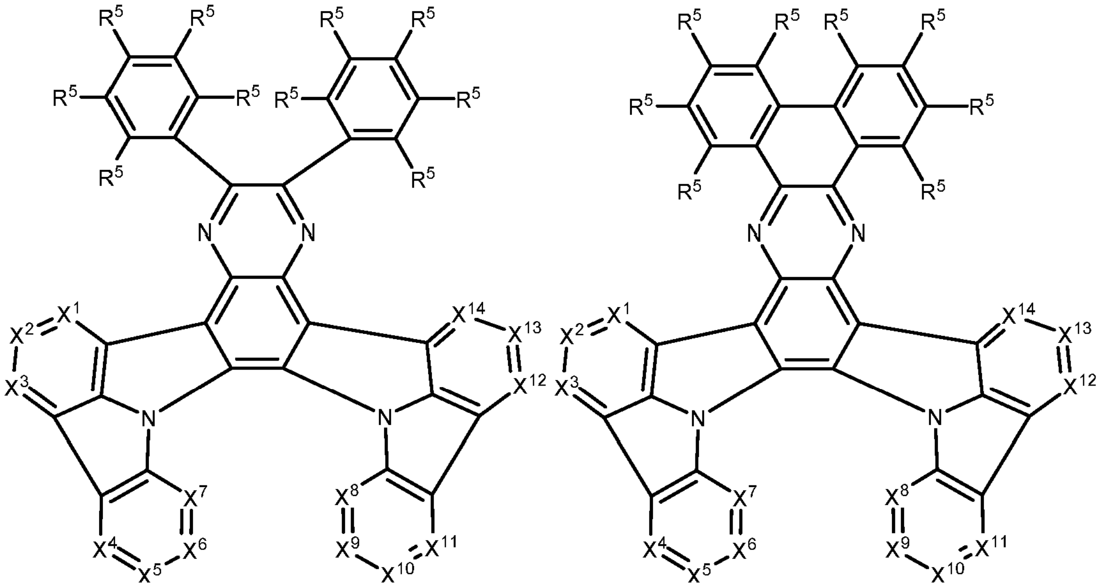

Formula IVa

Formula IVb

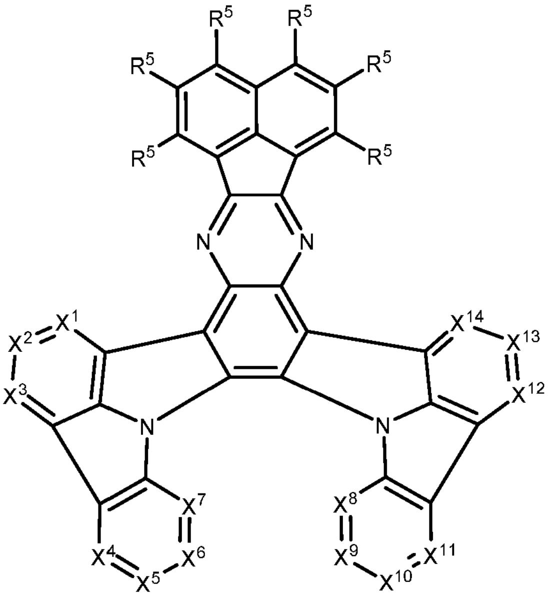

Formula IVc

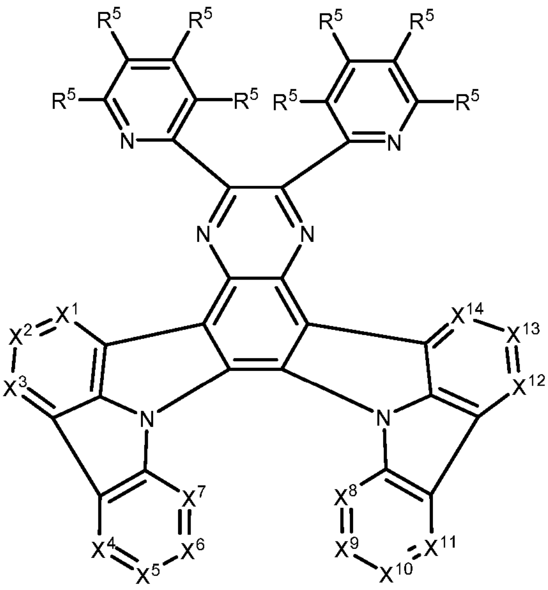

Formula IVd

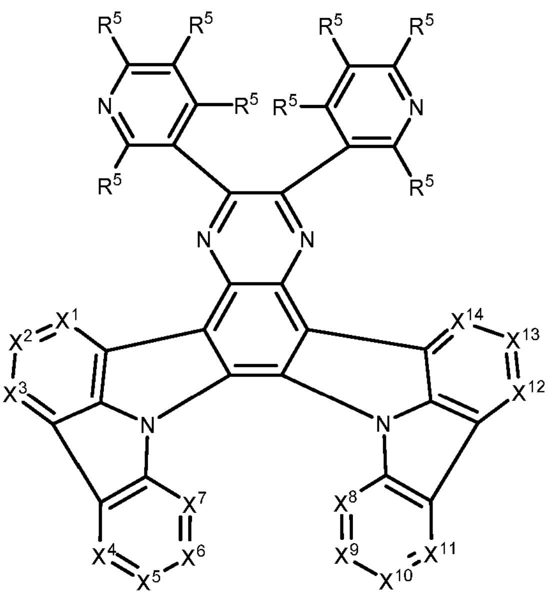

Formula IVe

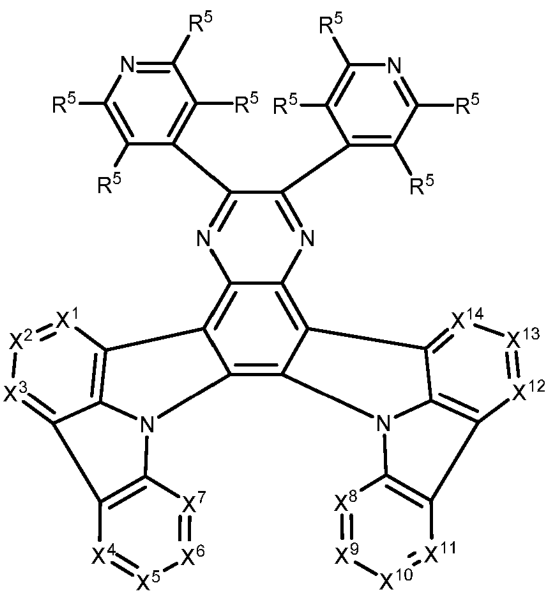

Formula IVf

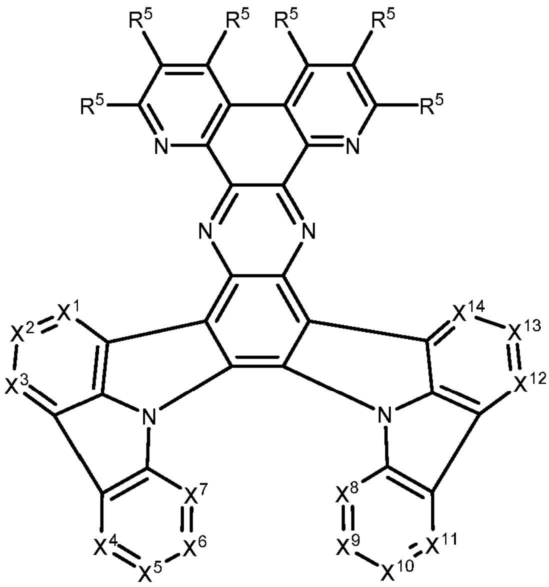

Formula IVg

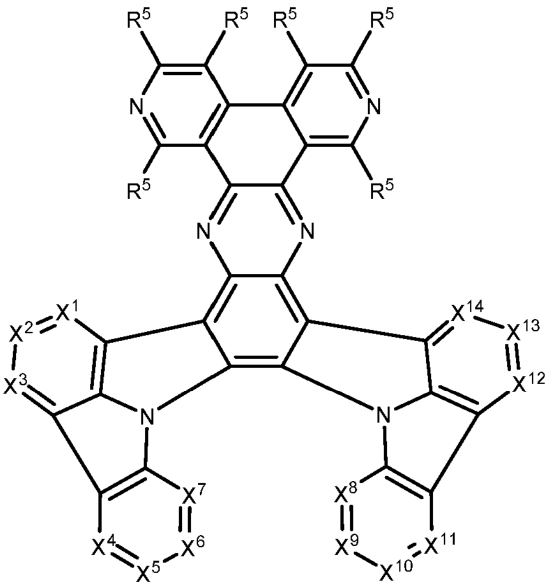

Formula IVh

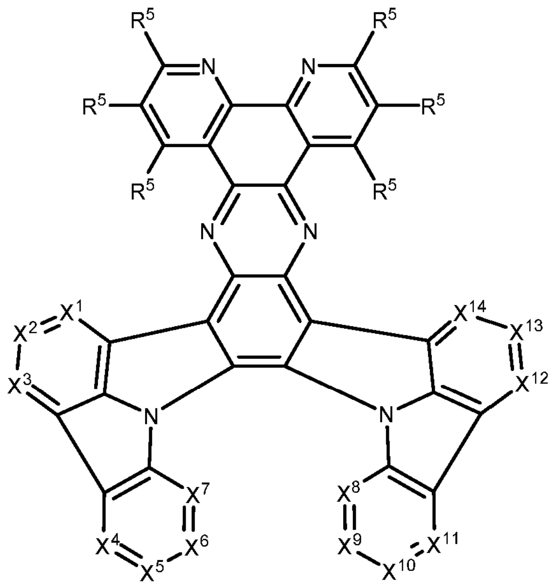

Formula IVi

In one embodiment, wherein the substituents $R^2$ and $R^1$ are the same, i.e. the substituent $R^2$ is equal to $R^1$; put differently, $R^2 = R^1$.

In one embodiment of the invention, the organic molecules comprise or consist of a structure of formula V:

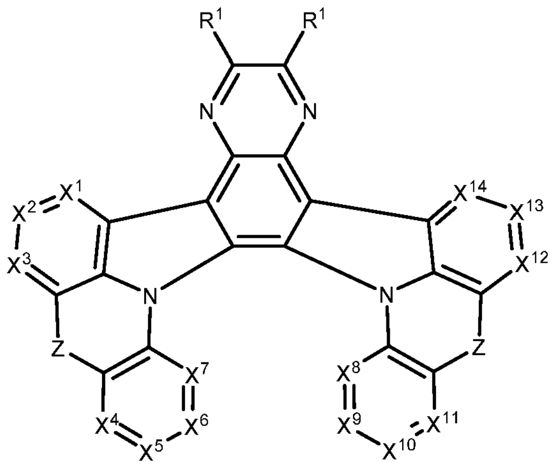

Formula V

wherein the substituents $R^1$, $R^a$, or $R^5$ independently from each other optionally form a mono- or polycyclic, aliphatic, aromatic and/or benzo-fused ring system with one or more substituents $R^1$, $R^a$, or $R^5$.

In one embodiment of the invention, the organic molecules comprise or consist of a structure of formula Va:

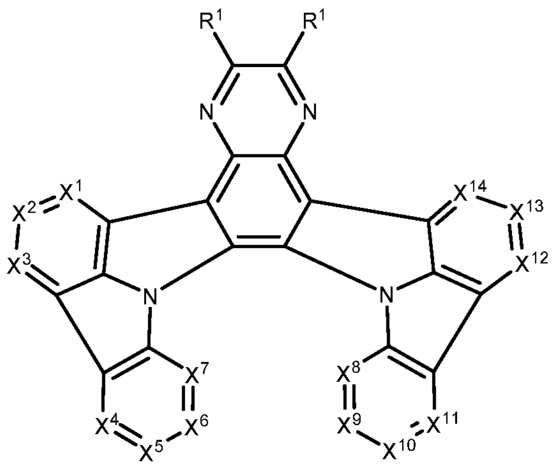

Formula Va

wherein the substituents $R^1$, $R^a$, or $R^5$ independently from each other optionally form a mono- or polycyclic, aliphatic, aromatic and/or benzo-fused ring system with one or more substituents $R^1$, $R^a$, or $R^5$.

In one embodiment of the invention, the organic molecules comprise or consist of a structure of formula Vb:

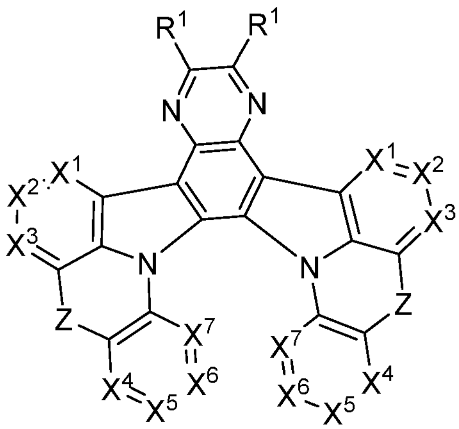

Formula Vb,

wherein

$X^1$, $X^2$, $X^3$, $X^4$, $X^5$, and $X^6$ is $CR^a$, and
the substituents $R^1$, $R^a$, or $R^5$ independently from each other optionally form a mono- or polycyclic, aliphatic, aromatic and/or benzo-fused ring system with one or more substituents $R^1$, $R^a$, or $R^5$.

In one embodiment of the invention, the organic molecules comprise or consist of a structure selected from the group consisting of formula Vb-1, formula Vb-2, formula Vb-3, formula Vb-4, formula Vb-5, formula Vb-6, formula Vb-7, formula Vb-8, and formula Vb-9:

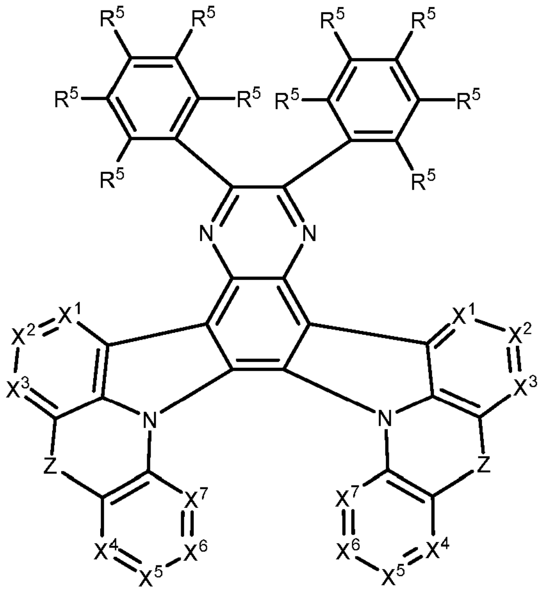

Formula Vb-1

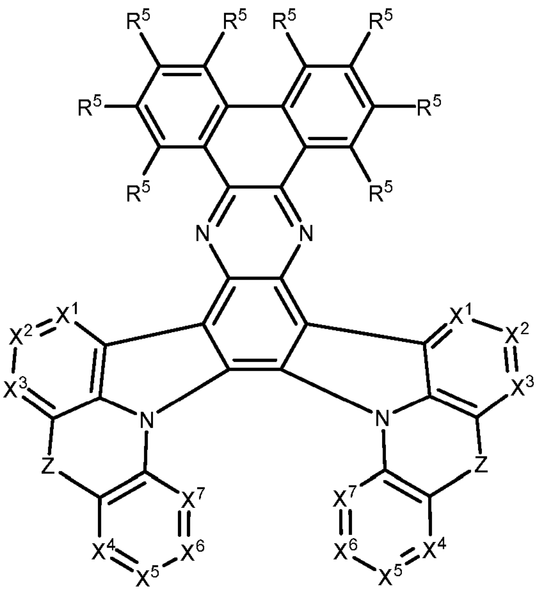

Formula Vb-2

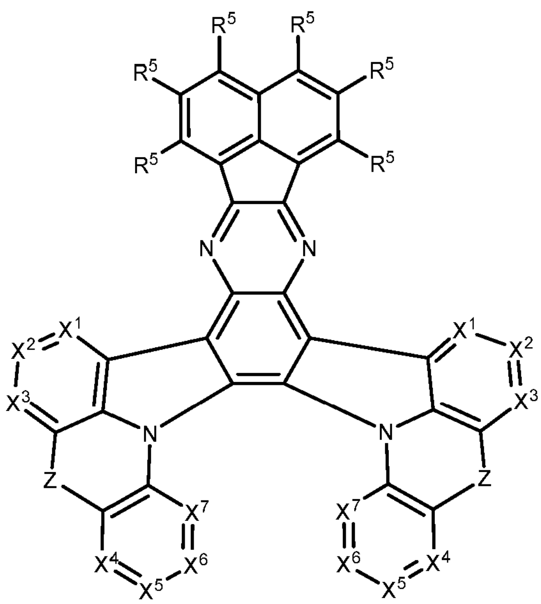

Formula Vb-3

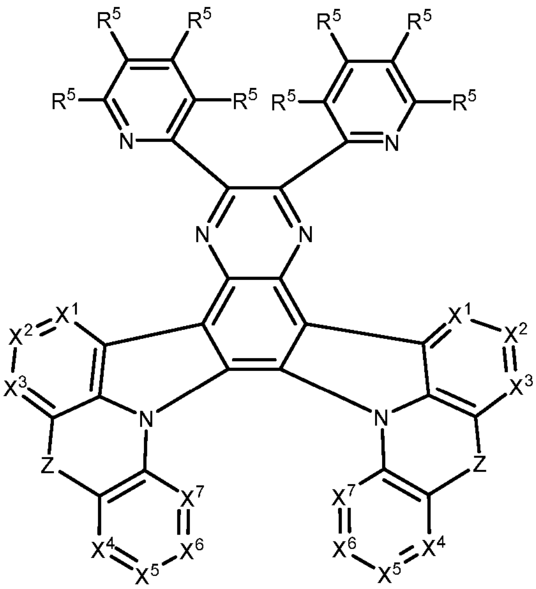

Formula Vb-4

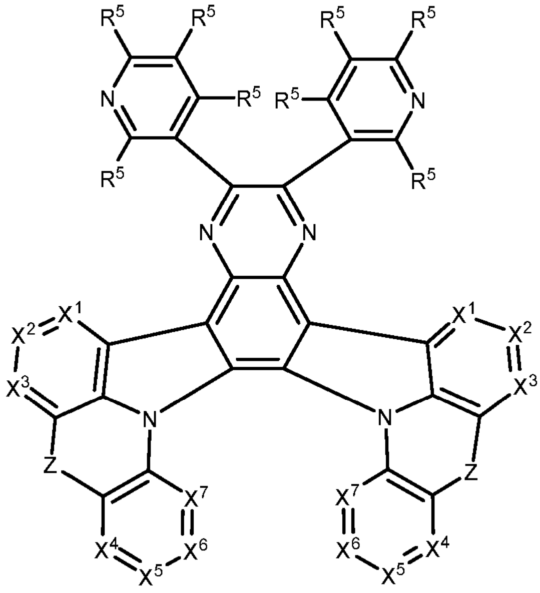

Formula Vb-5

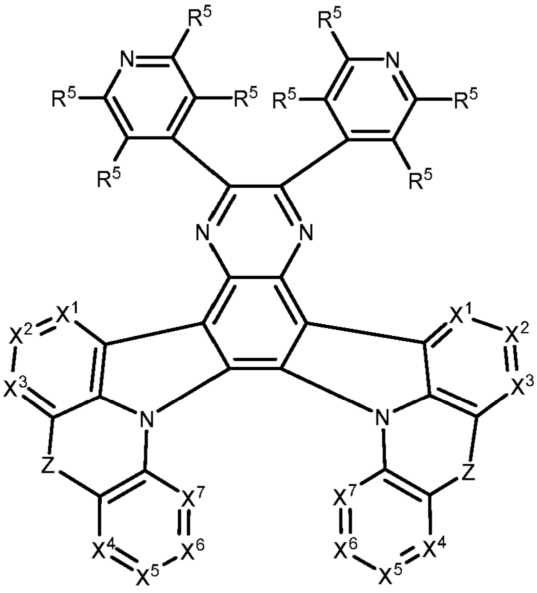

Formula Vb-6

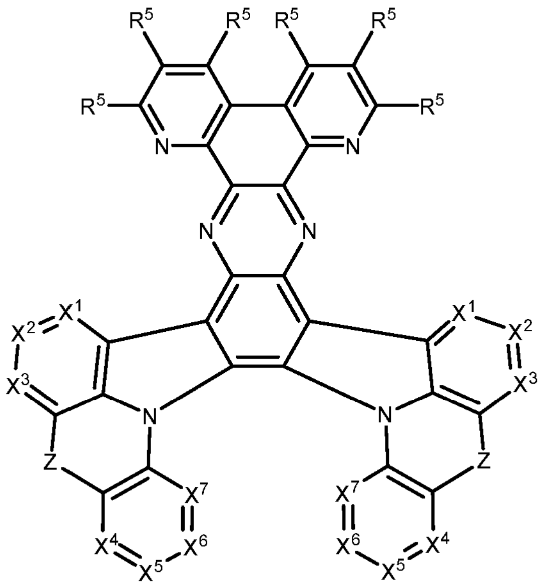

Formula Vb-7

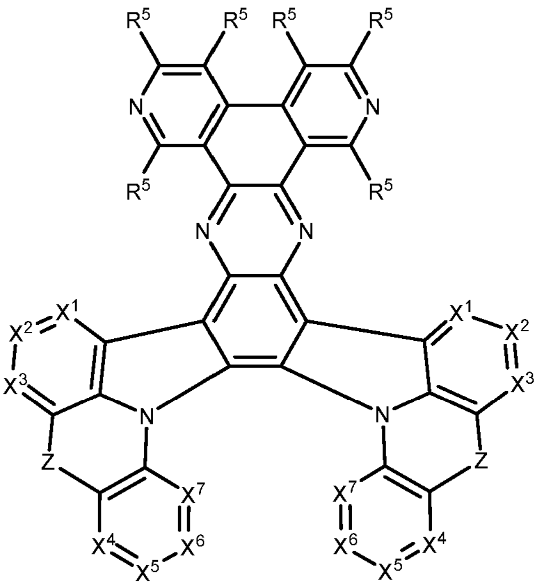

Formula Vb-8

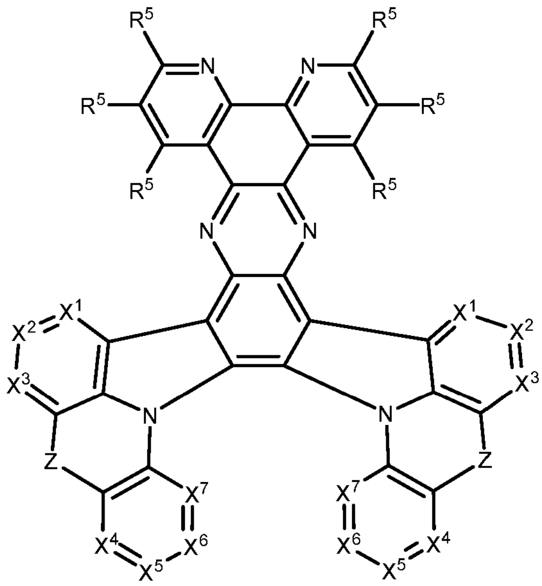

Formula Vb-9

In one embodiment of the invention, the organic molecules comprise or consist of a structure of formula Vc:

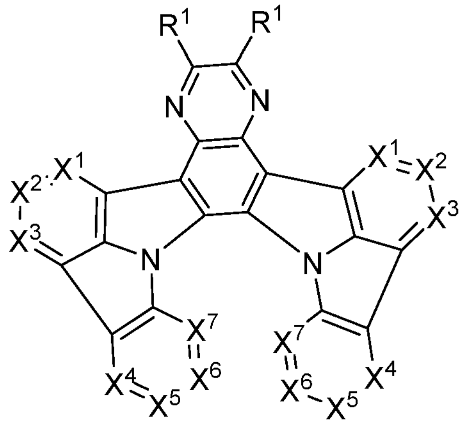

Formula Vc

wherein

$X^1$, $X^2$, $X^3$, $X^4$, $X^5$, $X^6$ and $X^7$ $CR^a$, and
the substituents $R^1$, $R^a$ or $R^5$ independently from each other optionally form a mono- or polycyclic, aliphatic, aromatic and/or benzo-fused ring system with one or more substituents $R^1$, $R^2$ or $R^5$.

In one embodiment of the invention, the organic molecules comprise or consist of a structure selected from the group consisting of formula Vc-1, formula Vc-2, formula Vc-3, formula Vc-4, formula Vc-5, formula Vc-6, formula Vc-7, formula Vc-8, and formula Vc-9:

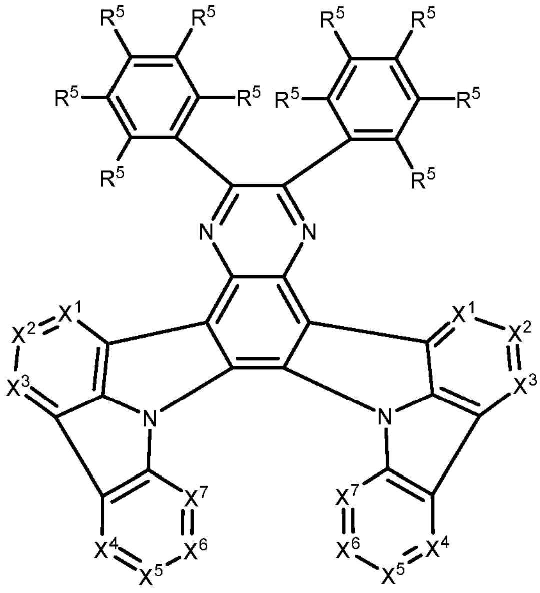

Formula Vc-1

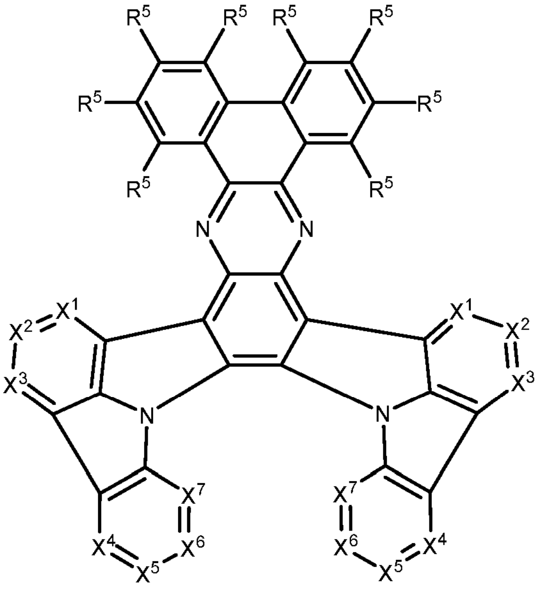

Formula Vc-2

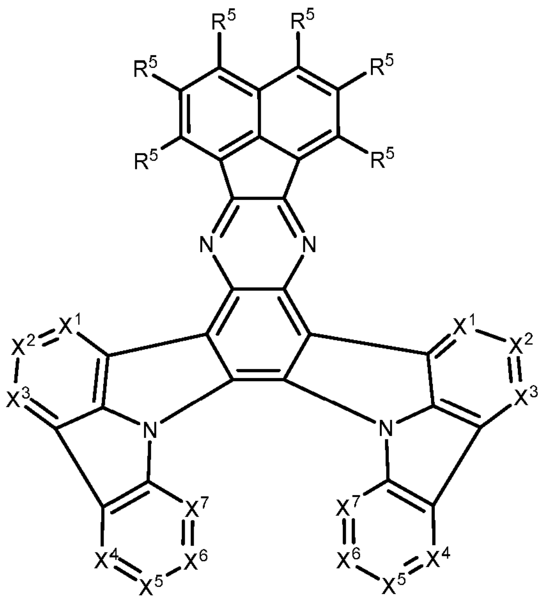

Formula Vc-3

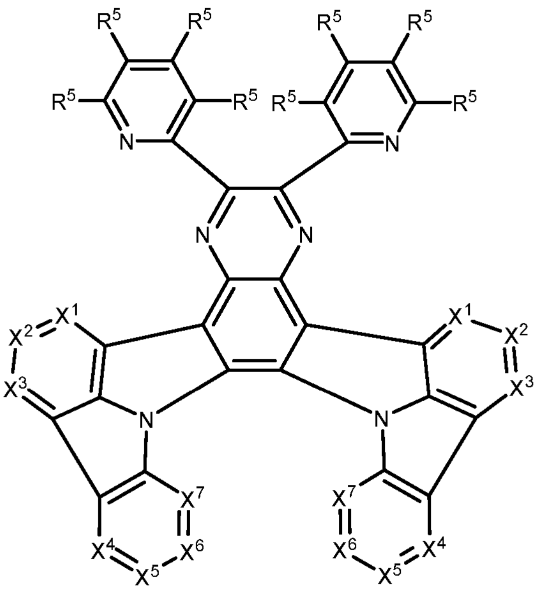

Formula Vc-4

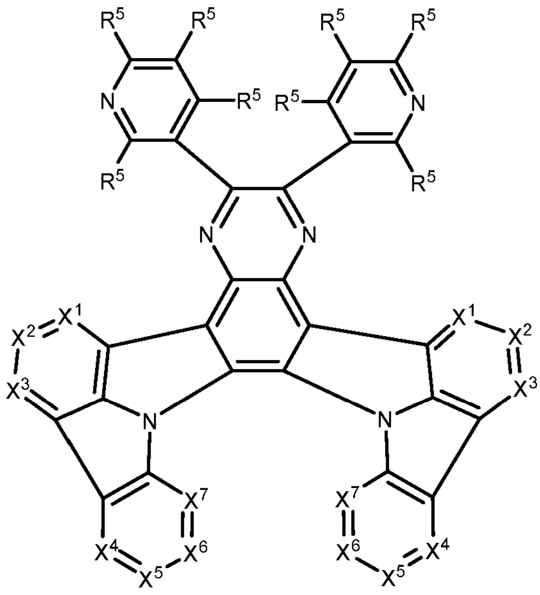

Formula Vc-5

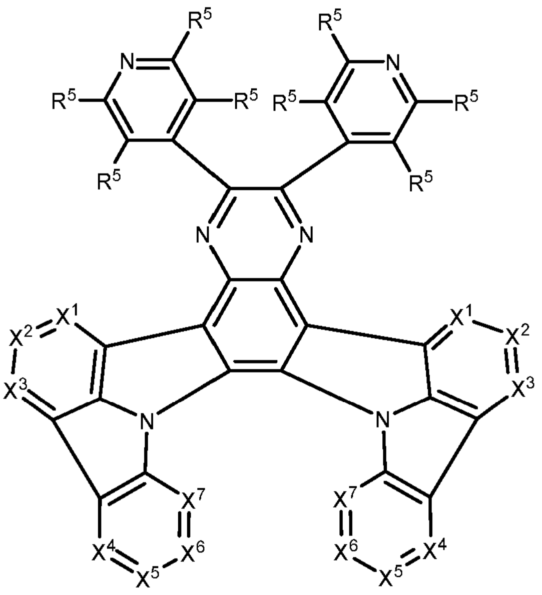

Formula Vc-6

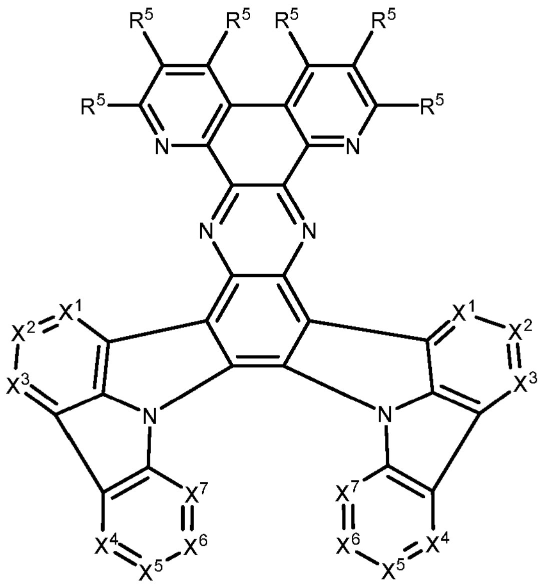

Formula Vc-7

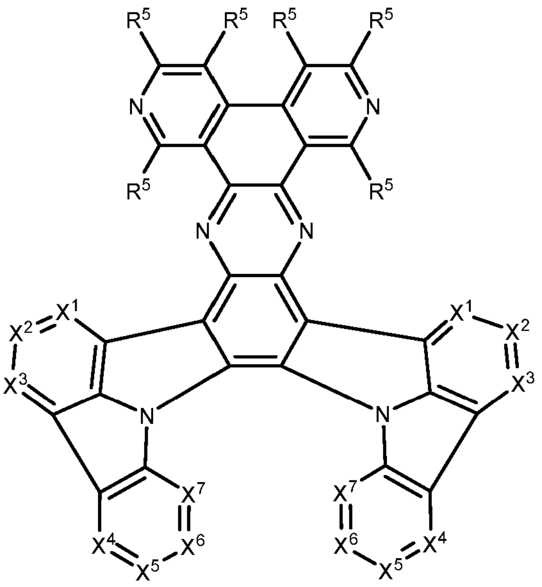

Formula Vc-8

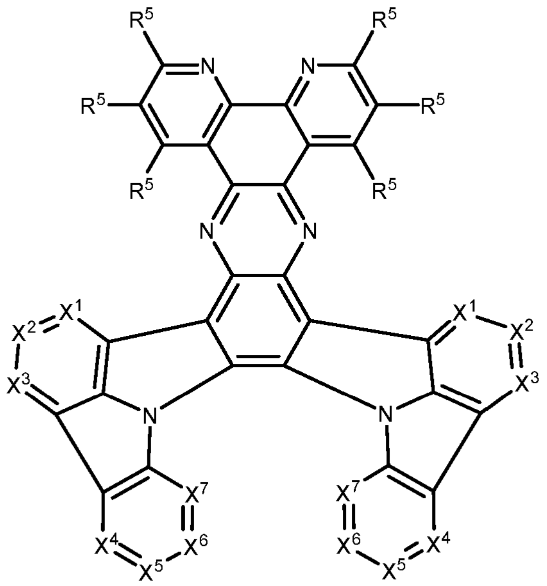

Formula Vc-9

In certain embodiments of the invention, $R^5$ is at each occurrence independently from another selected from the group consisting of:

hydrogen,
Me,
$^iPr$,
$^tBu$,
CN,
$CF_3$,
Ph, which is optionally substituted with one or more substituents independently from each other selected from the group consisting of Me, $^iPr$, $^tBu$, CN, $CF_3$, and Ph,
pyridinyl, which is optionally substituted with one or more substituents independently from each other selected from the group consisting of Me, $^iPr$, $^tBu$, CN, $CF_3$, and Ph,
pyrimidinyl, which is optionally substituted with one or more substituents independently from each other selected from the group consisting of Me, $^iPr$, $^tBu$, CN, $CF_3$, and Ph,
carbazolyl, which is optionally substituted with one or more substituents independently from each other selected from the group consisting of Me, $^iPr$, $^tBu$, CN, $CF_3$, and Ph,
triazinyl, which is optionally substituted with one or more substituents independently from each other selected from the group consisting of Me, $^iPr$, $^tBu$, CN, $CF_3$, and Ph,
and $N(Ph)_2$.

In certain embodiments of the invention, $R^5$ is at each occurrence independently from another selected from the group consisting of:

hydrogen,
$C_1$-$C_{40}$-alkyl, which is optionally substituted with one or more substituents $R^6$, and
$C_6$-$C_{60}$-aryl, which is optionally substituted with one or more substituents $R^6$.

In certain embodiments of the invention, $R^5$ is at each occurrence independently from another selected from the group consisting of:

hydrogen,
$C_1$-$C_4$-alkyl, which is optionally substituted with one or more substituents $R^6$; and
phenyl, which is optionally substituted with one or more substituents $R^6$.

In certain embodiments of the invention, $R^5$ is at each occurrence independently from another selected from the group consisting of:

hydrogen,
Me,
$^{i}Pr$,
$^{t}Bu$,
CN,
$CF_3$,
Ph, which is optionally substituted with one or more substituents independently from each other selected from the group consisting of Me, $^{i}Pr$, $^{t}Bu$, CN, $CF_3$, and Ph.

In a further embodiment of the invention, $R^a$ is at each occurrence independently from another selected from the group consisting of:

hydrogen,
Me,
$^{i}Pr$,
$^{t}Bu$,
CN,
$CF_3$,
Ph, which is optionally substituted with one or more substituents independently from each other selected from the group consisting of Me, $^{i}Pr$, $^{t}Bu$, CN, $CF_3$, and Ph,
pyridinyl, which is optionally substituted with one or more substituents independently from each other selected from the group consisting of Me, $^{i}Pr$, $^{t}Bu$, CN, $CF_3$, and Ph,
pyrimidinyl, which is optionally substituted with one or more substituents independently from each other selected from the group consisting of Me, $^{i}Pr$, $^{t}Bu$, CN, $CF_3$, and Ph,
carbazolyl, which is optionally substituted with one or more substituents independently from each other selected from the group consisting of Me, $^{i}Pr$, $^{t}Bu$, CN, $CF_3$, and Ph,
triazinyl, which is optionally substituted with one or more substituents independently from each other selected from the group consisting of Me, $^{i}Pr$, $^{t}Bu$, CN, $CF_3$, and Ph,
and $N(Ph)_2$.

In a further embodiment of the invention, $R^a$ is at each occurrence independently from another selected from the group consisting of:

hydrogen,
Me,
$^{i}Pr$,
$^{t}Bu$,
CN,
$CF_3$,
Ph, which is optionally substituted with one or more substituents independently from each other selected from the group consisting of Me, $^{i}Pr$, $^{t}Bu$, CN, $CF_3$, and Ph,
pyridinyl, which is optionally substituted with one or more substituents independently from each other selected from the group consisting of Me, $^{i}Pr$, $^{t}Bu$, CN, $CF_3$, and Ph,
pyrimidinyl, which is optionally substituted with one or more substituents independently from each other selected from the group consisting of Me, $^{i}Pr$, $^{t}Bu$, CN, $CF_3$, and Ph, and
triazinyl, which is optionally substituted with one or more substituents independently from each other selected from the group consisting of Me, $^{i}Pr$, $^{t}Bu$, CN, $CF_3$, and Ph.

In one embodiment of the invention, the organic molecules comprise or consist of a structure of formula VI:

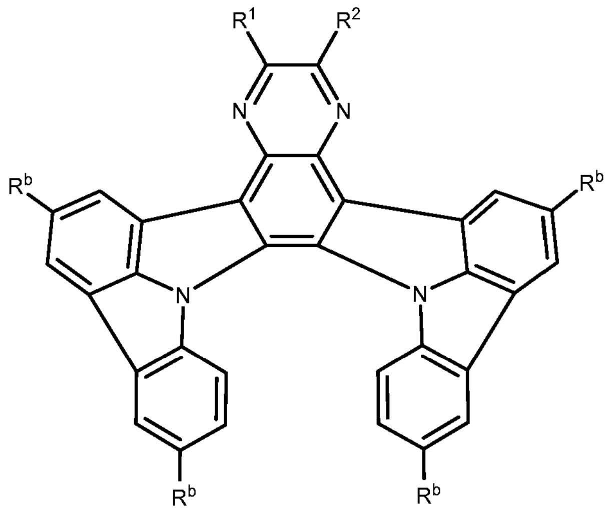

Formula VI

wherein the substituents $R^1$, $R^2$, or $R^5$ independently from each other optionally form a mono- or polycyclic, aliphatic, aromatic and/or benzo-fused ring system with one or more substituents $R^1$, $R^2$, or $R^5$, and
wherein $R^b$ is at each occurrence independently from another selected from the group consisting of:

hydrogen,
Me,
$^i$Pr,
$^t$Bu,
CN,
$CF_3$,
Ph, which is optionally substituted with one or more substituents independently from each other selected from the group consisting of Me, $^i$Pr, $^t$Bu, CN, $CF_3$, and Ph,
pyridinyl, which is optionally substituted with one or more substituents independently from each other selected from the group consisting of Me, $^i$Pr, $^t$Bu, CN, $CF_3$, and Ph,
pyrimidinyl, which is optionally substituted with one or more substituents independently from each other selected from the group consisting of Me, $^i$Pr, $^t$Bu, CN, $CF_3$, and Ph, and
triazinyl, which is optionally substituted with one or more substituents independently from each other selected from the group consisting of Me, $^i$Pr, $^t$Bu, CN, $CF_3$, and Ph, and
$N(Ph)_2$.

In one embodiment of the invention, the organic molecules comprise or consist of a structure of formula VIa:

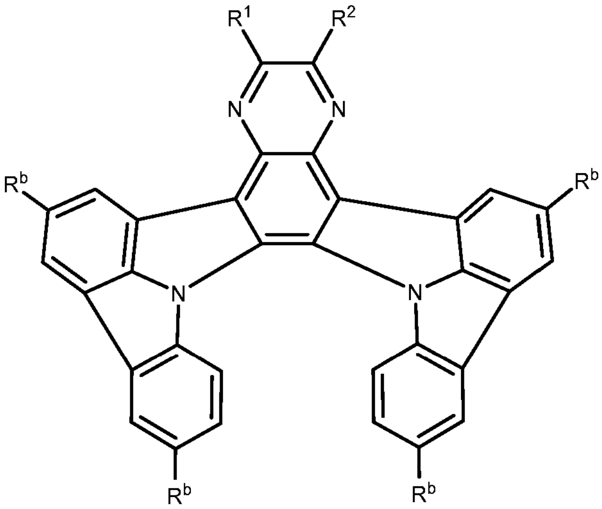

Formula VIa

wherein the substituents $R^1$ or $R^5$ independently from each other optionally form a mono- or polycyclic, aliphatic, aromatic and/or benzo-fused ring system with one or more substituents $R^1$or $R^5$.

In one embodiment of the invention, the organic molecules comprise or consist of a structure selected from the group consisting of formula VIa-1, formula VIa-2, formula VIa-3, formula VIa-4, formula VIa-5, formula VIa-6, formula VIa-7, formula VIa-8, and formula VIa-9:

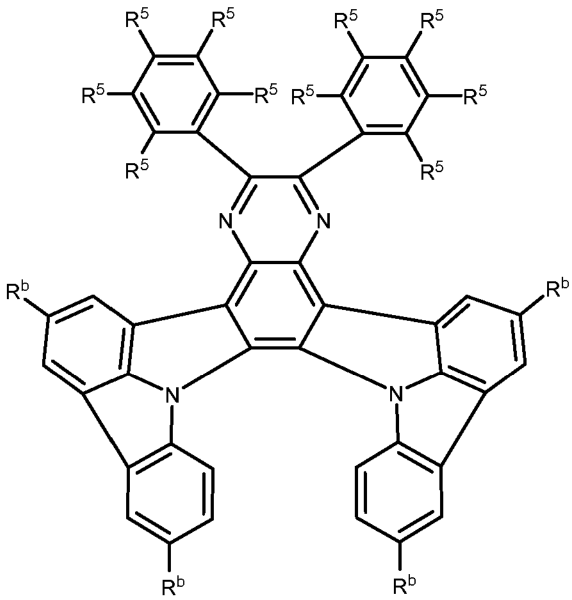

Formula VIa-1

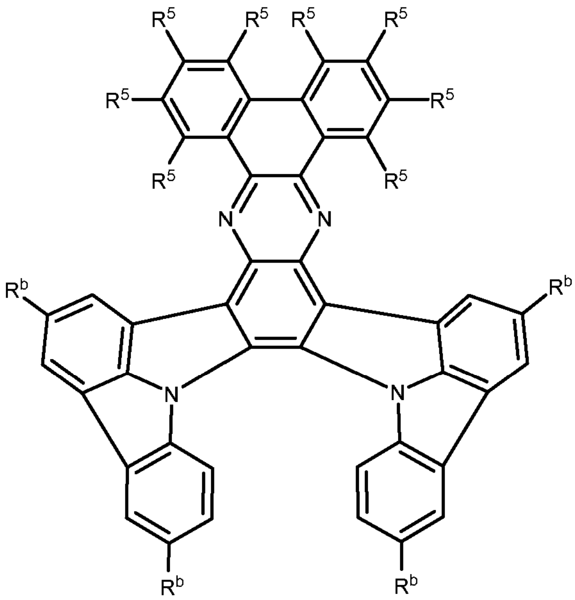

Formula VIa-2

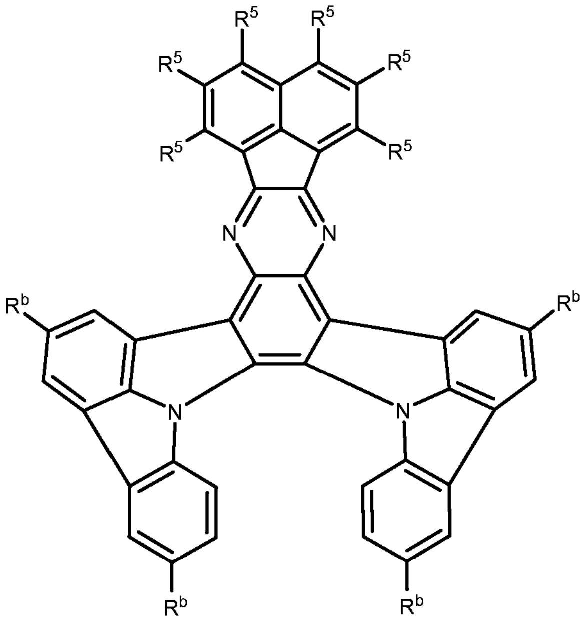

Formula VIa-3

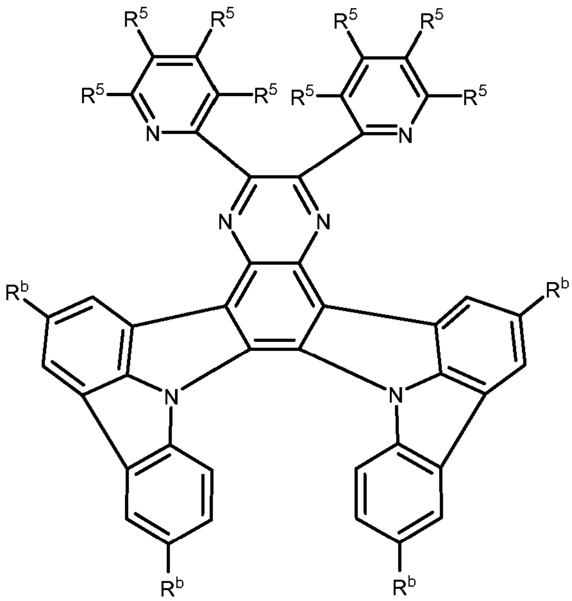

Formula VIa-4

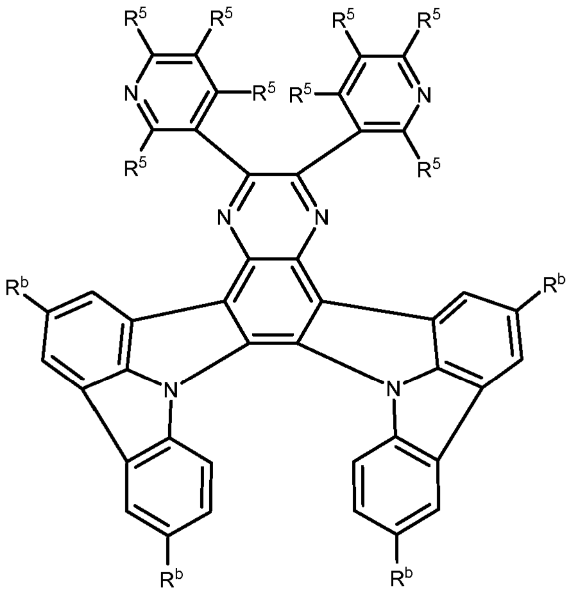

Formula VIa-5

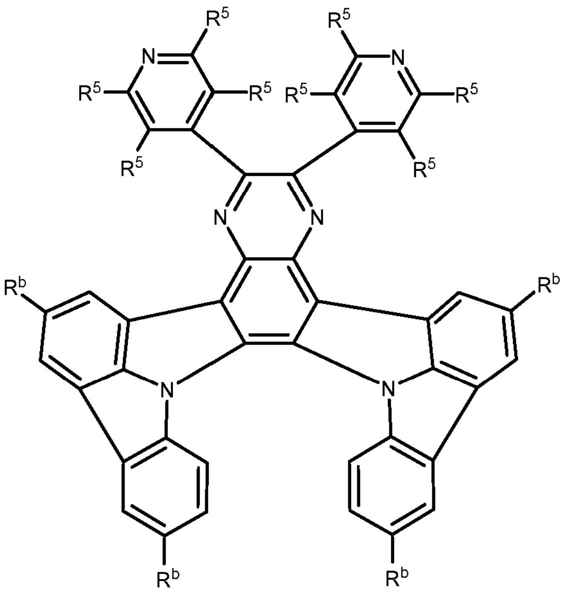

Formula VIa-6

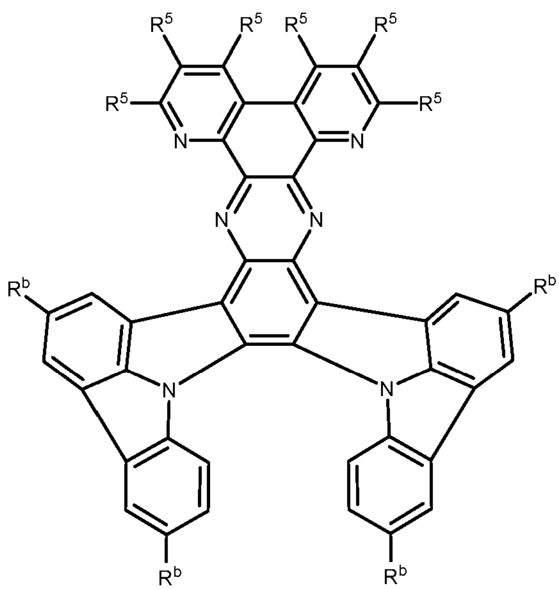

Formula VIa-7

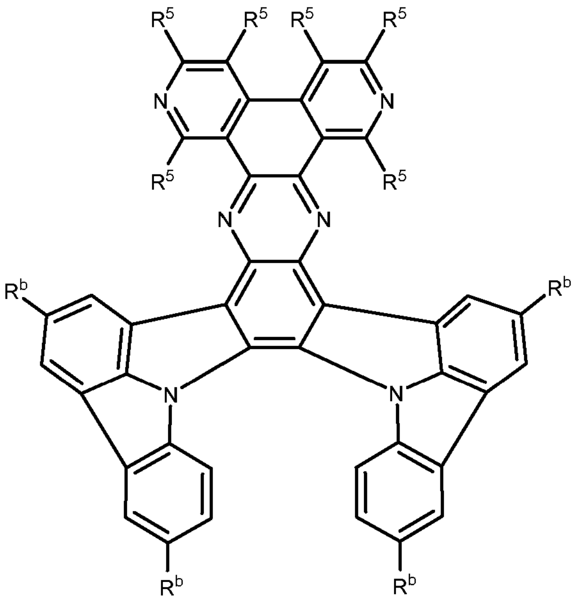

Formula VIa-8

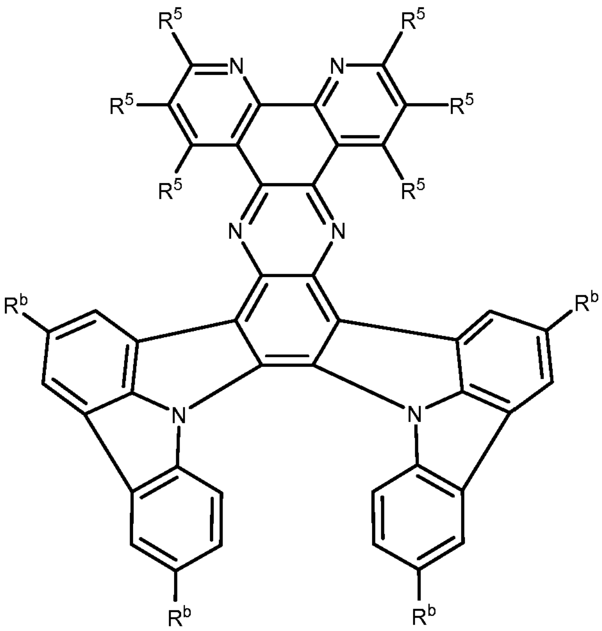

Formula VIa-9.

In one embodiment of the invention, the organic molecules comprise or consist of a structure of formula VIb:

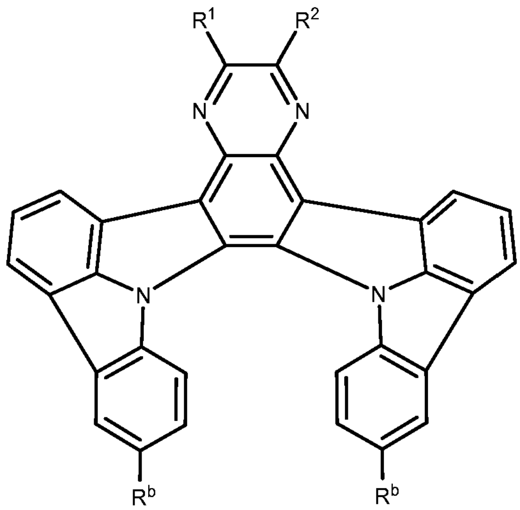

Formula VIb

wherein the substituents $R^1$, $R^2$, or $R^5$ independently from each other optionally form a mono- or polycyclic, aliphatic, aromatic and/or benzo-fused ring system with one or more substituents $R^1$, $R^2$, or $R^5$, and
wherein $R^b$ is at each occurrence independently from another selected from the group consisting of:

hydrogen,
Me,
$^i$Pr,
$^t$Bu,
CN,
$CF_3$,
Ph, which is optionally substituted with one or more substituents independently from each other selected from the group consisting of Me, $^i$Pr, $^t$Bu, CN, $CF_3$, and Ph,
pyridinyl, which is optionally substituted with one or more substituents independently from each other selected from the group consisting of Me, $^i$Pr, $^t$Bu, CN, $CF_3$, and Ph,
pyrimidinyl, which is optionally substituted with one or more substituents independently from each other selected from the group consisting of Me, $^i$Pr, $^t$Bu, CN, $CF_3$, and Ph, and
triazinyl, which is optionally substituted with one or more substituents independently from each other selected from the group consisting of Me, $^i$Pr, $^t$Bu, CN, $CF_3$, and Ph, and
$N(Ph)_2$.

In one embodiment of the invention, the organic molecules comprise or consist of a structure of formula VIb-1:

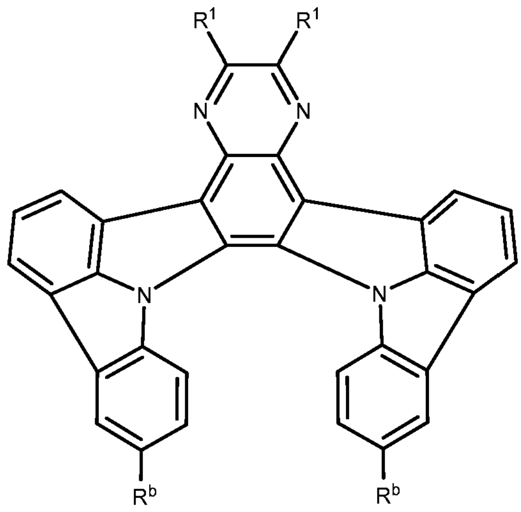

Formula VIb-1

wherein the substituents $R^1$ or $R^5$ independently from each other optionally form a mono- or polycyclic, aliphatic, aromatic and/or benzo-fused ring system with one or more substituents $R^1$or $R^5$.

In one embodiment of the invention, the organic molecules comprise or consist of a structure of formula VIc:

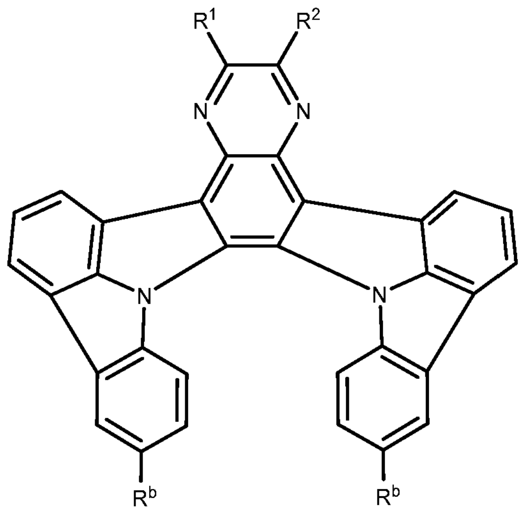

Formula VIc

wherein the substituents $R^1$, $R^2$, or $R^5$ independently from each other optionally form a mono-or polycyclic, aliphatic, aromatic and/or benzo-fused ring system with one or more substituents $R^1$, $R^2$, or $R^5$, and
wherein $R^b$ is at each occurrence independently from another selected from the group consisting of:

hydrogen,
Me,
$^i$Pr,
$^t$Bu,
CN,
$CF_3$,
Ph, which is optionally substituted with one or more substituents independently from each other selected from the group consisting of Me, $^i$Pr, $^t$Bu, CN, $CF_3$, and Ph,
pyridinyl, which is optionally substituted with one or more substituents independently from each other selected from the group consisting of Me, $^i$Pr, $^t$Bu, CN, $CF_3$, and Ph, pyrimidinyl, which is optionally substituted with one or more substituents independently from each other selected from the group consisting of Me, $^i$Pr, $^t$Bu, CN, $CF_3$, and Ph, and
triazinyl, which is optionally substituted with one or more substituents independently from each other selected from the

group consisting of Me, $^iPr$, $^tBu$, CN, $CF_3$, and Ph, and
$N(Ph)_2$.

In one embodiment of the invention, the organic molecules comprise or consist of a structure of formula VIc-1:

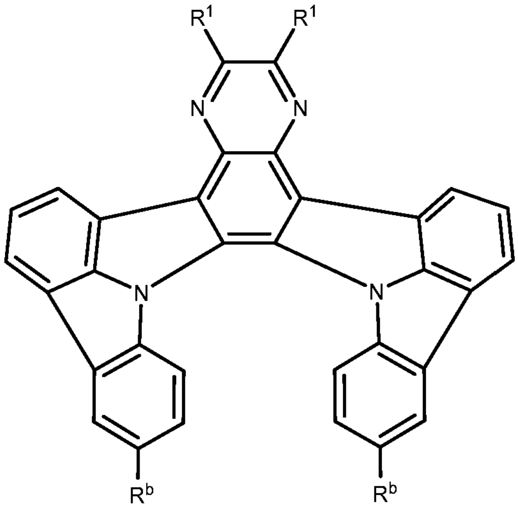

Formula VIc-1

wherein the substituents $R^1$ or $R^5$ independently from each other optionally form a mono- or polycyclic, aliphatic, aromatic and/or benzo-fused ring system with one or more substituents $R^1$or $R^5$.

In one embodiment of the invention, the organic molecules comprise or consist of a structure of formula VII:

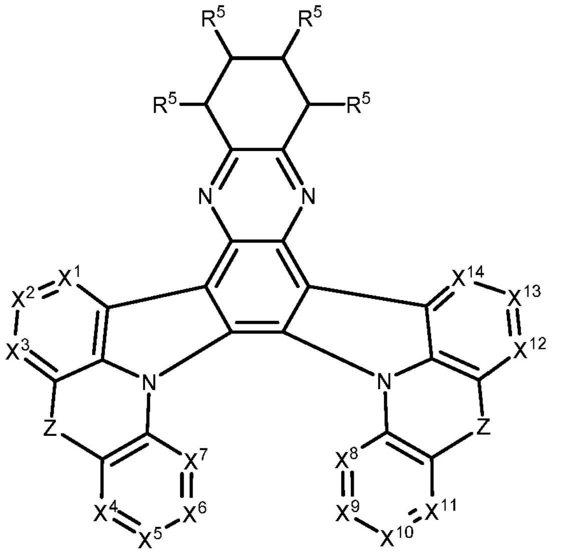

Formula VII.

As used throughout the present application, the terms "aryl" and "aromatic" may be understood in the broadest sense as any mono-, bi- or polycyclic aromatic moieties. Accordingly, an aryl group contains 6 to 60 aromatic ring atoms, and a heteroaryl group contains 5 to 60 aromatic ring atoms, of which at least one is a heteroatom. Notwithstanding, throughout the application the number of aromatic ring atoms may be given as subscripted number in the definition of certain substituents. In particular, the heteroaromatic ring includes one to three heteroatoms. Again, the terms "heteroaryl" and "heteroaromatic" may be understood in the broadest sense as any mono-, bi- or polycyclic hetero-aromatic moieties that include at least one heteroatom. The heteroatoms may at each occurrence be the same or different and be individually selected from the group consisting of N, O and S. Accordingly, the term "arylene" refers to a divalent substituent that bears two binding sites to other molecular structures and thereby serving as a linker structure. In case, a group in the exemplary embodiments is defined differently from the definitions given here, for example, the number of aromatic ring atoms or

number of heteroatoms differs from the given definition, the definition in the exemplary embodiments is to be applied. According to the invention, a condensed (annulated) aromatic or heteroaromatic polycycle is built of two or more single aromatic or heteroaromatic cycles, which formed the polycycle via a condensation reaction.

In particular, as used here throughout, the term "aryl group or heteroaryl group" comprises groups which can be bound via any position of the aromatic or heteroaromatic group, derived from benzene, naphthaline, anthracene, phenanthrene, pyrene, dihydropyrene, chrysene, perylene, fluoranthene, benzanthracene, benzphenanthrene, tetracene, pentacene, benzpyrene, furan, benzofuran, isobenzofuran, dibenzofuran, thiophene, benzothiophene, isobenzothiophene, dibenzothiophene; pyrrole, indole, isoindole, carbazole, pyridine, quinoline, isoquinoline, acridine, phenanthridine, benzo-5,6-quinoline, benzo-6,7-quinoline, benzo-7,8-quinoline, phenothiazine, phenoxazine, pyrazole, indazole, imidazole, benzimidazole, naphthoimidazole, phenanthroimidazole, pyridoimidazole, pyrazinoimidazole, quinoxalinoimidazole, oxazole, benzoxazole, napthooxazole, anthroxazol, phenanthroxazol, isoxazole, 1,2-thiazole, 1,3-thiazole, benzothiazole, pyridazine, benzopyridazine, pyrimidine, benzopyrimidine, 1,3,5-triazine, quinoxaline, pyrazine, phenazine, naphthyridine, carboline, benzocarboline, phenanthroline, 1,2,3-triazole, 1,2,4-triazole, benzotriazole, 1,2,3-oxadiazole, 1,2,4-oxadiazole, 1,2,5-oxadiazole, 1,2,3,4-tetrazine, purine, pteridine, indolizine and benzothiadiazole or combinations of the abovementioned groups.

As used here throughout, the term "cyclic group" may be understood in the broadest sense as any mono-, bi- or polycyclic moieties.

As used here throughout, the term "biphenyl" as a substituent may be understood in the broadest sense as ortho-biphenyl, meta-biphenyl, or para-biphenyl, wherein ortho, meta and para is defined in regard to the binding site to another chemical moiety.

As used here throughout, the term "terphenyl" as a substituent may be understood in the broadest sense as 3-ortho-terphenyl, 4-ortho-terphenyl, 4-meta-terphenyl, 5-meta-terphenyl, 2-para-terphenyl or 3-para-terphenyl, wherein ortho, meta and para is defined in regard to the position of the Ph moieties to each other and "2-", "3-", "4-" and "5-" is defined in regard to the binding site to another chemical moiety, i.e.:

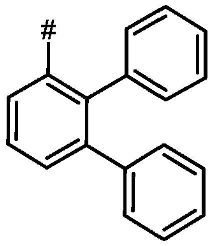

3-ortho-terphenyl

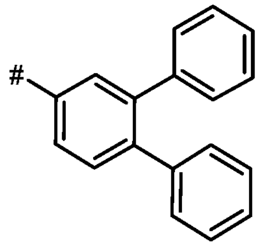

4-ortho-terphenyl

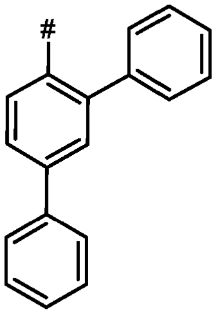

4-meta-terphenyl

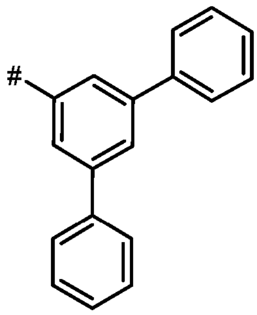

5-meta-terphenyl

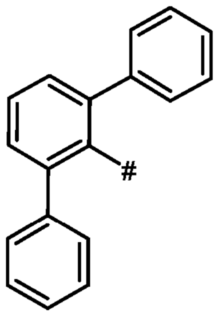

2-meta-terphenyl

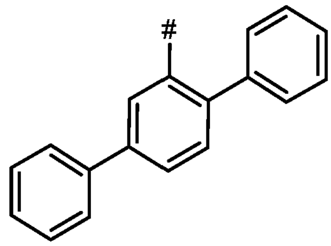

2-para-terphenyl

wherein # indicates the binding site to another chemical moiety.

As used here throughout, the term "naphthyl" as a naphthalene substituent may be understood in the broadest sense as 1-naphthyl and 2-naphthyl, wherein "1-" and "2-" is defined in regard to the binding site to another chemical moiety, i.e.:

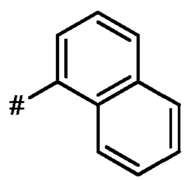

1- naphthyl

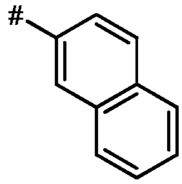

2- naphthyl

wherein # indicates the binding site to another chemical moiety.

As used here throughout, the term "anthracene" as a substituent may be understood in the broadest sense as 1-anthracenyl, 2-anthracenyl and 9-anthracenyl wherein "1-", "2-" and "9-" is defined in regard to the binding site to another chemical moiety, i.e.:

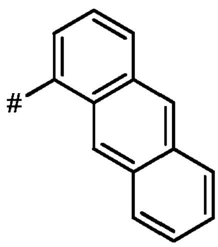

1- anthracenyl

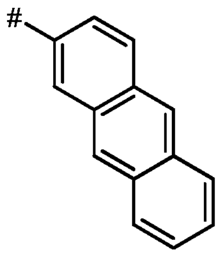

2- anthracenyl

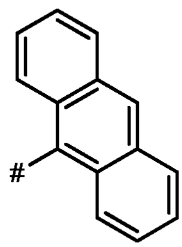

9- anthracenyl

wherein # indicates the binding site to another chemical moiety.

As used here throughout, the term "alkyl group" may be understood in the broadest sense as any linear, branched, or cyclic alkyl substituent. In particular, the term alkyl comprises the substituents methyl (Me), ethyl (Et), n-propyl ($^n$Pr), i-propyl ($^i$Pr), cyclopropyl, n-butyl ($^n$Bu), i-butyl ($^i$Bu), s-butyl ($^s$Bu), t-butyl ($^t$Bu), cyclobutyl, 2-methylbutyl, n-pentyl, s-pentyl, t-pentyl, 2-pentyl, neo-pentyl, cyclopentyl, n-hexyl, s-hexyl, t-hexyl, 2-hexyl, 3-hexyl, neo-hexyl, cyclohexyl, 1-methylcyclopentyl, 2-methylpentyl, n-heptyl, 2-heptyl, 3-heptyl, 4-heptyl, cycloheptyl, 1-methylcyclohexyl, n-octyl, 2-ethylhexyl, cyclooctyl, 1-bicyclo[2,2,2]octyl, 2-bicyclo[2,2,2]-octyl, 2-(2,6-dimethyl)octyl, 3-(3,7-dimethyl)octyl, adamantyl, 2,2,2-trifluorethyl, 1,1-dimethyl-n-hex-1-yl, 1,1-dimethyl-n-hept-1-yl, 1,1-dimethyl-n-oct-1-yl, 1,1-dimethyl-n-dec-1-yl, 1,1-dimethyl-n-dodec-1-yl, 1,1-dimethyl-n-tetradec-1-yl, 1,1-dimethyl-n-hexadec-1-yl, 1,1-dimethyl-n-octadec-1-yl, 1,1-diethyl-n-hex-1-yl, 1,1-diethyl-n-hept-1-yl, 1,1-diethyl-n-oct-1-yl, 1,1-diethyl-n-dec-1-yl, 1,1-diethyl-n-dodec-1-yl, 1,1-diethyl-n-tetradec-1-yl, 1,1-diethyln-n-hexadec-1-yl, 1,1-diethyl-n-octadec-1-yl, 1-(n-propyl)-cyclohex-1-yl, 1-(n-butyl)-cyclohex-1-yl, 1-(n-hexyl)-cyclohex-1-yl, 1-(n-octyl)-cyclohex-1-yl and 1-(n-decyl)-cyclohex-1-yl.

As used throughout, the term "alkenyl" comprises linear, branched, and cyclic alkenyl substituents. The term "alkenyl group", for example, comprises the substituents ethenyl, propenyl, butenyl, pentenyl, cyclopentenyl, hexenyl, cyclohexenyl, heptenyl, cycloheptenyl, octenyl, cyclooctenyl or cyclooctadienyl.

As used throughout, the term "alkynyl" comprises linear, branched, and cyclic alkynyl substituents. The term "alkynyl group", for example, comprises ethynyl, propynyl, butynyl, pentynyl, hexynyl, heptynyl or octynyl.

As used throughout, the term "alkoxy" comprises linear, branched, and cyclic alkoxy substituents. The term

"alkoxy group" exemplarily comprises methoxy, ethoxy, n-propoxy, i-propoxy, n-butoxy, i-butoxy, s-butoxy, t-butoxy and 2-methylbutoxy.

As used throughout, the term "thioalkoxy" comprises linear, branched, and cyclic thioalkoxy substituents, in which the O of the exemplarily alkoxy groups is replaced by S.

As used throughout, the terms "halogen" and "halo" may be understood in the broadest sense as being preferably fluorine, chlorine, bromine or iodine.

Whenever hydrogen (H) is mentioned herein, it could also be replaced by deuterium at each occurrence.

It is understood that when a molecular fragment is described as being a substituent or otherwise attached to another moiety, its name may be written as if it were a fragment (e.g. naphthyl, dibenzofuryl) or as if it were the whole molecule (e.g. naphthalene, dibenzofuran). As used herein, these different ways of designating a substituent or attached fragment are considered to be equivalent.

In one embodiment of the invention, the organic molecules according to the invention have an emission peak in the visible or nearest ultraviolet range, i.e., in the range of a wavelength of from 380 nm to 800 nm, with a full width at half maximum of less than 0.35 eV, preferably less than 0.30 eV, more preferably less than 0.26 eV, even more preferably less than 0.22 eV or even less than 0.18 eV with 0.001 mg/mL in an organic solvent, preferably in DCM or toluene, of organic molecule at room temperature.

The energy of the first excited triplet state T1 is determined from the onset of the emission spectrum at low temperature, typically at 77 K. The phosphorescence is usually visible in a steady-state spectrum in a film of 2% by weight emitter and 98% by weight PMMA. The triplet energy can thus be determined as the onset of the phosphorescence spectrum. For Fluorescent emitter molecules, the energy of the first excited triplet state T1 is determined from the onset of the delayed emission spectrum at 77 K.

The onset of an emission spectrum is determined by computing the intersection of the tangent to the emission spectrum with the x-axis. The tangent to the emission spectrum is set at the high-energy side of the emission band and at the point at half maximum of the maximum intensity of the emission spectrum.

In one embodiment, the organic molecules according to the invention have an onset of the emission spectrum, which is energetically close to the emission maximum, i.e. the energy difference between the onset of the emission spectrum and the energy of the emission maximum is below 0.14 eV, preferably below 0.13 eV, or even below 0.12 eV, while the full width at half maximum (FWHM) of the organic molecules is less than 0.35 eV, preferably less than 0.30 eV, more preferably less than 0.26 eV, even more preferably less than 0.22 eV or even less than 0.18 eV with 0.001 mg/mL in DCM of organic molecule at room temperature, resulting in a CIEy coordinate below 0.20, preferably below 0.18, more preferably below 0.16 or even more preferred below 0.14.

A further aspect of the invention relates to the use of an organic molecule of the invention as a luminescent emitter or as an absorber, and/or as a host material and/or as an electron transport material, and/or as a hole injection material, and/or as a hole blocking material in an optoelectronic device.

A preferred embodiment relates to the use of an organic molecule according to the invention as a luminescent emitter in an optoelectronic device.

The optoelectronic device may be understood in the broadest sense as any device based on organic materials that is suitable for emitting light in the visible or nearest ultraviolet (UV) range, i.e. in the range of a wavelength of from 380 to 800 nm. More preferably, the optoelectronic device may be able to emit light in the visible range, i.e. light of from 400 nm to 800 nm.

In the context of such use, the optoelectronic device is more particularly selected from the group consisting of:

- organic light-emitting diodes (OLEDs),
- light-emitting electrochemical cells,
- OLED sensors, especially in gas and vapor sensors that are not hermetically shielded to the surroundings,
- organic diodes,
- organic solar cells,
- organic transistors,
- organic field-effect transistors,
- organic lasers, and
- down-conversion elements.

In a preferred embodiment in the context of such use, the optoelectronic device is a device selected from the group consisting of an organic light emitting diode (OLED), a light emitting electrochemical cell (LEC), and a light-emitting transistor.

In the case of the use, the fraction of the organic molecule according to the invention in the emission layer in an optoelectronic device, more particularly in an OLED, is 0.1 % to 99 % by weight, more particularly 1 % to 80 % by weight. In an alternative embodiment, the proportion of the organic molecule in the emission layer is 100 % by weight.

In one embodiment, the light-emitting layer comprises not only the organic molecules according to the invention, but also a host material whose triplet (T1) and singlet (S1) energy levels are energetically higher than the triplet (T1) and singlet (S1) energy levels of the organic molecule.

A further aspect of the invention relates to a composition comprising or consisting of:

(a) at least one organic molecule according to the invention, in particular in the form of an emitter, and
(b) one or more triplet-triplet annihilation (TTA) host materials, which differ from the organic molecule according to the invention, and
(c) optionally one or more TADF materials, and
(d) optionally one or more dyes and/or one or more solvents.

A further aspect of the invention relates to a composition comprising or consisting of:

(a) at least one organic molecule according to the invention, in particular in the form of an emitter, and
(b) one or more host materials, which differ from the organic molecule according to the invention, and
(c) one or more TADF materials, and
(d) optionally, one or more dyes and/or one or more solvents.

A further aspect of the invention relates to a composition comprising or consisting of:

(a) at least one organic molecule according to the invention, in particular in the form of an emitter, and
(b) one or more host materials, which differ from the organic molecule according to the invention, and
(c) one or more phosphorescent materials, and
(d) optionally, one or more dyes and/or one or more solvents.

In a particular embodiment, the light-emitting layer EML comprises or essentially consists of a composition comprising or consisting of:

(i) 0.1-10 % by weight, preferably 0.5-5 % by weight, in particular 1-3 % by weight, of one or more organic molecules according to the invention;
(ii) 5-99 % by weight, preferably 15-85 % by weight, in particular 20-75% by weight, of at least one host compound H; and
(iii) 0.9-94.9 % by weight, preferably 14.5-80 % by weight, in particular 24-77 % by weight, of at least one further host compound D with a structure differing from the structure of the molecules according to the invention; and
(iv) optionally 0-94 % by weight, preferably 0-65 % by weight, in particular 0-50 % by weight, of a solvent; and
(v) optionally 0-30 % by weight, in particular 0-20 % by weight, preferably 0-5 % by weight, of at least one further emitter molecule F with a structure differing from the structure of the molecules according to the invention.

*Compositions with one or more TTA host material*

In a preferred embodiment, in the optoelectronic device of the present invention, the light-emitting layer B comprises (or consist of):

(i) 10-84 % by weight of the TTA material $H^N$;
(ii) 0-30 % by weight of the TADF material $E^B$; and
(iii) 0.1-10 % by weight of the emitter according to the invention; and optionally
(iv) 0-74 % by weight of one or more solvents.

In a preferred embodiment, the percentage numbers of (i)-(iv) sum up to 100 % by weight.

In another preferred embodiment, in the optoelectronic device of the present invention, the light-emitting layer B comprises (or consist of):

(i) 56-90 % by weight of the TTA material $H^N$;
(ii) 0-5 % by weight of the TADF material $E^B$; and
(iii) 0.5-5 % by weight of the emitter according to the invention; and optionally
(iv) 0-34 % by weight of one or more solvents.

In a preferred embodiment, the percentage numbers of (i)-(iv) sum up to 100 % by weight.

*Compositions with one or more TADF material*

In one embodiment, the light-emitting layer B comprises:

(i) 10-89.9 % by weight of one or more p-host compound $H^P$;
(ii) 0-79.9 % by weight of one or more n-host compound $H^N$;
(iii) 10-50 % by weight of one or more TADF material $E^B$; and
(iv) 0.1-10 % by weight of one or more emitter according to the invention; and
(v) 0-79.9 % by weight of one or more solvents.

In one embodiment, the light-emitting layer B comprises:

(i) 22-87.5 % by weight of one or more p-host compound $H^P$;
(ii) 0-65.5 % by weight of one or more n-host compound $H^N$;
(iii) 12-40 % by weight of one or more TADF material $E^B$; and
(iv) 0.5-5 % by weight of one or more emitter according to the invention; and
(v) 0-65.5 % by weight of one or more solvents.

*Compositions with one or more phosphorescent material*

In a preferred embodiment where $H^N$ is optional, in the optoelectronic device of the present invention, the light-emitting layer B comprises (or consists of):

(i) 10-84.9 % by weight of the host compound $H^P$;
(ii) 0-84.9 % by weight of the host compound $H^N$;
(iii) 5-15 % by weight of the Phosphorescence material $E^B$; and
(iv) 0.1-10 % by weight of the emitter according to the invention; and optionally
(v) 0-84.9 % by weight of one or more solvents.

In a preferred embodiment where $H^N$ is optional, in the optoelectronic device of the present invention, the light-emitting layer B comprises (or consists of):

(i) 22-70.5 % by weight of the host compound $H^P$;
(ii) 0-72.5 % by weight of the host compound $H^N$;
(iii) 5-10 % by weight of the phosphorescence material $E^B$; and
(iv) 0.5-5 % by weight of the emitter according to the invention; and optionally
(v) 0-72.5 % by weight of one or more solvents.

Preferably, energy can be transferred from the host compound H to the one or more organic molecules according to the invention, in particular transferred from the first excited triplet state T1(H) of the host compound H to the first excited triplet state T1(E) of the one or more organic molecules according to the invention E and/ or from the first excited singlet state S1(H) of the host compound H to the first excited singlet state S1(E) of the one or more organic molecules according to the invention E.

In one embodiment, the host compound H has a highest occupied molecular orbital HOMO(H) having an energy $E^{HOMO}(H)$ in the range of from -5 to -6.5 eV and the at least one further host compound D has a highest occupied molecular orbital HOMO(D) having an energy $E^{HOMO}(D)$, wherein $E^{HOMO}(H) > E^{HOMO}(D)$.

In a further embodiment, the host compound H has a lowest unoccupied molecular orbital LUMO(H) having an energy $E^{LUMO}(H)$ and the at least one further host compound D has a lowest unoccupied molecular orbital LUMO(D) having an energy $E^{LUMO}(D)$, wherein $E^{LUMO}(H) > E^{LUMO}(D)$,

In one embodiment, the host compound H has a highest occupied molecular orbital HOMO(H) having an energy $E^{HOMO}(H)$ and a lowest unoccupied molecular orbital LUMO(H) having an energy $E^{LUMO}(H)$, and

the at least one further host compound D has a highest occupied molecular orbital HOMO(D) having an energy $E^{HOMO}(D)$ and a lowest unoccupied molecular orbital LUMO(D) having an energy $E^{LUMO}(D)$,
the organic molecule according to the invention E has a highest occupied molecular orbital HOMO(E) having an energy $E^{HOMO}(E)$ and a lowest unoccupied molecular orbital LUMO(E) having an energy $E^{LUMO}(E)$,

wherein

$E^{HOMO}(H) > E^{HOMO}(D)$ and the difference between the energy level of the highest occupied molecular orbital HOMO(E) of the organic molecule according to the invention E ($E^{HOMO}(E)$) and the energy level of the highest occupied molecular orbital HOMO(H) of the host compound H ($E^{HOMO}(H)$) is between -0.5 eV and 0.5 eV, more preferably between -0.3 eV and 0.3 eV, even more preferably between -0.2 eV and 0.2 eV or even between -0.1 eV and 0.1 eV; and $E^{LUMO}(H) > E^{LUMO}(D)$ and the difference between the energy level of the lowest unoccupied molecular orbital LUMO(E) of the organic molecule according to the invention E ($E^{LUMO}(E)$) and the lowest unoccupied molecular orbital LUMO(D) of the at least one further host compound D ($E^{LUMO}(D)$) is between -0.5 eV and 0.5 eV, more preferably between -0.3 eV and 0.3 eV, even more preferably between -0.2 eV and 0.2 eV or even between -0.1 eV and 0.1 eV.

In one embodiment of the invention the host compound D and/or the host compound H is a thermally-activated delayed fluorescence (TADF)-material. TADF materials exhibit a $\Delta E_{ST}$ value, which corresponds to the energy difference between the first excited singlet state (S1) and the first excited triplet state (T1), of less than 2500 $cm^{-1}$. Preferably the TADF material exhibits a $\Delta E_{ST}$ value of less than 3000 $cm^{-1}$, more preferably less than 1500 $cm^{-1}$, even more preferably less than 1000 $cm^{-1}$ or even less than 500 $cm^{-1}$.

In one embodiment, the host compound D is a TADF material and the host compound H exhibits a $\Delta E_{ST}$ value of more than 2500 $cm^{-1}$. In a particular embodiment, the host compound D is a TADF material and the host compound H is selected from group consisting of CBP, mCP, mCBP, 9-[3-(dibenzofuran-2-yl)phenyl]-9H-carbazole, 9-[3-(dibenzofuran-2-yl)phenyl]-9H-carbazole, 9-[3-(dibenzothiophen-2-yl)phenyl]-9H-carbazole, 9-[3,5-bis(2-dibenzofuranyl)phenyl]-9H-carbazole and 9-[3,5-bis(2-dibenzothiophenyl)phenyl]-9H-carbazole.

In one embodiment, the host compound H is a TADF material and the host compound D exhibits a $\Delta E_{ST}$ value of more than 2500 $cm^{-1}$. In a particular embodiment, the host compound H is a TADF material and the host compound D is selected from group consisting of T2T (2,4,6-tris(biphenyl-3-yl)-1,3,5-triazine), T3T (2,4,6-tris(triphenyl-3-yl)-1,3,5-triazine) and/or TST (2,4,6-tris(9,9'-spirobifluorene-2-yl)-1,3,5-triazine).

In a further aspect, the invention relates to an optoelectronic device comprising an organic molecule or a composition of the type described here, more particularly in the form of a device selected from the group consisting of organic light-emitting diode (OLED), light-emitting electrochemical cell, OLED sensor (particularly gas and vapor sensors not hermetically externally shielded), organic diode, organic solar cell, organic transistor, organic field-effect transistor, organic laser and down-conversion element.

In a preferred embodiment, the optoelectronic device is a device selected from the group consisting of an organic light emitting diode (OLED), a light emitting electrochemical cell (LEC), and a light-emitting transistor.

In one embodiment of the optoelectronic device of the invention, the organic molecule according to the invention E is used as emission material in a light-emitting layer EML.

In one embodiment of the optoelectronic device of the invention, the light-emitting layer EML consists of the composition according to the invention described here.

When the optoelectronic device is an OLED, it may, for example, have the following layer structure:

1. substrate
2. anode layer A
3. hole injection layer, HIL
4. hole transport layer, HTL
5. electron blocking layer, EBL
6. emitting layer, EML
7. hole blocking layer, HBL
8. electron transport layer, ETL
9. electron injection layer, EIL
10. cathode layer,

wherein the OLED comprises each layer selected from the group of HIL, HTL, EBL, HBL, ETL, and EIL only optionally, different layers may be merged and the OLED may comprise more than one layer of each layer type defined above.

Furthermore, the optoelectronic device may, in one embodiment, comprise one or more protective layers protecting the device from damaging exposure to harmful species in the environment including, for example, moisture, vapor and/or gases.

In one embodiment of the invention, the optoelectronic device is an OLED, with the following inverted layer structure:

1. substrate
2. cathode layer
3. electron injection layer, EIL
4. electron transport layer, ETL

5. hole blocking layer, HBL
6. emitting layer, B
7. electron blocking layer, EBL
8. hole transport layer, HTL
9. hole injection layer, HIL
10. anode layer A

wherein the OLED comprises each layer selected from the group of HIL, HTL, EBL, HBL, ETL, and EIL only optionally, different layers may be merged and the OLED may comprise more than one layer of each layer types defined above.

In one embodiment of the invention, the optoelectronic device is an OLED, which may have a stacked architecture. In this architecture, contrary to the typical arrangement in which the OLEDs are placed side by side, the individual units are stacked on top of each other. Blended light may be generated with OLEDs exhibiting a stacked architecture, in particular white light may be generated by stacking blue, green and red OLEDs. Furthermore, the OLED exhibiting a stacked architecture may comprise a charge generation layer (CGL), which is typically located between two OLED subunits and typically consists of a n-doped and p-doped layer with the n-doped layer of one CGL being typically located closer to the anode layer.

In one embodiment of the invention, the optoelectronic device is an OLED, which comprises two or more emission layers between anode and cathode. In particular, this so-called tandem OLED comprises three emission layers, wherein one emission layer emits red light, one emission layer emits green light and one emission layer emits blue light, and optionally may comprise further layers such as charge generation layers, blocking or transporting layers between the individual emission layers. In a further embodiment, the emission layers are adjacently stacked. In a further embodiment, the tandem OLED comprises a charge generation layer between each two emission layers. In addition, adjacent emission layers or emission layers separated by a charge generation layer may be merged.

The substrate may be formed by any material or composition of materials. Most frequently, glass slides are used as substrates. Alternatively, thin metal layers (e.g., copper, gold, silver or aluminum films) or plastic films or slides may be used. This may allow for a higher degree of flexibility. The anode layer A is mostly composed of materials allowing to obtain an (essentially) transparent film. As at least one of both electrodes should be (essentially) transparent in order to allow light emission from the OLED, either the anode layer A or the cathode layer C is transparent. Preferably, the anode layer A comprises a large content or even consists of transparent conductive oxides (TCOs). Such anode layer A may, for example, comprise indium tin oxide, aluminum zinc oxide, fluorine doped tin oxide, indium zinc oxide, PbO, SnO, zirconium oxide, molybdenum oxide, vanadium oxide, tungsten oxide, graphite, doped Si, doped Ge, doped GaAs, doped polyaniline, doped polypyrrol and/or doped polythiophene.

The anode layer A (essentially) may consist of indium tin oxide (ITO) (e.g., $(InO_3)_{0.9}(SnO_2)_{0.1}$). The roughness of the anode layer A caused by the transparent conductive oxides (TCOs) may be compensated by using a hole injection layer (HIL). Further, the HIL may facilitate the injection of quasi charge carriers (i.e., holes) in that the transport of the quasi charge carriers from the TCO to the hole transport layer (HTL) is facilitated. The hole injection layer (HIL) may comprise poly-3,4-ethylendioxy thiophene (PEDOT), polystyrene sulfonate (PSS), $MoO_2$, $V_2O_5$, CuPC or CuI, in particular a mixture of PEDOT and PSS. The hole injection layer (HIL) may also prevent the diffusion of metals from the anode layer A into the hole transport layer (HTL). The HIL may, for example, comprise PEDOT:PSS (poly-3,4-ethylendioxy thiophene: polystyrene sulfonate), PEDOT (poly-3,4-ethylendioxy thiophene), mMTDATA (4,4',4"-tris[phenyl(m-tolyl)amino]triphenylamine), Spiro-TAD (2,2',7,7'-tetrakis(n,n-diphenylamino)-9,9'-spirobifluorene), DNTPD (N1,N1'-(biphenyl-4,4'-diyl) bis(N1-phenyl-N4,N4-di-m-tolylbenzene-1,4-diamine), NPB (N,N'-nis-(1-naphthalenyl)-N,N'-bis-phenyl-(1,1'-biphenyl)-4,4'-diamine), NPNPB (N,N'-diphenyl-N,N'-di-[4-(N,N-diphenyl-amino)phenyl]benzidine), MeO-TPD (N,N,N',N'-tetrakis(4-methoxyphenyl)benzidine), HAT-CN (1,4,5,8,9,11-hexaazatriphenylen-hexacarbonitrile) and/or Spiro-NPD (N,N'-diphenyl-N,N'-bis-(1-naphthyl)-9,9'-spirobifluorene-2,7-diamine).

Adjacent to the anode layer A or hole injection layer (HIL), a hole transport layer (HTL) is typically located. Herein, any hole transport compound may be used. For example, electron-rich heteroaromatic compounds such as triarylamines and/or carbazoles may be used as hole transport compound. The HTL may decrease the energy barrier between the anode layer A and the light-emitting layer EML. The hole transport layer (HTL) may also be an electron blocking layer (EBL). Preferably, hole transport compounds bear comparably high energy levels of their triplet states T1. For example, the hole transport layer (HTL) may comprise a star-shaped heterocycle such as tris(4-carbazoyl-9-ylphenyl)amine (TCTA), poly-TPD (poly(4-butylphenyl-diphenyl-amine)), [alpha]-NPD (poly(4-butylphenyl-diphenyl-amine)), TAPC (4,4'-cyclohexyliden-bis[N,N-bis(4-methylphenyl)benzenamine]), 2-TNATA (4,4',4"-tris[2-naphthyl(phenyl)amino]triphenylamine), Spiro-TAD, DNTPD, NPB, NPNPB, MeO-TPD, HAT-CN and/or TrisPcz (9,9'-diphenyl-6-(9-phenyl-9H-carbazol-3-yl)-9H,9'H-3,3'-bicarbazole). In addition, the HTL may comprise a p-doped layer, which may be composed of an inorganic or organic dopant in an organic hole-transporting matrix. Transition metal oxides such as vanadium oxide, molybdenum oxide or tungsten oxide may, for example, be used as inorganic dopant. Tetrafluorotetracyanoquinodimethane ($F_4$-TCNQ), copper-pentafluorobenzoate (Cu(I)pFBz) or transition metal complexes may, for example, be used as

organic dopant.

The EBL may, for example, comprise mCP (1,3-bis(carbazol-9-yl)benzene), TCTA, 2-TNATA, mCBP (3,3-di(9H-carbazol-9-yl)biphenyl), tris-Pcz, CzSi (9-(4-tert-Butylphenyl)-3,6-bis(triphenylsilyl)-9H-carbazole), and/or DCB (N,N'-dicarbazolyl-1,4-dimethylbenzene).

Adjacent to the hole transport layer (HTL), the light-emitting layer EML is typically located. The light-emitting layer EML comprises at least one light emitting molecule. Particularly, the EML comprises at least one light emitting molecule according to the invention E. In one embodiment, the light-emitting layer comprises only the organic molecules according to the invention. Typically, the EML additionally comprises one or more host materials H. For example, the host material H is selected from CBP (4,4'-Bis-(N-carbazolyl)-biphenyl), mCP, mCBP Sif87 (dibenzo[b,d]thiophen-2-yltriphenylsilane), CzSi, Sif88 (dibenzo[b,d]thiophen-2-yl)diphenylsilane), DPEPO (bis[2-(diphenylphosphino)phenyl] ether oxide), 9-[3-(dibenzofuran-2-yl)phenyl]-9H-carbazole, 9-[3-(dibenzofuran-2-yl)phenyl]-9H-carbazole, 9-[3-(dibenzothiophen-2-yl)phenyl]-9H-carbazole, 9-[3,5-bis(2-dibenzofuranyl)phenyl]-9H-carbazole, 9-[3,5-bis(2-dibenzothiophenyl)phenyl]-9H-carbazole, T2T (2,4,6-tris(biphenyl-3-yl)-1,3,5-triazine), T3T (2,4,6-tris(triphenyl-3-yl)-1,3,5-triazine) and/or TST (2,4,6-tris(9,9'-spirobifluorene-2-yl)-1,3,5-triazine). The host material H typically should be selected to exhibit first triplet (T1) and first singlet (S1) energy levels, which are energetically higher than the first triplet (T1) and first singlet (S1) energy levels of the organic molecule.

In one embodiment of the invention, the EML comprises a so-called mixed-host system with at least one hole-dominant host and one electron-dominant host. In a particular embodiment, the EML comprises exactly one light emitting organic molecule according to the invention and a mixed-host system comprising T2T as electron-dominant host and a host selected from CBP, mCP, mCBP, 9-[3-(dibenzofuran-2-yl)phenyl]-9H-carbazole, 9-[3-(dibenzofuran-2-yl)phenyl]-9H-carbazole, 9-[3-(dibenzothiophen-2-yl)phenyl]-9H-carbazole, 9-[3,5-bis(2-dibenzofuranyl)phenyl]-9H-carbazole and 9-[3,5-bis(2-dibenzothiophenyl)phenyl]-9H-carbazole as hole-dominant host. In a further embodiment the EML comprises 50-80 % by weight, preferably 60-75 % by weight of a host selected from CBP, mCP, mCBP, 9-[3-(dibenzofuran-2-yl)phenyl]-9H-carbazole, 9-[3-(dibenzofuran-2-yl)phenyl]-9H-carbazole, 9-[3-(dibenzothiophen-2-yl)phenyl]-9H-carbazole, 9-[3,5-bis(2-dibenzofuranyl)phenyl]-9H-carbazole and 9-[3,5-bis(2-dibenzothiophenyl)phenyl]-9H-carbazole; 10-45 % by weight, preferably 15-30 % by weight of T2T and 5-40 % by weight, preferably 10-30 % by weight of light emitting molecule according to the invention.

Adjacent to the light-emitting layer EML, an electron transport layer (ETL) may be located. Herein, any electron transporter may be used. Exemplarily, electron-poor compounds such as, e.g., benzimidazoles, pyridines, triazoles, oxadiazoles (e.g., 1,3,4-oxadiazole), phosphinoxides and sulfone, may be used. An electron transporter may also be a star-shaped heterocycle such as 1,3,5-tri(1-phenyl-1H-benzo[d]imidazol-2-yl)phenyl (TPBi). The ETL may comprise NBphen (2,9-bis(naphthalen-2-yl)-4,7-diphenyl-1,10-phenanthroline), $Alq_3$ (aluminum-tris(8-hydroxyquinoline)), TSPO1 (diphenyl-4-triphenylsilylphenyl-phosphinoxide), BPyTP2 (2,7-di(2,2'-bipyridin-5-yl)triphenyle), Sif87 (dibenzo[b,d]thiophen-2-yltriphenylsilane), Sif88 (dibenzo[b,d]thiophen-2-yl)diphenylsilane), BmPyPhB (1,3-bis[3,5-di(pyridin-3-yl)phenyl]benzene) and/or BTB (4,4'-bis-[2-(4,6-diphenyl-1,3,5-triazinyl)]-1,1'-biphenyl). Optionally, the ETL may be doped with materials such as Liq. The electron transport layer (ETL) may also block holes or a holeblocking layer (HBL) is introduced.

The HBL may, for example, comprise BCP (2,9-dimethyl-4,7-diphenyl-1,10-phenanthroline = bathocuproine), BAlq (bis(8-hydroxy-2-methylquinoline)-(4-phenylphenoxy)aluminum), NBphen (2,9-bis(naphthalen-2-yl)-4,7-diphenyl-1,10-phenanthroline), $Alq_3$ (aluminum-tris(8-hydroxyquinoline)), TSPO1 (diphenyl-4-triphenylsilylphenyl-phosphinoxide), T2T (2,4,6-tris(biphenyl-3-yl)-1,3,5-triazine), T3T (2,4,6-tris(triphenyl-3-yl)-1,3,5-triazine), TST (2,4,6-tris(9,9'-spirobifluorene-2-yl)-1,3,5-triazine), and/or TCB/TCP (1,3,5-tris(N-carbazolyl)benzol/ 1,3,5-tris(carbazol)-9-yl) benzene).

Adjacent to the electron transport layer (ETL), a cathode layer C may be located. The cathode layer C may, for example, comprise or may consist of a metal (e.g., Al, Au, Ag, Pt, Cu, Zn, Ni, Fe, Pb, LiF, Ca, Ba, Mg, In, W, or Pd) or a metal alloy. For practical reasons, the cathode layer may also consist of (essentially) intransparent metals such as Mg, Ca or Al. Alternatively or additionally, the cathode layer C may also comprise graphite and or carbon nanotubes (CNTs). Alternatively, the cathode layer C may also consist of nanoscalic silver wires.

An OLED may further, optionally, comprise a protection layer between the electron transport layer (ETL) and the cathode layer C (which may be designated as electron injection layer (EIL)). This layer may comprise lithium fluoride, cesium fluoride, silver, Liq (8-hydroxyquinolinolatolithium), $Li_2O$, $BaF_2$, MgO and/or NaF.

Optionally, the electron transport layer (ETL) and/or a hole blocking layer (HBL) may also comprise one or more host compounds H.

In order to modify the emission spectrum and/or the absorption spectrum of the light-emitting layer EML further, the light-emitting layer EML may further comprise one or more further emitter molecules F. Such an emitter molecule F may be any emitter molecule known in the art. Preferably such an emitter molecule F is a molecule with a structure differing from the structure of the molecules according to the invention E. The emitter molecule F may optionally be a TADF emitter. Alternatively, the emitter molecule F may optionally be a fluorescent and/or phosphorescent emitter molecule which is able to shift the emission spectrum and/or the absorption spectrum of the light-emitting layer EML. Exemplarily, the triplet and/or

singlet excitons may be transferred from the organic emitter molecule according to the invention to the emitter molecule F before relaxing to the ground state S0 by emitting light typically red-shifted in comparison to the light emitted by an organic molecule. Optionally, the emitter molecule F may also provoke two-photon effects (i.e., the absorption of two photons of half the energy of the absorption maximum).

Optionally, an optoelectronic device (e.g., an OLED) may, for example, be an essentially white optoelectronic device. For example, such white optoelectronic device may comprise at least one (deep) blue emitter molecule and one or more emitter molecules emitting green and/or red light. Then, there may also optionally be energy transmittance between two or more molecules as described above.

As used herein, if not defined more specifically in the particular context, the designation of the colors of emitted and/or absorbed light is as follows:

| | |
|---|---|
| violet: | wavelength range of >380-420 nm; |
| deep blue: | wavelength range of >420-480 nm; |
| sky blue: | wavelength range of >480-500 nm; |
| green: | wavelength range of >500-560 nm; |
| yellow: | wavelength range of >560-580 nm; |
| orange: | wavelength range of >580-620 nm; |
| red: | wavelength range of >620-800 nm. |

With respect to emitter molecules, such colors refer to the emission maximum. Therefore, for example, a deep blue emitter has an emission maximum in the range of from >420 to 480 nm, a sky blue emitter has an emission maximum in the range of from >480 to 500 nm, a green emitter has an emission maximum in a range of from >500 to 560 nm, a red emitter has an emission maximum in a range of from >620 to 800 nm.

A green emitter may preferably have an emission maximum between 500 and 560 nm, more preferably between 510 and 550 nm, and even more preferably between 520 and 540 nm. A further embodiment of the present invention relates to an OLED, which emits light with CIEx and CIEy color coordinates close to the CIEx (= 0.170) and CIEy (= 0.797) color coordinates of the primary color green (CIEx = 0.170 and CIEy = 0.797) as defined by ITU-R Recommendation BT.2020 (Rec. 2020) and thus is suited for the use in Ultra High Definition (UHD) displays, e.g. UHD-TVs. In this context, the term "close to" refers to the ranges of CIEx and CIEy coordinates provided at the end of this paragraph. In commercial applications, typically top-emitting (top-electrode is transparent) devices are used, whereas test devices as used throughout the present application represent bottom-emitting devices (bottom-electrode and substrate are transparent). Accordingly, a further aspect of the present invention relates to an OLED, whose emission exhibits a CIEx color coordinate of between 0.06 and 0.34, preferably between 0.07 and 0.29, more preferably between 0.09 and 0.24 or even more preferably between 0.12 and 0.22 or even between 0.14 and 0.19 and/ or a CIEy color coordinate of between 0.44 and 0.84, preferably between 0.55 and 0.83, more preferably between 0.65 and 0.82 or even more preferably between 0.70 and 0.81 or even between 0.75 and 0.8.

Accordingly, a further aspect of the present invention relates to an OLED, which exhibits an external quantum efficiency at 14500 cd/m$^2$ of more than 10%, more preferably of more than 13%, more preferably of more than 15%, even more preferably of more than 17% or even more than 20% and/or exhibits an emission maximum between 495 nm and 580 nm, preferably between 500 nm and 560 nm, more preferably between 510 nm and 550 nm, even more preferably between 515 nm and 540 nm

A deep blue emitter may preferably have an emission maximum of below 480 nm, more preferably below 470 nm, even more preferably below 465 nm or even below 460 nm. It will typically be above 420 nm, preferably above 430 nm, more preferably above 440 nm or even above 450 nm.

Accordingly, a further aspect of the present invention relates to an OLED, which exhibits an external quantum efficiency at 1000 cd/m$^2$ of more than 8 %, more preferably of more than 10 %, more preferably of more than 13 %, even more preferably of more than 15 % or even more than 20 % and/or exhibits an emission maximum between 420 nm and 500 nm, preferably between 430 nm and 490 nm, more preferably between 440 nm and 480 nm, even more preferably between 450 nm and 470 nm and/or exhibits a LT80 value at 500 cd/m$^2$ of more than 100 h, preferably more than 200 h, more preferably more than 400 h, even more preferably more than 750 h or even more than 1000 h. Accordingly, a further aspect of the present invention relates to an OLED, whose emission exhibits a CIEy color coordinate of less than 0.45, preferably less than 0.30, more preferably less than 0.20 or even more preferably less than 0.15 or even less than 0.10.

A further aspect of the present invention relates to an OLED, which emits light at a distinct color point. According to the present invention, the OLED emits light with a narrow emission band (small full width at half maximum (FWHM)). In one aspect, the OLED according to the invention emits light with a FWHM of the main emission peak of less than 0.30 eV, preferably less than 0.25 eV, more preferably less than 0.20 eV, even more preferably less than 0.19 eV or even less than

0.17 eV.

A further aspect of the present invention relates to an OLED, which emits light with CIEx and CIEy color coordinates close to the CIEx (= 0.131) and CIEy (= 0.046) color coordinates of the primary color blue (CIEx = 0.131 and CIEy = 0.046) as defined by ITU-R Recommendation BT.2020 (Rec. 2020) and thus is suited for the use in Ultra High Definition (UHD) displays, e.g. UHD-TVs. Accordingly, a further aspect of the present invention relates to an OLED, whose emission exhibits a CIEx color coordinate of between 0.02 and 0.30, preferably between 0.03 and 0.25, more preferably between 0.05 and 0.20 or even more preferably between 0.08 and 0.18 or even between 0.10 and 0.15 and/ or a CIEy color coordinate of between 0.00 and 0.45, preferably between 0.01 and 0.30, more preferably between 0.02 and 0.20 or even more preferably between 0.03 and 0.15 or even between 0.04 and 0.10.

In a further aspect, the invention relates to a method for producing an optoelectronic component. In this case an organic molecule of the invention is used.

The optoelectronic device, in particular the OLED according to the present invention can be fabricated by any means of vapor deposition and/ or liquid processing. Accordingly, at least one layer is

- prepared by means of a sublimation process,
- prepared by means of an organic vapor phase deposition process,
- prepared by means of a carrier gas sublimation process,
- solution processed or printed.

The methods used to fabricate the optoelectronic device, in particular the OLED according to the present invention are known in the art. The different layers are individually and successively deposited on a suitable substrate by means of subsequent deposition processes. The individual layers may be deposited using the same or differing deposition methods.

Vapor deposition processes, for example, comprise thermal (co)evaporation, chemical vapor deposition and physical vapor deposition. For active matrix OLED display, an AMOLED backplane is used as substrate. The individual layer may be processed from solutions or dispersions employing adequate solvents. Solution deposition process, for example, comprise spin coating, dip coating and jet printing. Liquid processing may optionally be carried out in an inert atmosphere (e.g., in a nitrogen atmosphere) and the solvent may be completely or partially removed by means known in the state of the art.

## Examples

General synthesis scheme I,
wherein $X^1 = X^{14}$, $X^2 = X^{13}$, $X^3 = X^{12}$, $X^4 = X^{11}$, $X^5 = X^{16}$, $X^6 = X^9$, and $X^7 = X^8$:

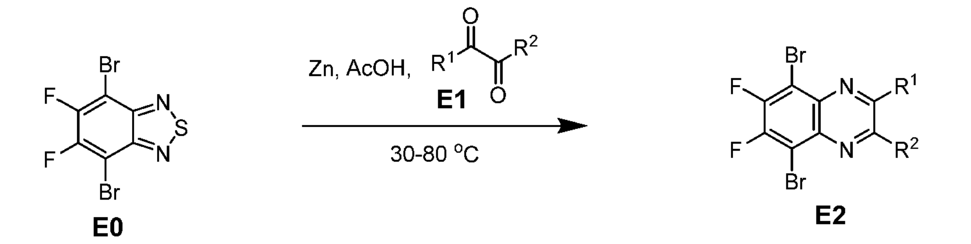


E0 E2

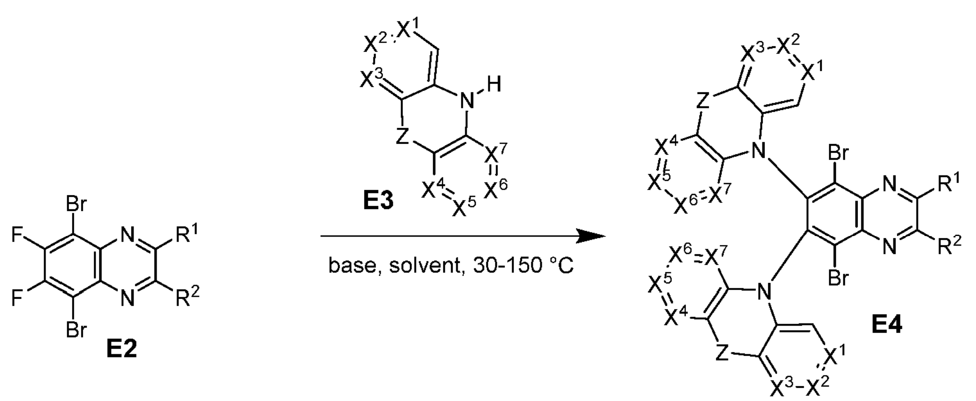


E2 E4

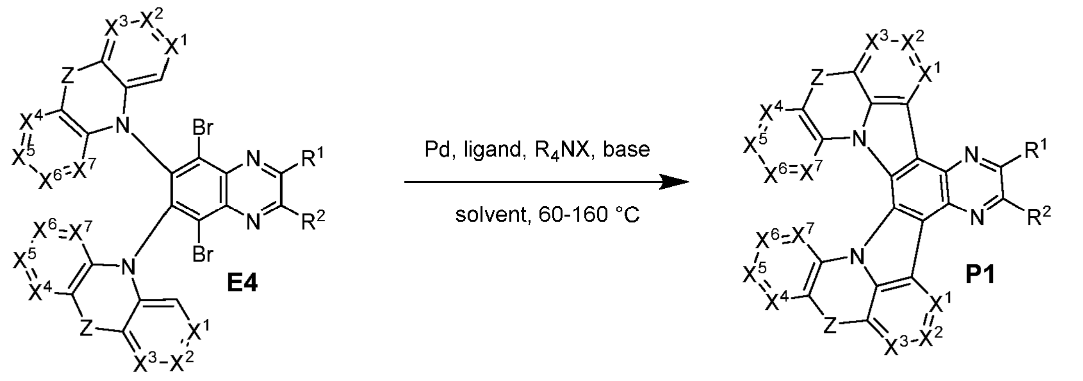


E4 P1

*General procedure for synthesis* ***AAV1*:**

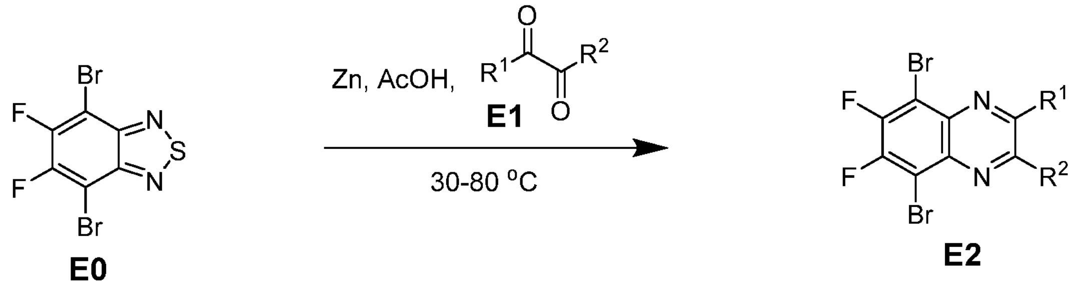


E0 E1 E2

In the first step acetic acid (50 eq) added to a flask which contains 4,7-dibromo-5,6-difluorobenzo[c][1,2,5] thiadiazole (**E0**; 1eq) and Zn (1.5eq) and the mixture stirred at 60 °C. Then 1,2-dione compounds **E1** were added to the obtained compound and the reaction was stirred at elevated temperature (30 °C or higher) to generate the corresponding 5,8-dibromo-6,7-difluoroquinoxaline derivatives **E2.** Variations in the amount of compounds and temperature could be employed.

*General procedure for synthesis* ***AAV2:***

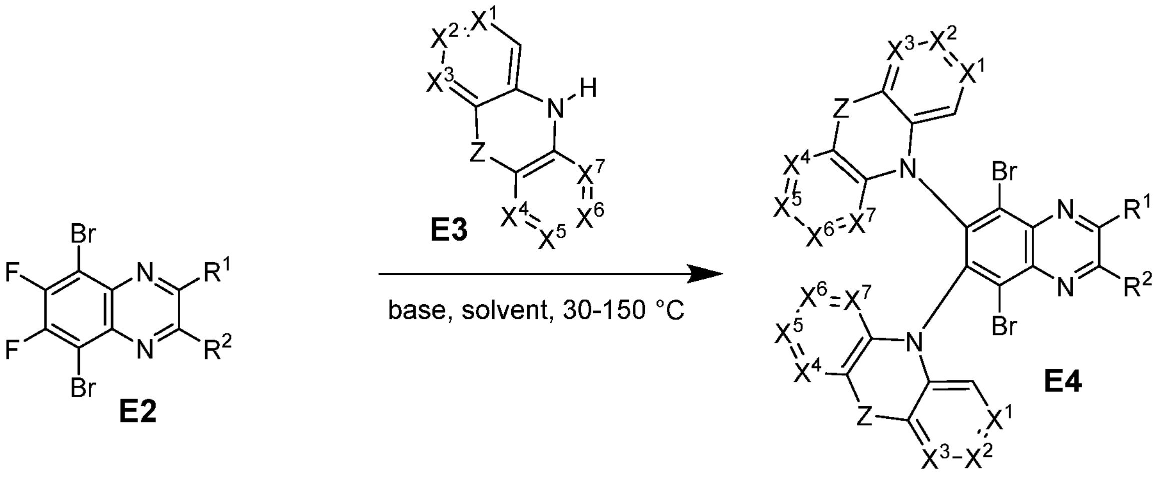


E2 E3 E4

The second step was a nucleophilic aromatic substitution reaction between **E2** (1eq) and secondary amines **E3** (2.3eq) in organic solvent (for example DMSO, DMF, NMP, DMAc, DME, EtOH, MeCN, Tol, MeCN, or DCM) in the presence of base (4 eq; for example $K_2CO_3$, $K_3PO_4$, NaH, NaOtBu, KOtBu, $Cs_2CO_3$, KOH, NaOH, LDA, LDEA) at elevated temperature (30 °C or higher, and overnight) to obtain di-substituted compound **E4.** Variations in the amount of

compounds, base, solvent, time and temperature could be employed.

*General procedure for synthesis* ***AAV3:***

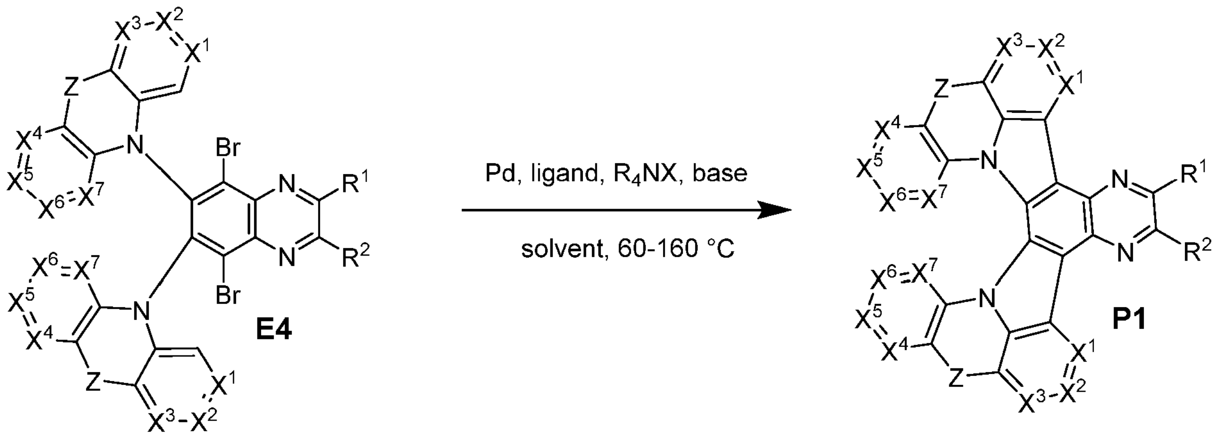


In the last step, C-H activation conducted in the presence of Pd" or Pd° catalyst (0.05eq; for example $Pd(OAc)_2$, $PdCl_2(PPh_3)_2$, $Pd(dppf)Cl_2$, $PdCl_2(PPh_3)_2$, $Pd(PPh_3)_4$), ligand (0.08eq; for example $PPh_3$, $PCy_3$, $PCy_3$-$HBF_4$, XPhos, S-Phos, R-Phos, xanphos, $(tBu)_3P$), quaternary ammonium salt (1eq; for example TBACI, TBAB, TBAI, TBAOH, TMAB, THACI, TOACI, tetrabutylammonium tetrafluoroborate, benzyltrimethylammonium bromide), base (5eq; for example $K_2CO_3$, $K_3PO_4$, NaH, NaO*t*Bu, KO*t*Bu, $Cs_2CO_3$, KOH, or NaOH), solvent (for example, DMAc, NMP, DMF, DME, MeCN, Tol, 1,4-dioxane, DMSO, EtOH, or THF) and at 140 °C to achieve organic molecules according to the invention, **P1.** Variations in the amount of compounds, phase catalyst, solvent, and temperature could be employed.

General synthesis scheme II

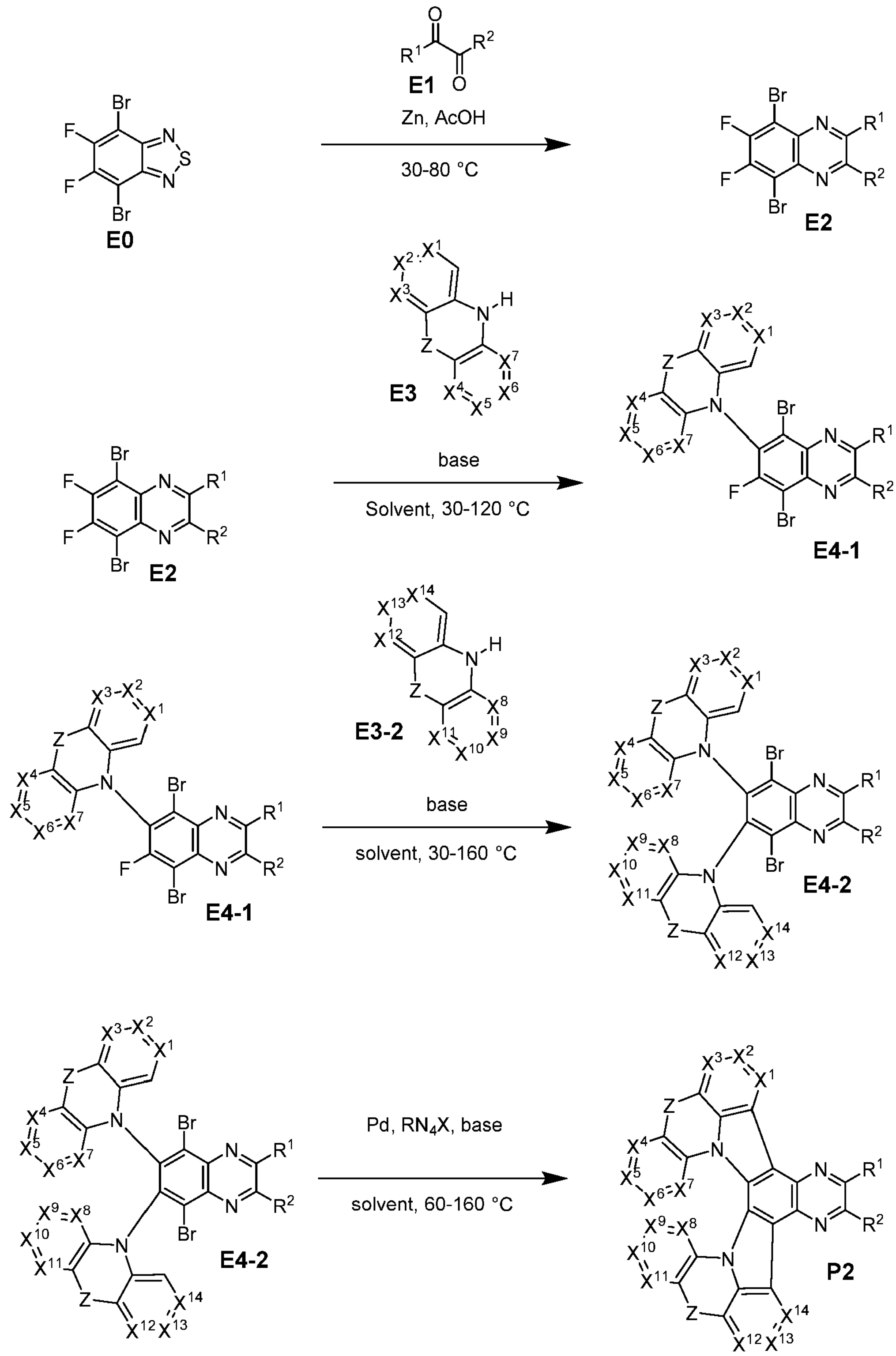


*General procedure for synthesis* ***AAV2-1*****:**

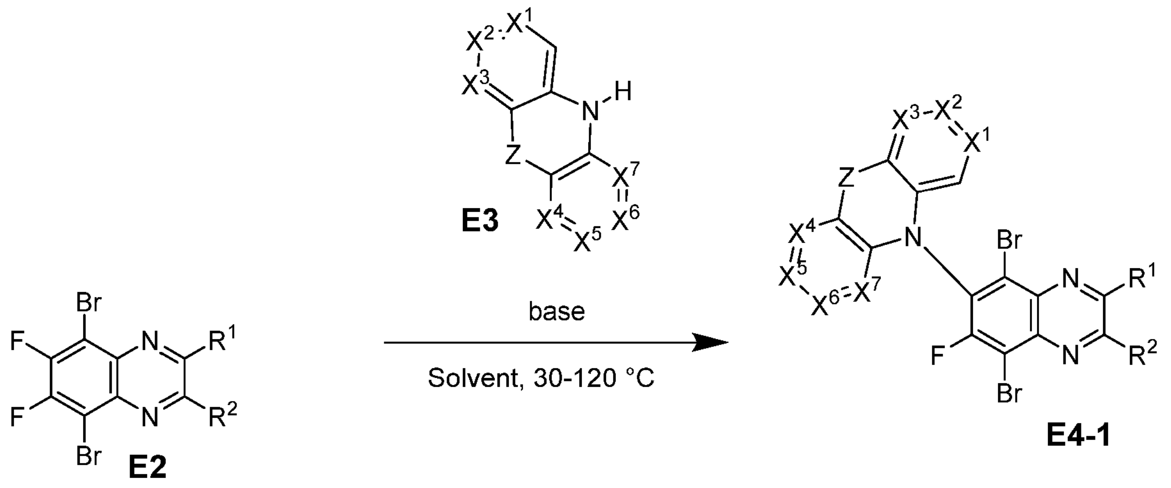


The synthesis of ***AAV2-1*** is carried out by reacting of **E2** (1.05eq), secondary amine **E3** (1eq) in the presence of base (1.5eq; for example $K_2CO_3$, $K_3PO_4$, NaH, NaOtBu, KOtBu, $Cs_2CO_3$, KOH, NaOH, LDA, or LDEA), and solvent (for example DMSO, DMF, NMP, DMAc, DME, EtOH, MeCN, Tol, MeCN, or DCM) at ambient temperature up to 120°C.

*General procedure for synthesis **AAV2-2:***

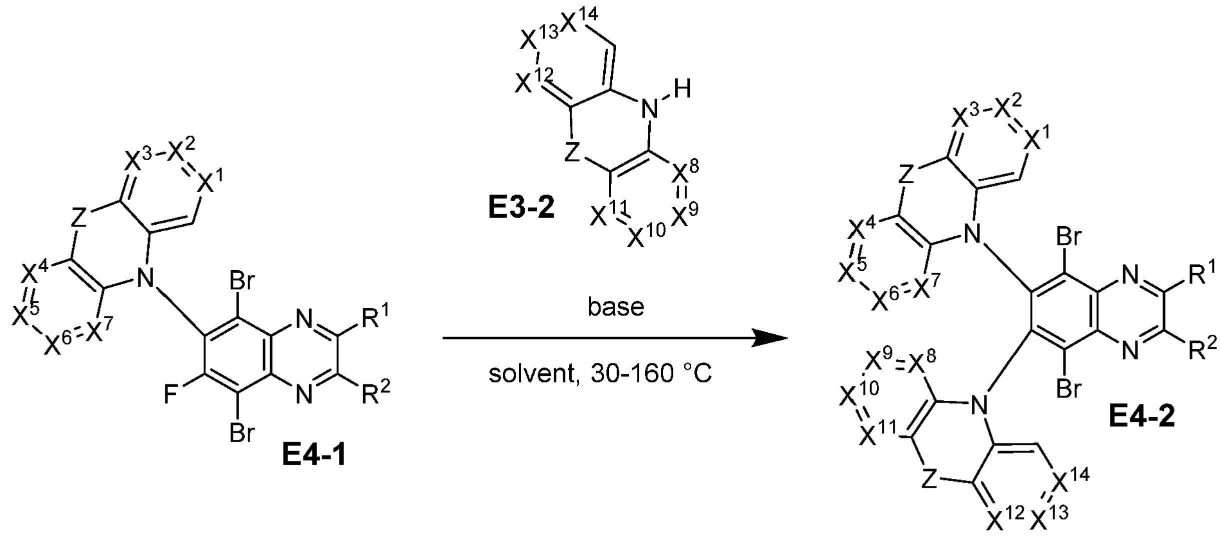


The synthesis of **E4-2** is carried out from reaction of **E4-1** and secondary amine **E3-2.** To a flask containing **E4-1** (1eq), base (1.5eq), **E3-2** (1.2eq) was added in a solvent (for example DMSO, DMF, NMP, DMAc, DME, EtOH, MeCN, Tol, MeCN, or DCM) and the reaction mixture stirred at elevated temperature (50 °C or higher) for 12 hours. Variations in the amount of compounds, base, solvent, and temperature could be employed.

*General procedure for synthesis **AAV2-3:***

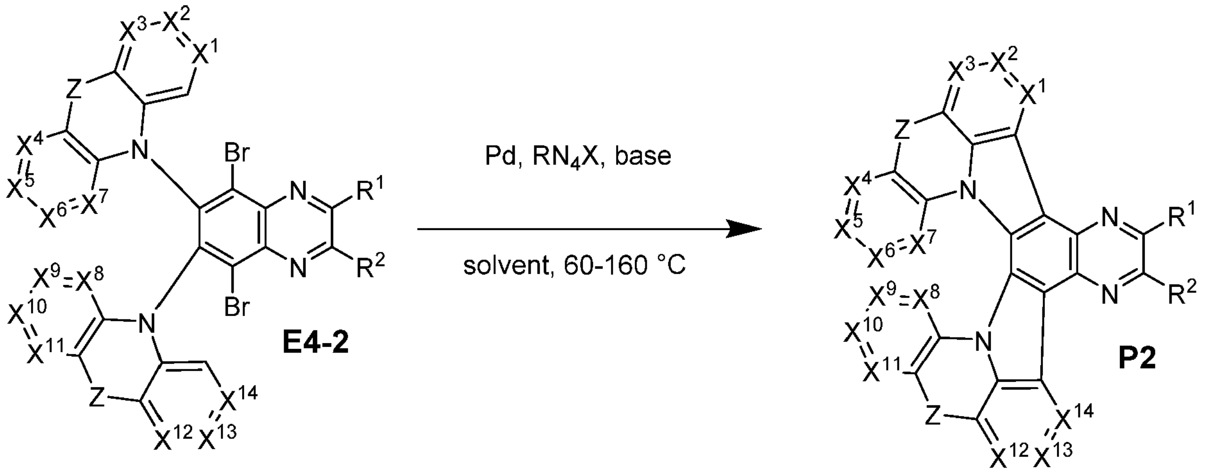


E4-2 P2

To 1eq of **E4-2,** palladium catalyst (0.05eq; for example $Pd(OAc)_2$, $PdCl_2(PPh_3)_2$, $Pd(dppf)Cl_2$, $PdCl_2(PPh_3)_2$, $Pd(PPh_3)_4$), ligand (0.08eq; for example $PPh_3$, $PCy_3$, $PCy_3$-$HBF_4$, XPhos, S-Phos, R-Phos, xanphos, $(tBu)_3P$), quaternary ammonium salt (1eq; for example TBACl, TBAB, TBAI, TBAOH, TMAB, THACl, TOACl, tetrabutylammonium tetrafluoroborate, benzyltrimethylammonium bromide), base (5eq; for example $K_2CO_3$, $K_3PO_4$, NaH, NaOtBu, KOtBu, $Cs_2CO_3$, KOH, NaOH, LDA, LDEA) were added to a solvent (for example MAc, NMP, DMF, DME, MeCN, Tol, 1,4-dioxane, DMSO, THF, EtOH) and at elevated temperatures (60-160 °C). Variations in the amount of compounds, phase catalyst, solvent, and temperature could be employed.

General synthesis scheme III,
wherein $X^1 = X^{14}$, $X^2 = X^{13}$, $X^3 = X^{12}$, $X^4 = X^{11}$, $X^5 = X^{10}$, $X^6 = X^9$, and $X^7 = X^8$:

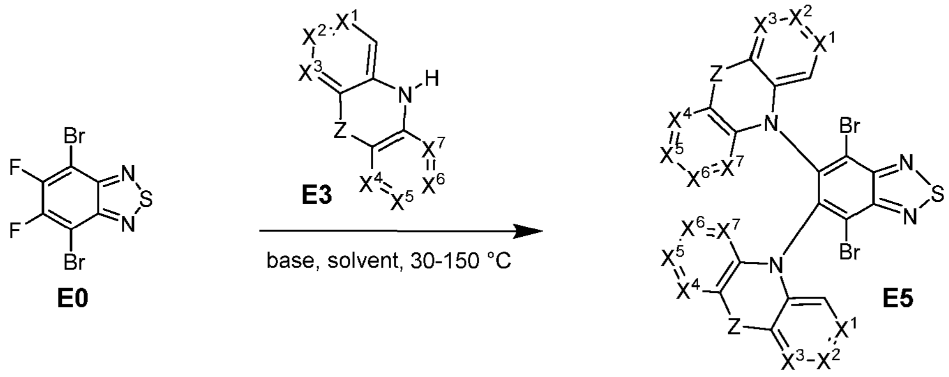


E0 E3 E5

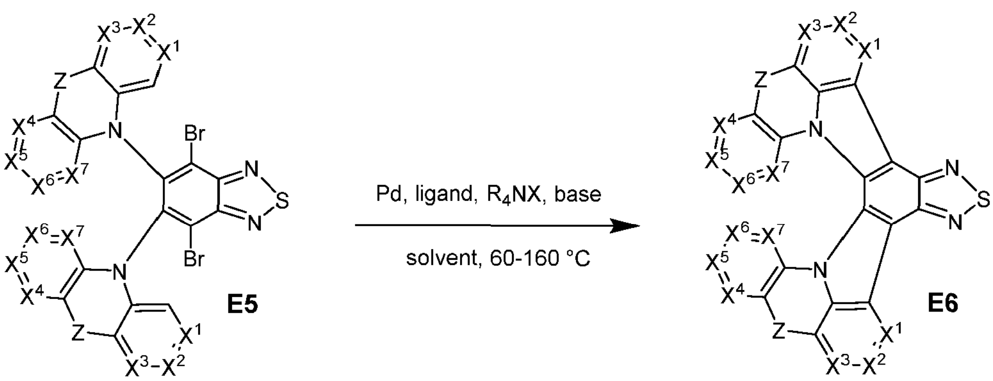


E5 E6

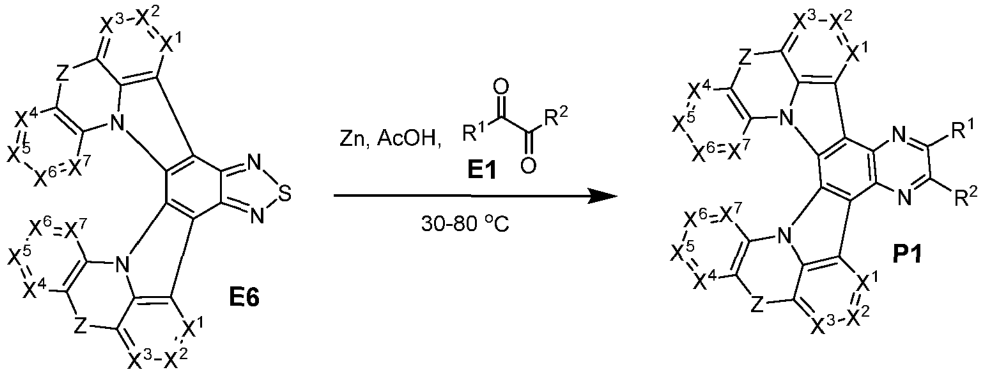


E6

P1

*General procedure for synthesis **AAV3-1:***

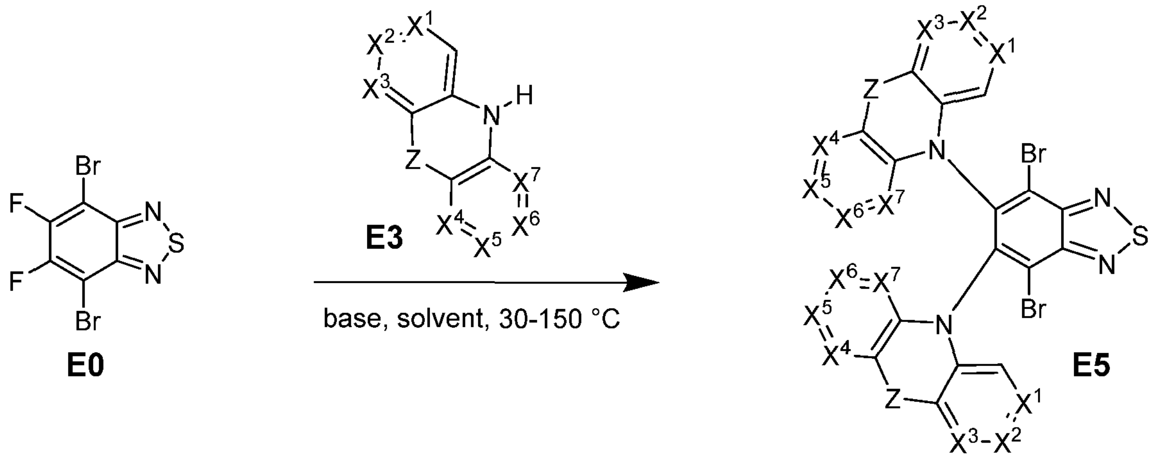


E0

E5

The first step was a nucleophilic aromatic substitution reaction between 4,7-dibromo-5,6-difluorobenzo[c][1,2,5] thiadiazole (**E0**; 1eq) and secondary amines **E3** (2.1eq) in organic solvent (for example DMSO, DMF, NMP, DMAc, DME, EtOH, MeCN, Tol, MeCN, or DCM) in the presence of base (4 eq; for example $K_2CO_3$, $K_3PO_4$, NaH, NaOtBu, KOtBu, $Cs_2CO_3$, KOH, NaOH, LDA, LDEA) at elevated temperature (30 °C or higher, and overnight) to obtain di-substituted compound **E5.** Variations in the amount of compounds, base, solvent, time and temperature may be employed.

*General procedure for synthesis **AAV3-2:***

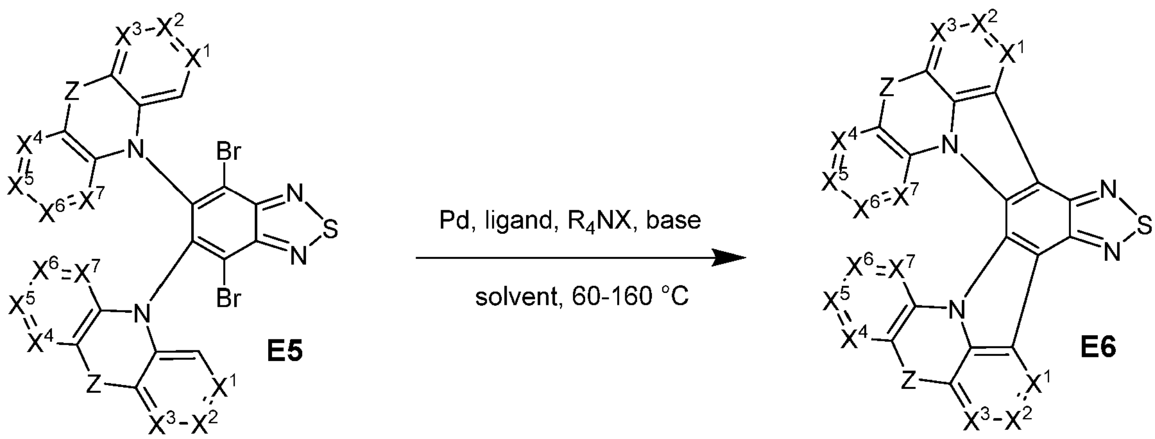


E5

E6

In the second step, C-H activation of **E5** (1eq) conducted in the presence of Pd" or Pd° catalyst (0.15eq; for example $Pd(OAc)_2$, $PdCl_2(PPh_3)_2$, $Pd(dppf)Cl_2$, $PdCl_2(PPh_3)_2$, $Pd(PPh_3)_4$), ligand (0.4eq; for example $PPh_3$, $PCy_3$, $PCy_3$-$HBF_4$, XPhos, S-Phos, R-Phos, xanphos, $(tBu)_3P$), quaternary ammonium salt (1eq; for example TBACI, TBAB, TBAI, TBAOH, TMAB, THACI, TOACI, tetrabutylammonium tetrafluoroborate, benzyltrimethylammonium bromide), base (5eq; for example $K_2CO_3$, $K_3PO_4$, NaH, NaO*t*Bu, KO*t*Bu, $Cs_2CO_3$, KOH, or NaOH), solvent (for example, DMAc, NMP, DMF, DME, MeCN, Tol, 1,4-dioxane, DMSO, EtOH, or THF) and at elevated temperature (60 °C or higher, and overnight) to achieve **E6.** Variations in the amount of compounds, phase catalyst, solvent, and temperature could be employed.

*General procedure for synthesis **AAV3-3:***

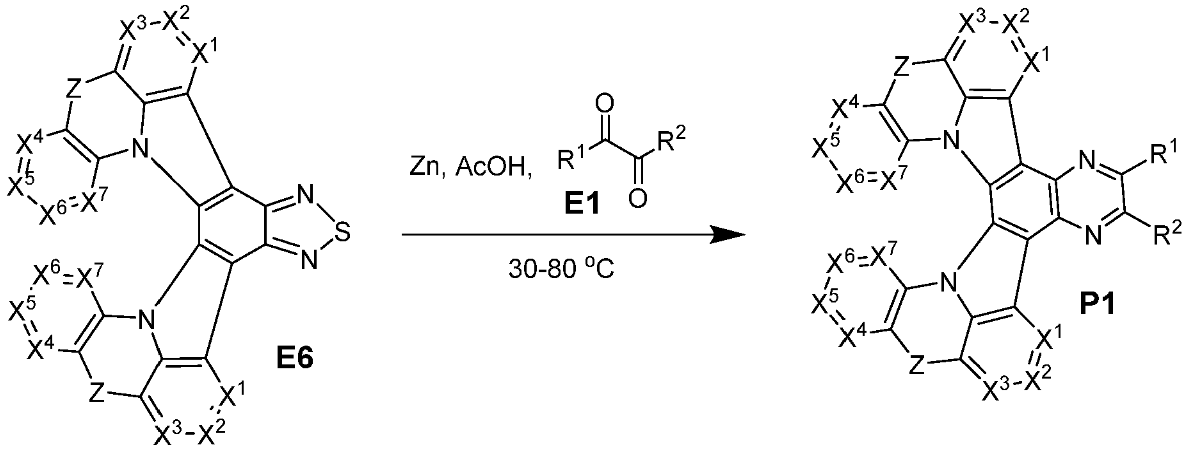


In the last step acetic acid (50 eq) added to a flask which contains **E6** (1eq) and Zn (10eq) and the mixture stirred at 60 °C. Then 1,2-dione compounds **E1** were added to the obtained compound and the reaction was stirred at elevated temperature (30 °C or higher) to generate the corresponding organic molecules according to the invention, **P1.** Variations in the amount of compounds and temperature could be employed.

*Cyclic voltammetry*

Cyclic voltammograms are measured from solutions having concentration of $10^{-3}$ mol/L of the organic molecules in dichloromethane or a suitable solvent and a suitable supporting electrolyte (e.g. 0.1 mol/L of tetrabutylammonium hexafluorophosphate). The measurements are conducted at room temperature under nitrogen atmosphere with a three-electrode assembly (Working and counter electrodes: Pt wire, reference electrode: Pt wire) and calibrated using $FeCp_2/FeCp_2^+$ as internal standard. The HOMO data was corrected using ferrocene as internal standard against a saturated calomel electrode (SCE).

*Density functional theory calculation*

Molecular structures are optimized employing the BP86 functional and the resolution of identity approach (RI). Excitation energies are calculated using the (BP86) optimized structures employing Time-Dependent DFT (TD-DFT) methods. Orbital and excited state energies are calculated with the B3LYP functional. Def2-SVP basis sets (and a m4-grid for numerical integration are used. The Turbomole program package is used for all calculations.

*Photophysical measurements*

Sample pretreatment: Spin-coating
Apparatus: Spin150, SPS euro.
The sample concentration is 0.2 mg/ml, dissolved in Toluene/DCM.
Program: 7 ) 30 sec. at 2000 U/min. After coating, the films are dried at 70 °C for 1 min.
Fluorescence Spectroscopy and Phosphorescence Spectroscopy

For the analysis of Phosphorescence and Photoluminescence spectroscopy a fluorescence spectrometer "Fluoromax 4P" from Horiba is used.
Time-resolved PL spectroscopy in the $\mu$s-range and ns-range (FS5)

Time-resolved PL measurements were performed on a FS5 fluorescence spectrometer from Edinburgh Instruments. Compared to measurements on the HORIBA setup, better light gathering allows for an optimized signal to noise ratio, which favors the FS5 system especially for transient PL measurements of delayed fluorescence characteristics. The FS5 consists of a xenon lamp providing a broad spectrum. The continuous light source is a 150W xenon arc lamp, selected wavelengths are chosen by a Czerny-Turner monochromator, which is also used to set specific emission wavelengths. The sample emission is directed towards a sensitive R928P photomultiplier tube (PMT), allowing the detection of single photons with a peak quantum efficiency of up to 25 % in the spectral range between 200 nm to 870 nm. The detector is a temperature stabilized PMT, providing dark counts below 300 cps (counts per second). Finally, to determine the transient decay lifetime of the delayed fluorescence, a tail fit using three exponential functions is applied. By weighting the specific lifetimes $\tau_i$ with their corresponding amplitudes $A_i$,

$$\tau_{\mathrm{DF}} = \sum\nolimits_{i=1}^{3} \frac{A_i \tau_i}{A_i}$$

the delayed fluorescence lifetime $\tau_{\mathrm{DF}}$ is determined.

Photoluminescence quantum yield measurements

For photoluminescence quantum yield (PLQY) measurements an *Absolute PL Quantum Yield Measurement C9920-03G* system (*Hamamatsu Photonics*) is used. Quantum yields and CIE coordinates are determined using the software U6039-05 version 3.6.0.
Emission maxima are given in nm, quantum yields $\Phi$ in % and CIE coordinates as x,y values. PLQY is determined using the following protocol:

1) Quality assurance: Anthracene in ethanol (known concentration) is used as reference
2) Excitation wavelength: the absorption maximum of the organic molecule is determined and the molecule is excited using this wavelength
3) Measurement
Quantum yields are measured, for sample, of solutions or films under nitrogen atmosphere. The yield is calculated using the equation:

$$\Phi_{PL} = \frac{n_{photon}, emitted}{n_{photon}, absorbed} = \frac{\int \frac{\lambda}{hc}\left[Int_{emitted}^{sample}(\lambda) - Int_{absorbed}^{sample}(\lambda)\right] d\lambda}{\int \frac{\lambda}{hc}\left[Int_{emitted}^{reference}(\lambda) - Int_{absorbed}^{reference}(\lambda)\right] d\lambda}$$

wherein $n_{photon}$ denotes the photon count and Int. the intensity.

Production and characterization of optoelectronic devices

Optoelectronic devices, such as OLED devices, comprising organic molecules according to the invention can be produced via vacuum-deposition methods. If a layer contains more than one compound, the weight-percentage of one or more compounds is given in %. The total weight-percentage values amount to 100 %, thus if a value is not given, the fraction of this compound equals to the difference between the given values and 100 %.
The not fully optimized OLEDs are characterized using standard methods and measuring electroluminescence spectra, the external quantum efficiency (in %) in dependency on the intensity, calculated using the light detected by the photodiode, and the current. The OLED device lifetime is extracted from the change of the luminance during operation at constant current density. The LT50 value corresponds to the time, where the measured luminance decreased to 50 % of the initial luminance, analogously LT80 corresponds to the time point, at which the measured luminance decreased to 80 % of the initial luminance, LT 95 to the time point, at which the measured luminance decreased to 95 % of the initial luminance etc.
Accelerated lifetime measurements are performed (e.g. applying increased current densities). For example, LT80 values at 500 cd/m$^2$ are determined using the following equation:

$$\mathrm{LT80}\left(500\frac{cd^2}{m^2}\right) = \mathrm{LT80}(L_0)\left(\frac{L_0}{500\frac{cd^2}{m^2}}\right)^{1.6}$$

wherein $L_0$ denotes the initial luminance at the applied current density.

The values correspond to the average of several pixels (typically two to eight), the standard deviation between these pixels is given.

HPLC-MS

HPLC-MS analysis is performed on an HPLC by Agilent (1260 series) with MS-detector (Thermo LTQ XL).

For example, a typical HPLC method is as follows: a reverse phase column 3.0 mm x 100 mm, particle size 2.7 μm from Agilent *(Poroshell 120EC-C18, 3.0 x 100 mm, 2.7 μm HPLC column)* is used in the HPLC. The HPLC-MS measurements are performed at room temperature (rt) following gradients

| Flow rate [ml/min] | Time [min] | A[%] | B[%] | C[%] |
|---|---|---|---|---|
| 1.5 | 30 | 40 | 40 | 30 |
| 1.5 | 45 | 10 | 10 | 80 |
| 1.5 | 50 | 40 | 10 | 80 |
| 1.5 | 51 | 30 | 40 | 30 |
| 1.5 | 55 | 30 | 10 | 30 |

using the following solvent mixtures containing 0.1% formic acid:

| | | |
|---|---|---|
| Solvent A: | $H_2O$ (10%) | MeCN (90%) |
| Solvent B: | $H_2O$ (90%) | MeCN (10%) |
| Solvent C: | THF (50%) | MeCN (50%) |

An injection volume of 2 μL from a solution with a concentration of 0.5 mg/mL of the analyte is taken for the measurements.

Ionization of the probe is performed using an atmospheric pressure chemical ionization (APCI) source either in positive (APCI +) or negative (APCI -) ionization mode or an atmospheric pressure photoionization (APPI) source.

Example 1

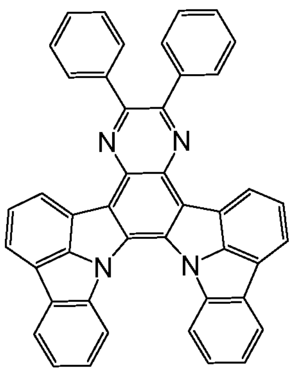

Example 1 was synthesized according to

***AAV1*** (69% yield), wherein benzil [134-81-6] was used as E1, reaction temperature set to 40 °C and reaction time of 16

hours;

**AAV2** (82% yield), wherein carbazole [201-696-0] was used as E3, DMF as solvent, potassium carbonate [584-08-7] as base, reaction temperature set to 80 °C and reaction time of 24 h; and

**AAV3** (80% yield), wherein $Pd(OAC)_2$ [3375-31-3], $PPh_3$ [603-35-0], tetrabutylammonium bromide [1643-19-2 were used as catalyst system, potassium carbonate [584-08-7] as base, N,N-dimethyl acetamide as a solvent, reaction temperature set to 140°C and reaction time of 24 h.

Figure 1 depicts the emission spectrum of example 1 (0,001 mg/ml in toluene). The emission maximum ($\lambda_{max}$) is at 461 nm. The photoluminescence quantum yield (PLQY) is 68%, the full width at half maximum (FWHM) is 0.22 eV. The resulting $CIE_x$ coordinate is 0.13 and the $CIE_y$ coordinate is 0.15.

Example 2

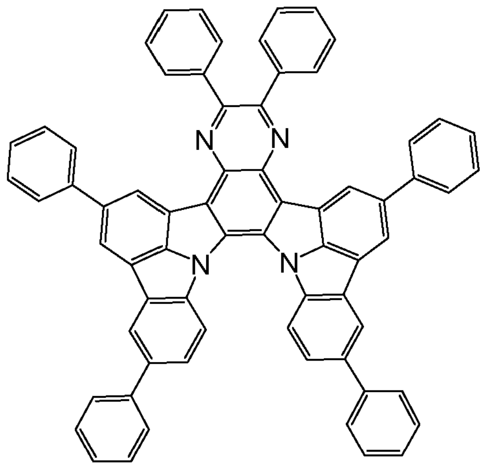

Example 2 was synthesized according to

***AAV1*** (69% yield), wherein benzil [134-81-6] was used as E1, reaction temperature set to 40 °C and reaction time of 16 hours;

**AAV2** (96% yield), wherein 3,6-diphenyl-9H-carbazole [56525-79-2] was used as E3, DMF as solvent, potassium carbonate [584-08-7] as base, reaction temperature set to 80 °C and reaction time of 24 h; and

**AAV3** (73% yield), wherein $Pd(OAC)_2$ [3375-31-3], $PPh_3$ [603-35-0], tetrabutylammonium bromide [1643-19-2 were used as catalyst system, potassium carbonate [584-08-7] as base, N,N-dimethyl acetamide as a solvent, reaction temperature set to 140 °C and reaction time of 36 h.

The emission spectrum of example 2 (0.001 mg/ml in DCM) has an emission maximum ($\lambda_{max}$) at 477 nm. The photoluminescence quantum yield (PLQY) is 70%, the full width at half maximum (FWHM) is 0.21 eV. The resulting $CIE_x$ coordinate is 0.129 and the $CIE_y$ coordinate is 0.344.

Example 3

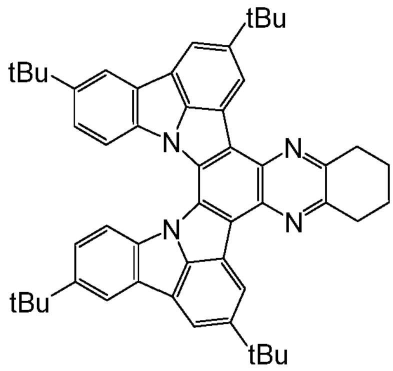

Example 3 was synthesized according to

***AAV3-1*** (37% yield), wherein 3,6-Di-tert-butylcarbazole [37500-95-1] was used as E3, DMF as solvent, potassium carbonate [584-08-7] as base, reaction temperature set to 100 °C and reaction time of 100 h; and

**AAV3-2** (54% yield), wherein Pd(OAC)$_2$ [3375-31-3], PPh$_3$ [603-35-0], tetrabutylammonium bromide [1643-19-2] were used as catalyst system, potassium carbonate [584-08-7] as base, N,N-dimethyl acetamide as a solvent, reaction temperature set to 140 °C and reaction time of 3 h; and

AAV3-3 (22% yield), wherein cyclohexane-1,2-dione [765-87-7] was used as E1, reaction temperature set to 40 °C and reaction time of 11 hours.

The emission spectrum of example 3 (0.001 mg/ml in toluene) has an emission maximum ($\lambda_{max}$) at 450 nm. The photoluminescence quantum yield (PLQY) is 57%, the full width at half maximum (FWHM) is 0.15 eV. The resulting CIE$_x$ coordinate is 0.139 and the CIE$_y$ coordinate is 0.104.

Example 4

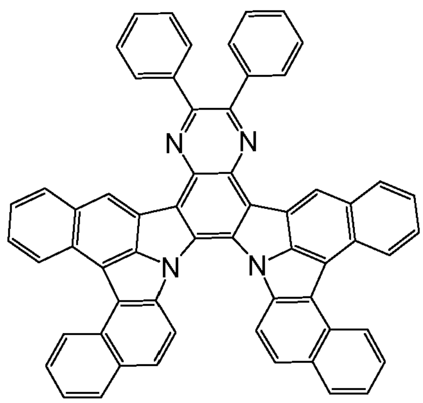

Example **4** was synthesized according to

***AAV1*** (69% yield), wherein benzil [134-81-6] was used as **E1,** reaction temperature set to 40 °C and reaction time of 16 hours;

**AAV2** (58% yield), wherein 7H-dibenzo[c,g]carbazole [194-59-2] was used as E3, DMF as solvent, potassium carbonate [584-08-7] as base, reaction temperature set to 100 °C and reaction time of 24 h; and

**AAV3** (21% yield), wherein Pd(OAC)$_2$ [3375-31-3], PPh$_3$ [603-35-0], tetrabutylammonium bromide [1643-19-2 were used as catalyst system, potassium carbonate [584-08-7] as base, N,N-dimethyl acetamide as a solvent, reaction temperature set to 140°C and reaction time of 72 h.

The emission spectrum of example 4 (0.001 mg/ml in toluene) has an emission maximum ($\lambda_{max}$) at 501 nm. The full width at half maximum (FWHM) is 0.13 eV. The resulting $CIE_x$ coordinate is 0.21 and the $CIE_y$ coordinate is 0.59.

**Additional Examples of Organic Molecules of the Invention**

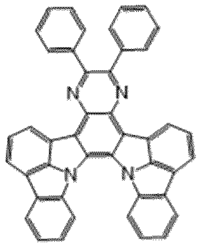
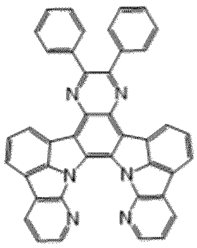
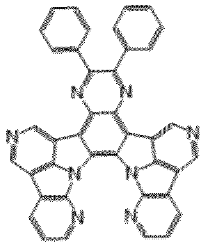
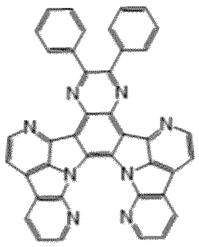
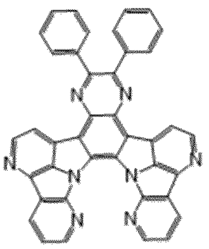

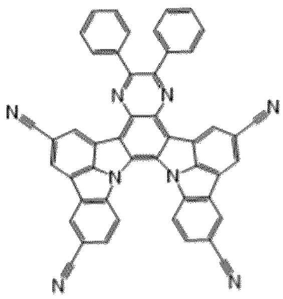
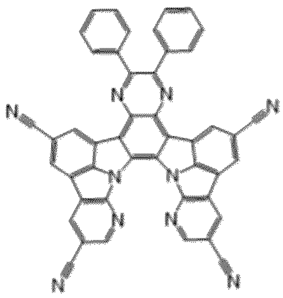
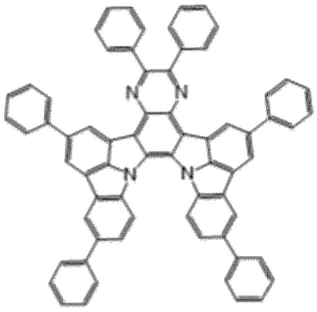
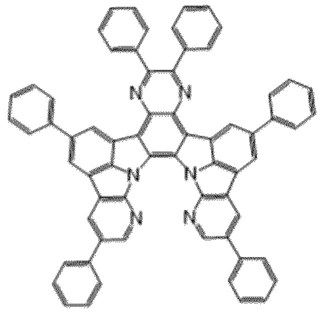

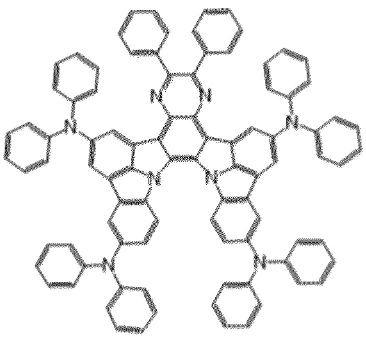
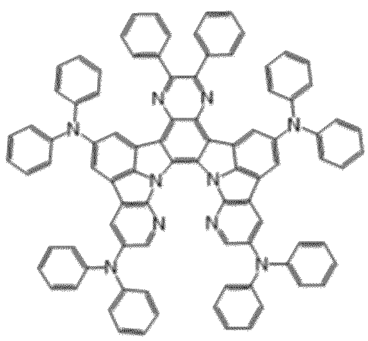
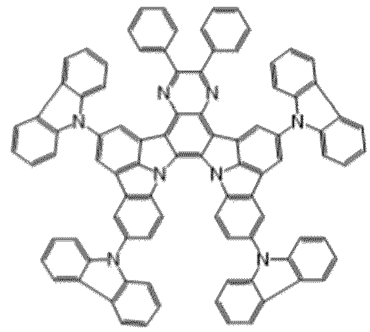

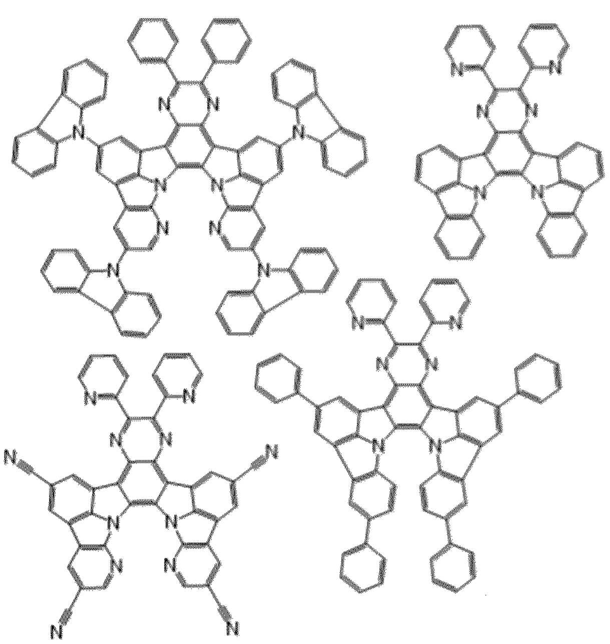
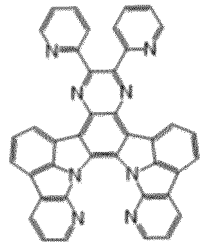
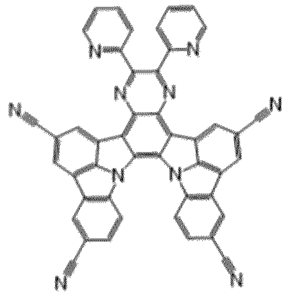

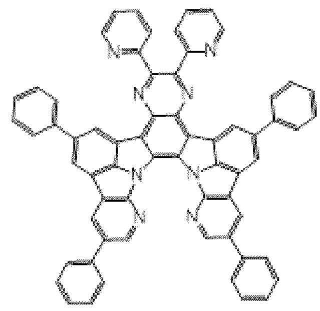

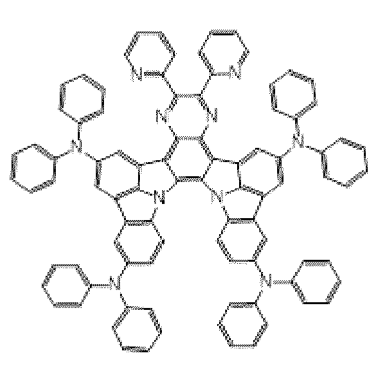
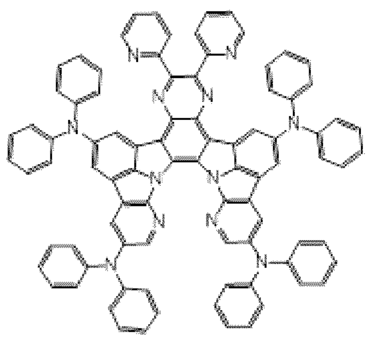
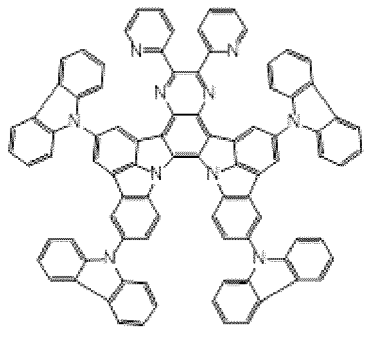

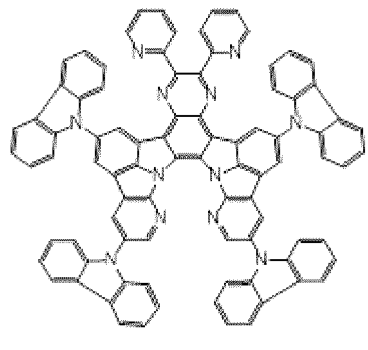
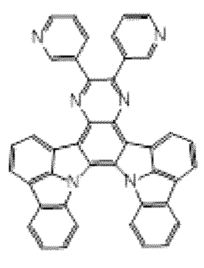
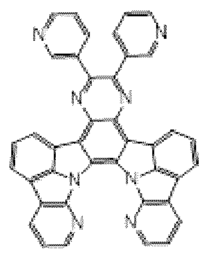
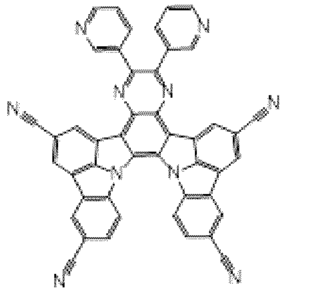

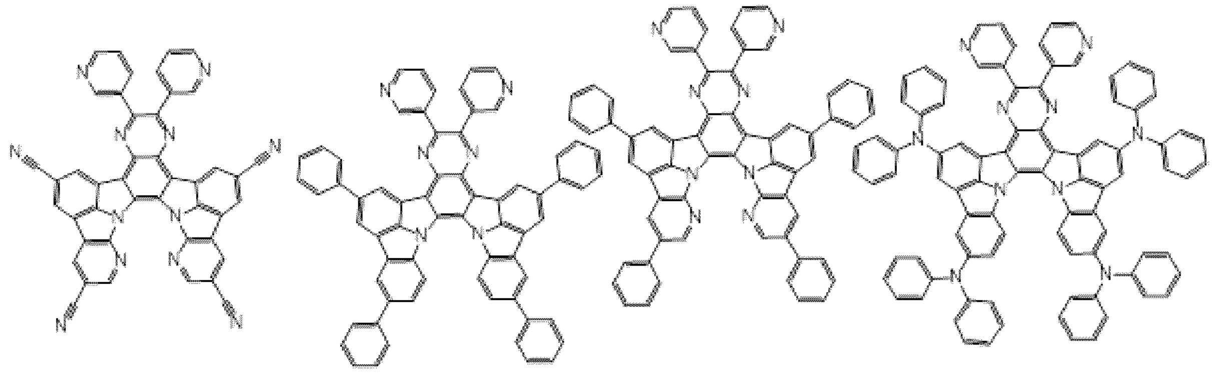

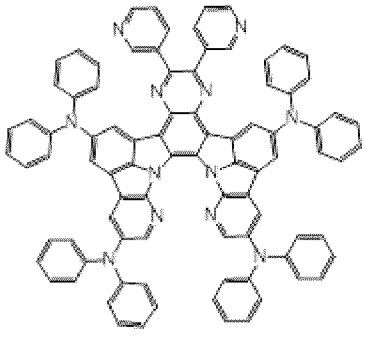

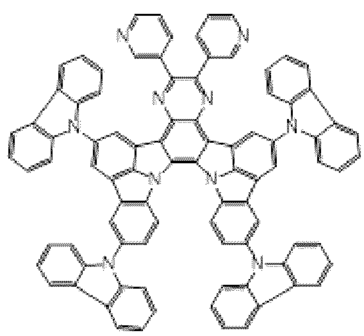

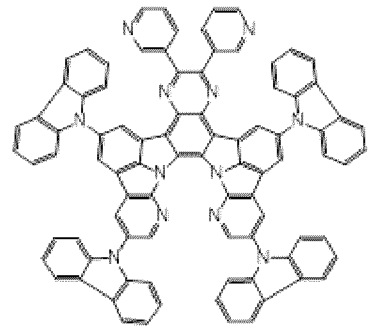

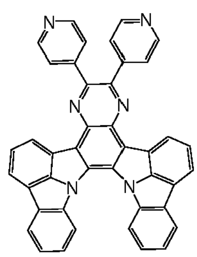

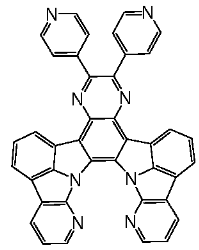

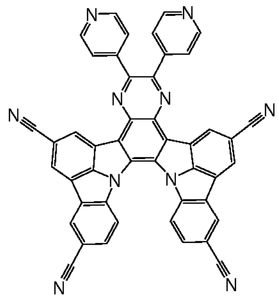

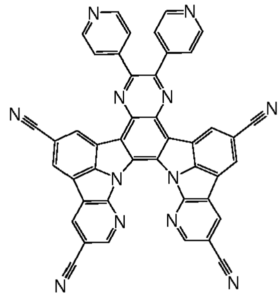

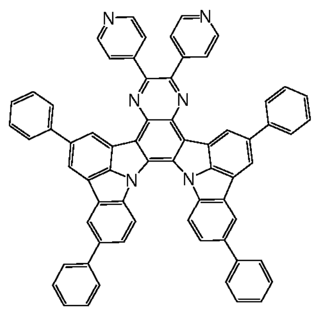

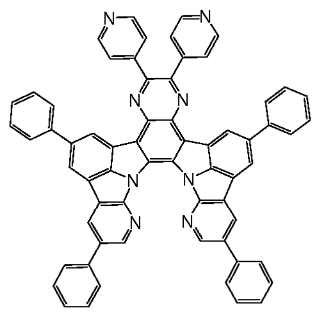

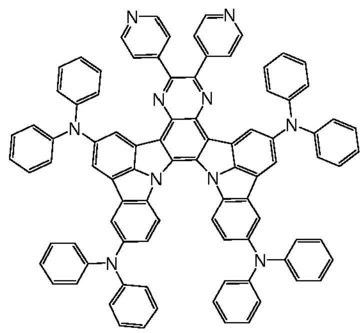

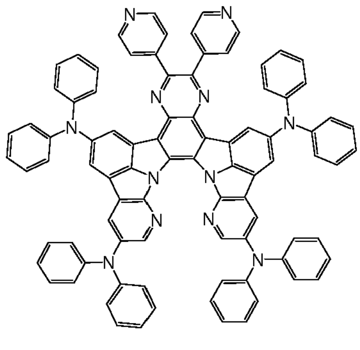
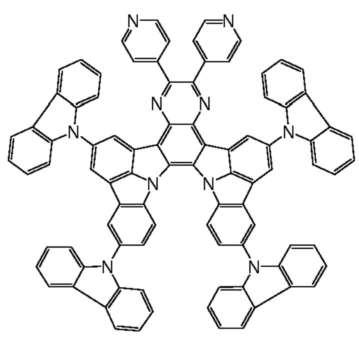
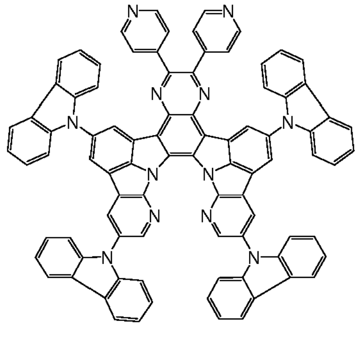

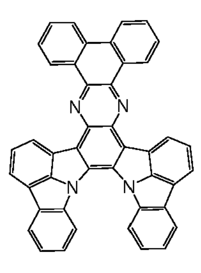
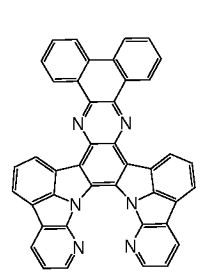
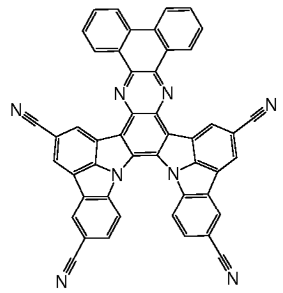
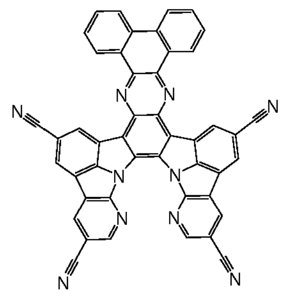

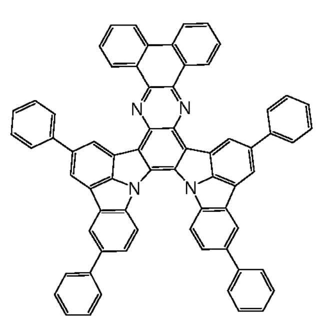
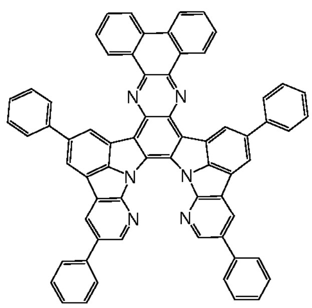
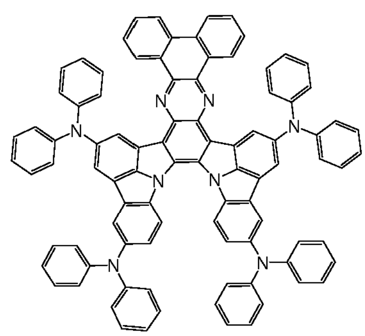

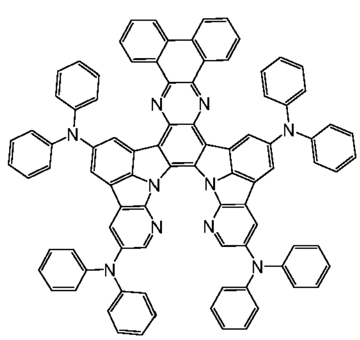
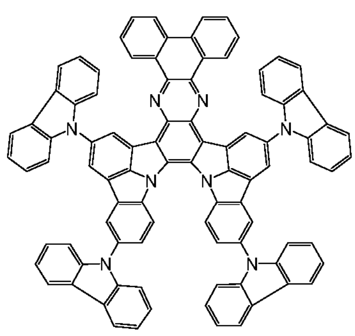
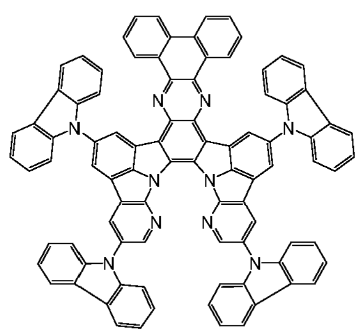

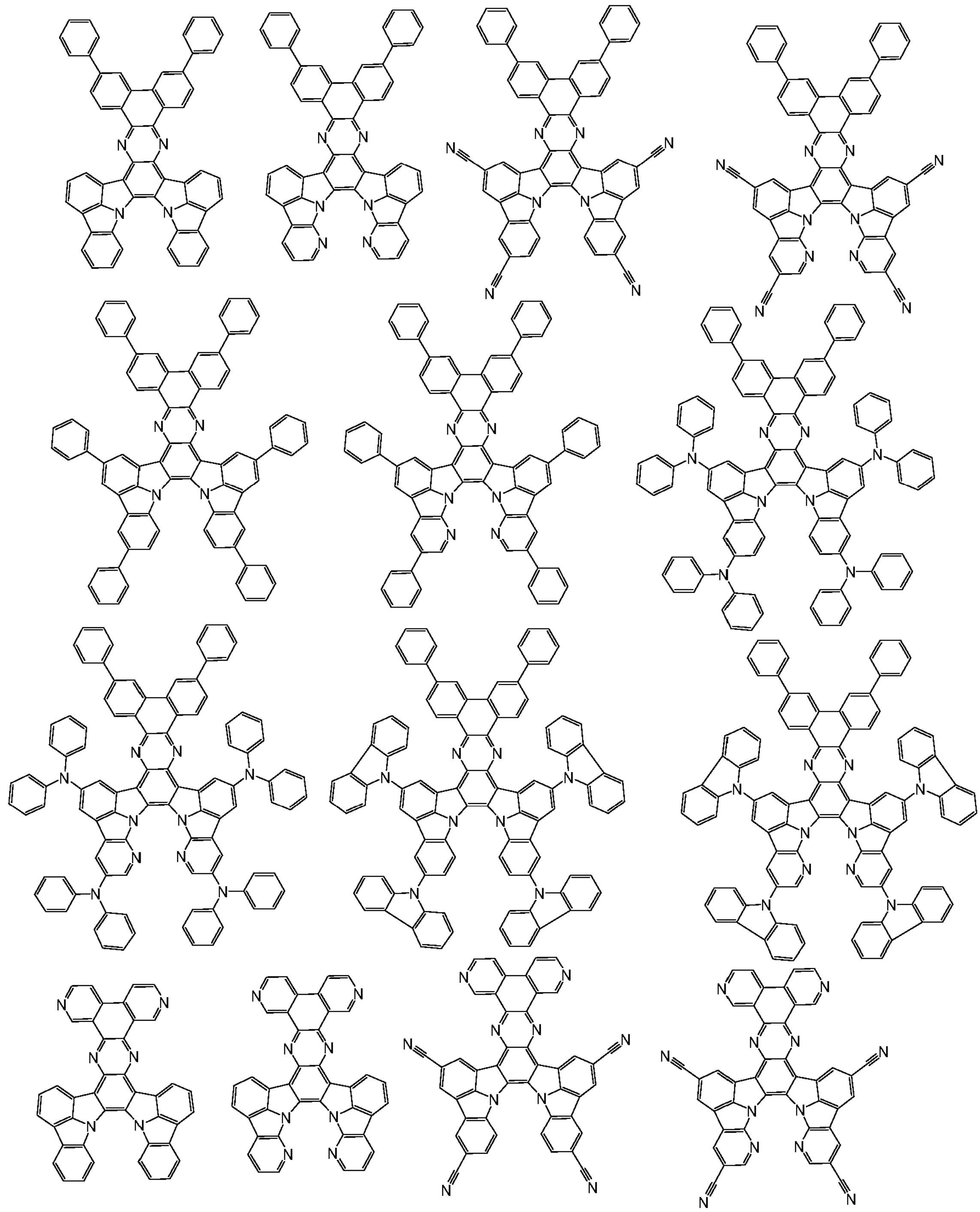

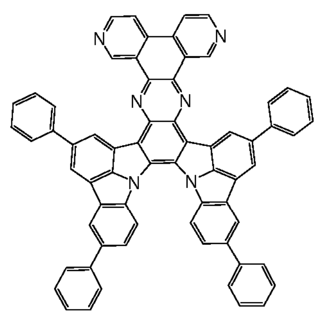

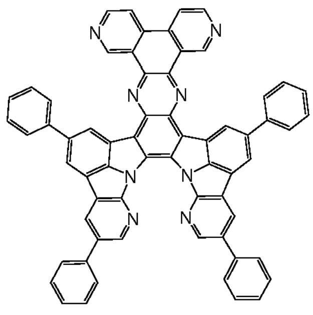

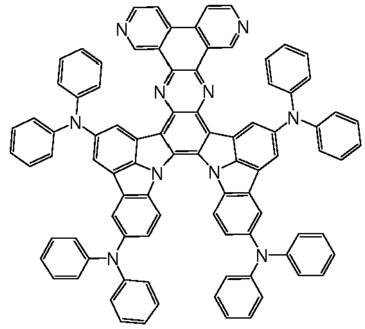

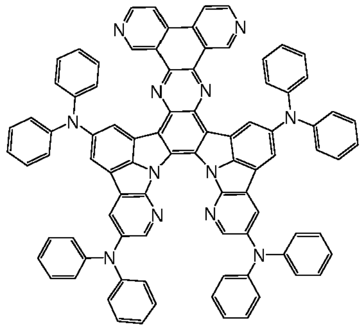

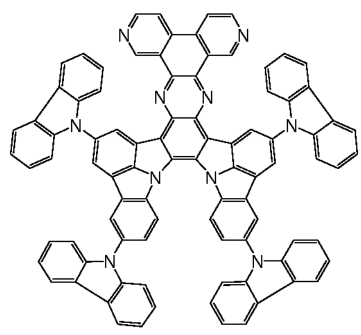

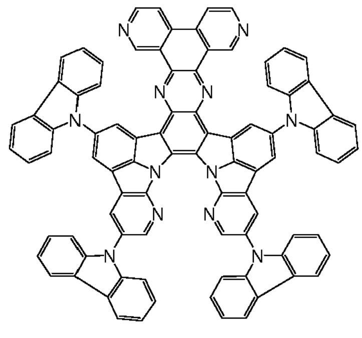

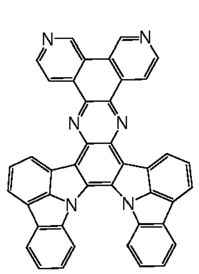

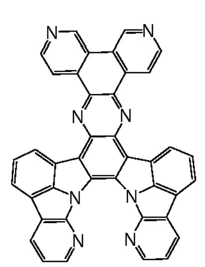

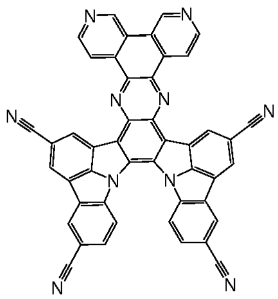

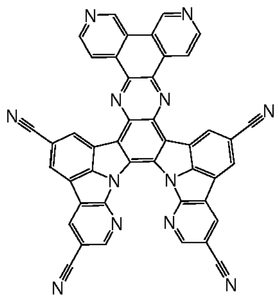

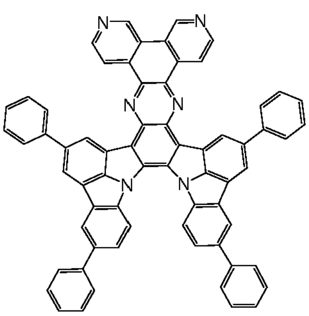

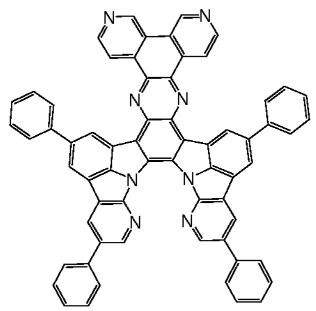

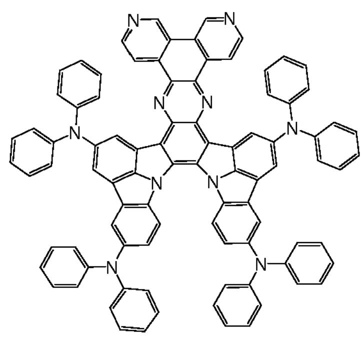

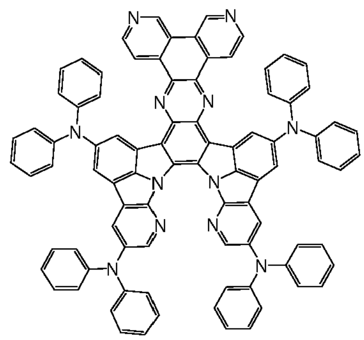

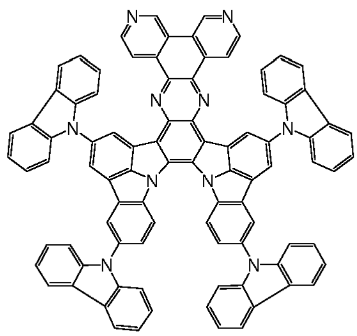

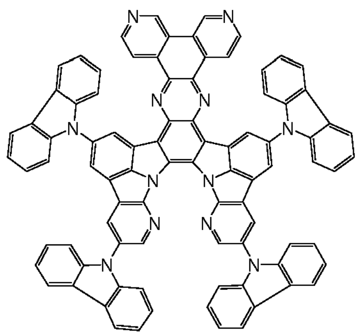

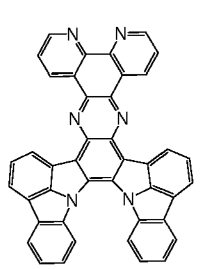

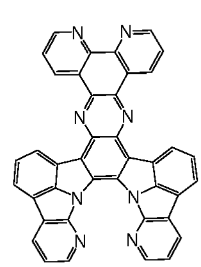

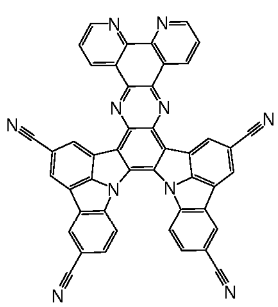

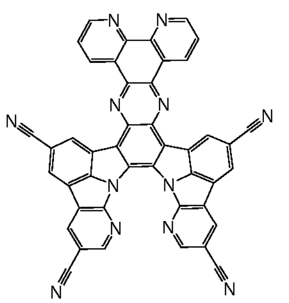

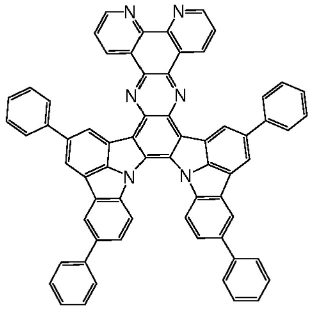

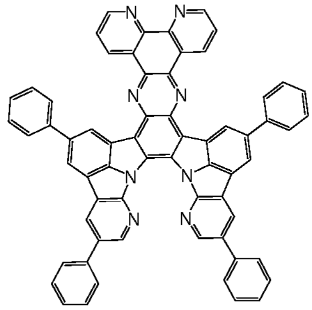

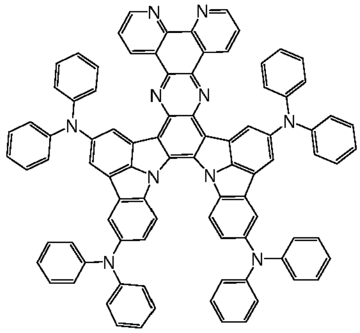

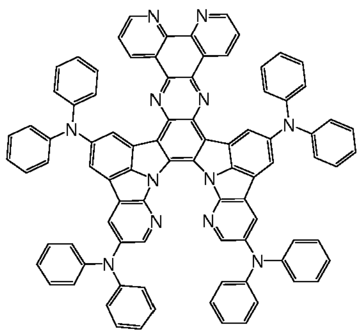

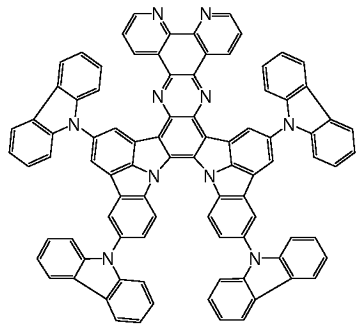

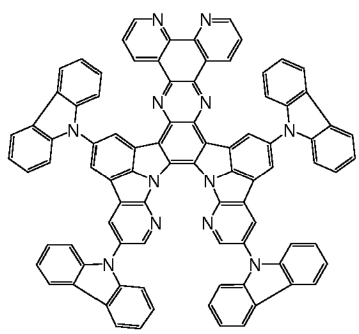

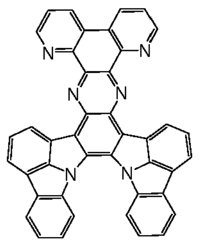

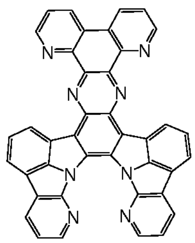

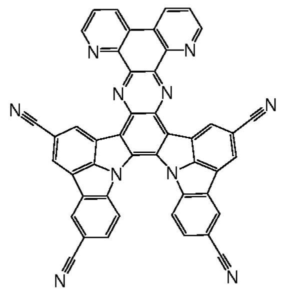

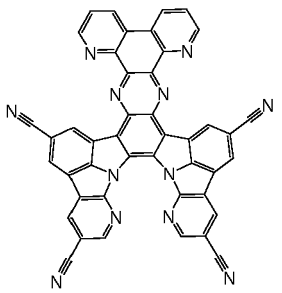

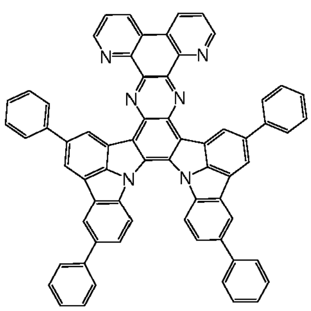

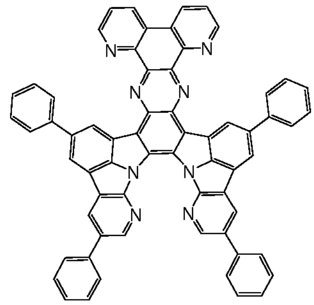

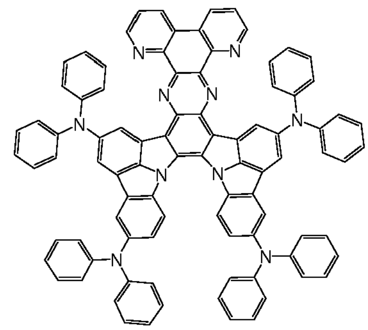

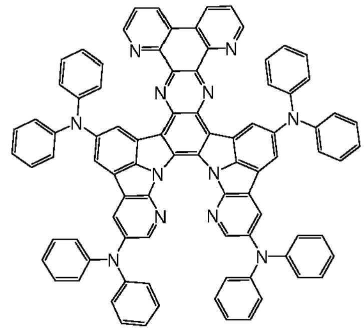

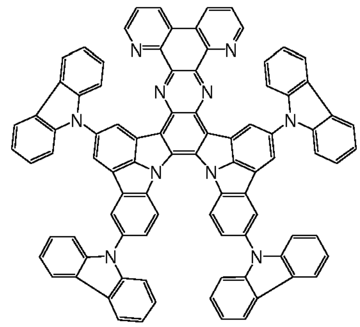

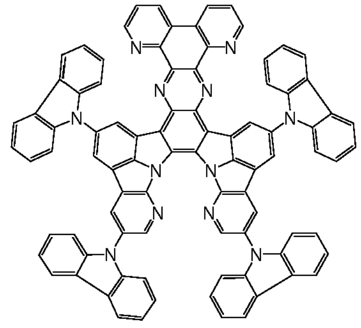

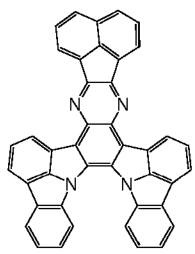

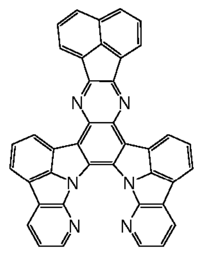

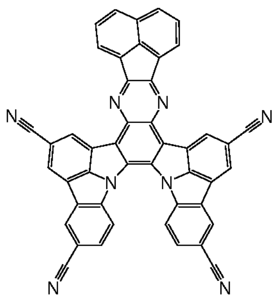

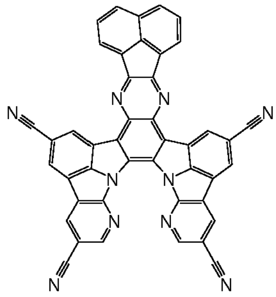

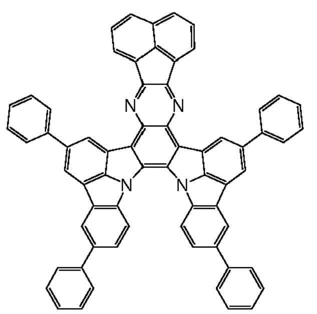

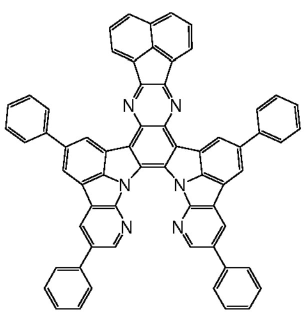

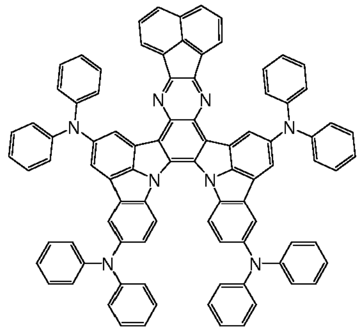

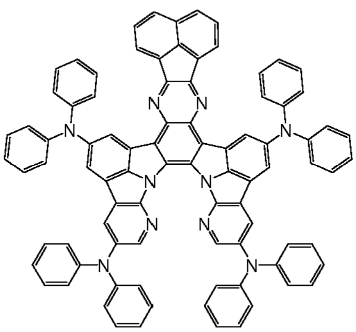

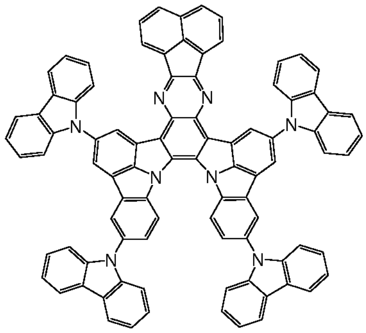

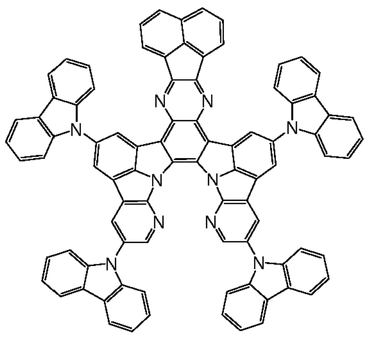

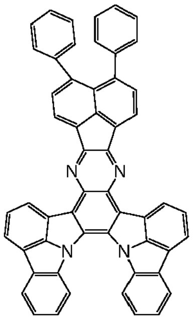
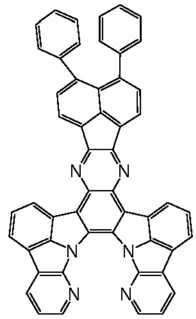
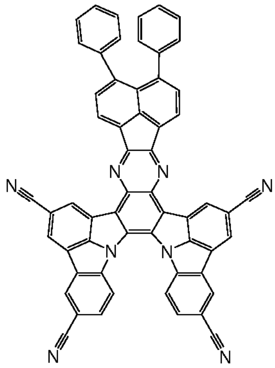
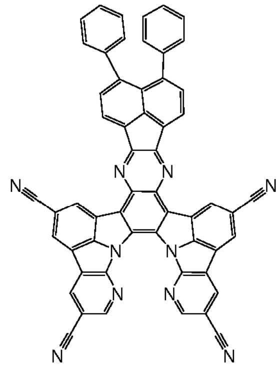

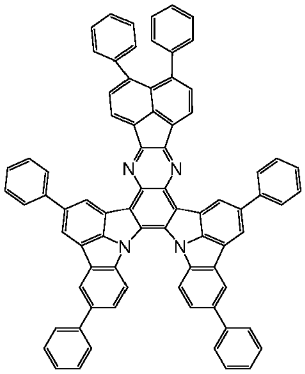
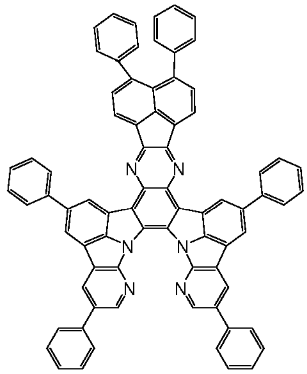
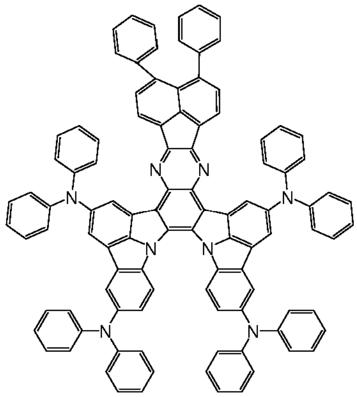

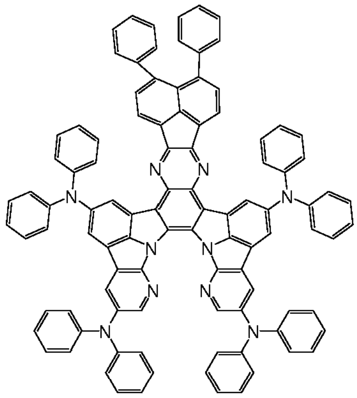
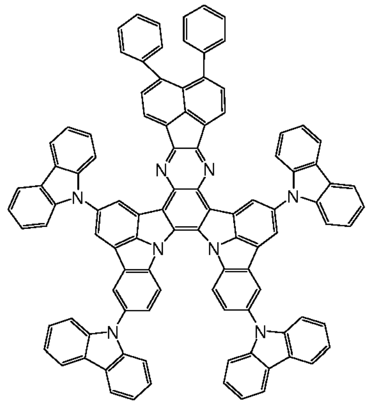
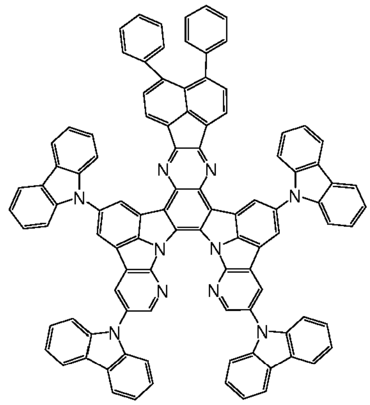

## Claims

1. An organic molecule of a structure of formula Ia or Ib:

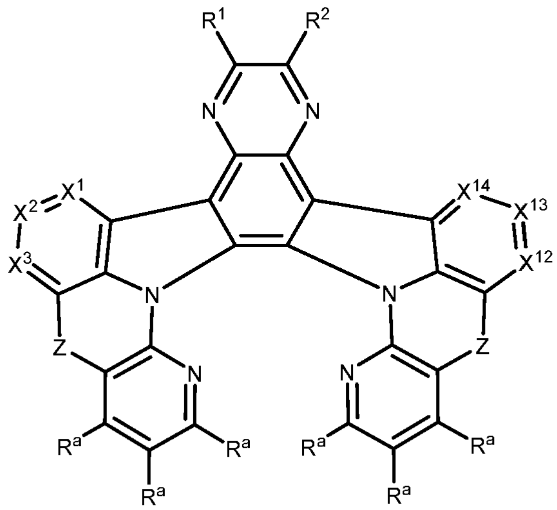

,

Formula Ia

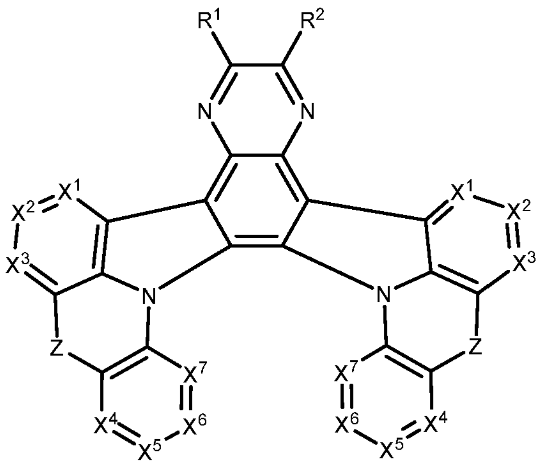

,

Formula Ib

wherein

$X^1$, $X^2$, $X^3$, $X^4$, $X^5$, $X^6$, $X^7$, $X^8$, $X^9$, $X^{10}$, $X^{11}$, $X^{12}$, $X^{13}$, and $X^{14}$ is $CR^a$;
Z is a direct bond;
$R^1$ and $R^2$ is at each occurrence independently from another selected from the group consisting of hydrogen, deuterium, $N(R^5)_2$, $OR^5$, $Si(R^5)_3$, $B(OR^5)_2$, $OSO_2R^5$, $CF_3$, CN, F, Br, I, $C_1$-$C_{40}$-alkyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-alkoxy,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-thioalkoxy,

which is optionally substituted with one or more substituents $R^5$ and

wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkenyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkynyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_6$-$C_{60}$-aryl,

which is optionally substituted with one or more substituents $R^5$; and $C_3$-$C_{57}$-heteroaryl,
which is optionally substituted with one or more substituents $R^5$; and

a mono- or polycyclic, aliphatic, aromatic and/or benzo-fused ring system formed by ring-closure with one or more other substituent selected from the group consisting of $R^1$, $R^2$ and $R^5$;
$R^a$ is at each occurrence independently from another selected from the group consisting of hydrogen,
deuterium,
$N(R^5)_2$,
$OR^5$,
$Si(R^5)_3$,
$B(OR^5)_2$,
$OSO_2R^5$,
$CF_3$,
CN,
F,
Br,
I,
$C_1$-$C_{40}$-alkyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-alkoxy,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-thioalkoxy,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkenyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkynyl,

which is optionally substituted with one or more substituents $R^5$ and wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_6$-$C_{60}$-aryl,

which is optionally substituted with one or more substituents $R^5$; and $C_3$-$C_{57}$-heteroaryl, which is optionally substituted with one or more substituents $R^5$;

$R^5$ is at each occurrence independently from another selected from the group consisting of hydrogen, deuterium, $N(R^6)_2$, $OR^6$, $Si(R^6)_3$, $B(OR^6)_2$, $OSO_2R^6$, $CF_3$, CN, F, Br, I,
$C_1$-$C_{40}$-alkyl,

which is optionally substituted with one or more substituents $R^6$ and wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_1$-$C_{40}$-alkoxy,

which is optionally substituted with one or more substituents $R^6$ and wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_1$-$C_{40}$-thioalkoxy,

which is optionally substituted with one or more substituents $R^6$ and wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_2$-$C_{40}$-alkenyl,

which is optionally substituted with one or more substituents $R^6$ and wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_2$-$C_{40}$-alkynyl,

which is optionally substituted with one or more substituents $R^6$ and wherein one or more non-adjacent $CH_2$-groups are optionally substituted by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_6$-$C_{60}$-aryl,

which is optionally substituted with one or more substituents $R^6$; and $C_3$-$C_{57}$-heteroaryl, which is optionally substituted with one or more substituents $R^6$;

$R^6$ is at each occurrence independently from another selected from the group consisting of hydrogen, deuterium, OPh, $CF_3$, CN, F,
$C_1$-$C_5$-alkyl,
wherein one or more hydrogen atoms are optionally, independently from each other substituted by deuterium, CN, $CF_3$, or F;
$C_1$-$C_5$-alkoxy,
wherein one or more hydrogen atoms are optionally, independently from each other substituted by deuterium, CN, $CF_3$, or F;
$C_1$-$C_5$-thioalkoxy,
wherein one or more hydrogen atoms are optionally, independently from each other substituted by deuterium,

CN, $CF_3$, or F;
$C_2$-$C_5$-alkenyl,
wherein one or more hydrogen atoms are optionally, independently from each other substituted by deuterium, CN, $CF_3$, or F;
$C_2$-$C_5$-alkynyl,
wherein one or more hydrogen atoms are optionally, independently from each other substituted by deuterium, CN, $CF_3$, or F;
$C_6$-$C_{18}$-aryl,
which is optionally substituted with one or more $C_1$-$C_5$-alkyl substituents;
$C_3$-$C_{17}$-heteroaryl,
which is optionally substituted with one or more $C_1$-$C_5$-alkyl substituents;
N($C_6$-$C_{18}$-aryl)$_2$;
N($C_3$-$C_{17}$-heteroaryl)$_2$,
and N($C_3$-$C_{17}$-heteroaryl)($C_6$-$C_{18}$-aryl);
wherein any of the substituents $R^1$, $R^2$, $R^a$, or $R^5$ independently from each other optionally form a mono- or polycyclic, aliphatic, aromatic and/or benzo-fused ring system with one or more other substituent $R^1$, $R^2$, $R^a$, or $R^5$.

**2.** Organic molecule according to claim 1, wherein $R^1$ and $R^2$ is independently selected from the group consisting of

phenyl, which is optionally substituted with one or more substituents $R^5$; and
pyridine, which is optionally substituted with one or more substituents $R^5$.

**3.** Organic molecule according to claim 1 or 2, wherein the substituents $R^2$ and $R^1$ are the same.

**4.** Organic molecule according to one or more of claims 1 to 3, wherein the organic molecule is a structure selected from the group consisting of formula IIIa, formula IIIb, formula IIIc, formula IIId, formula IIIe, formula IIIf, formula IIIg, formula IIIh, and formula IIIi:

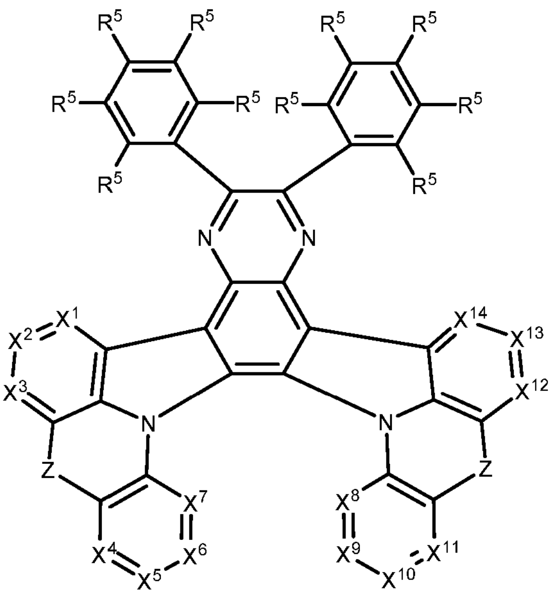

Formula IIIa

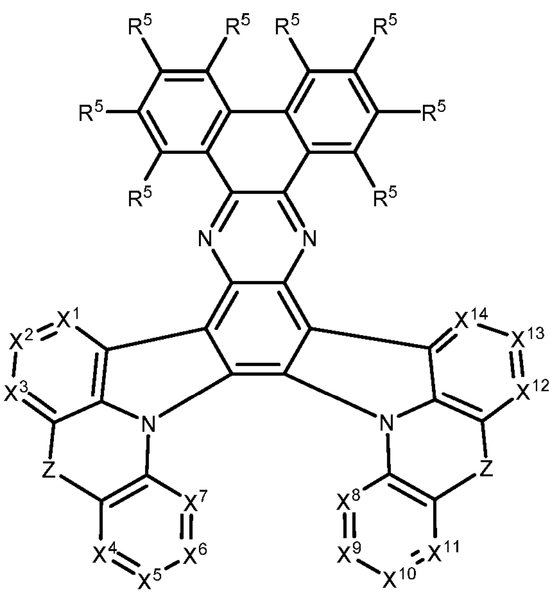

Formula IIIb

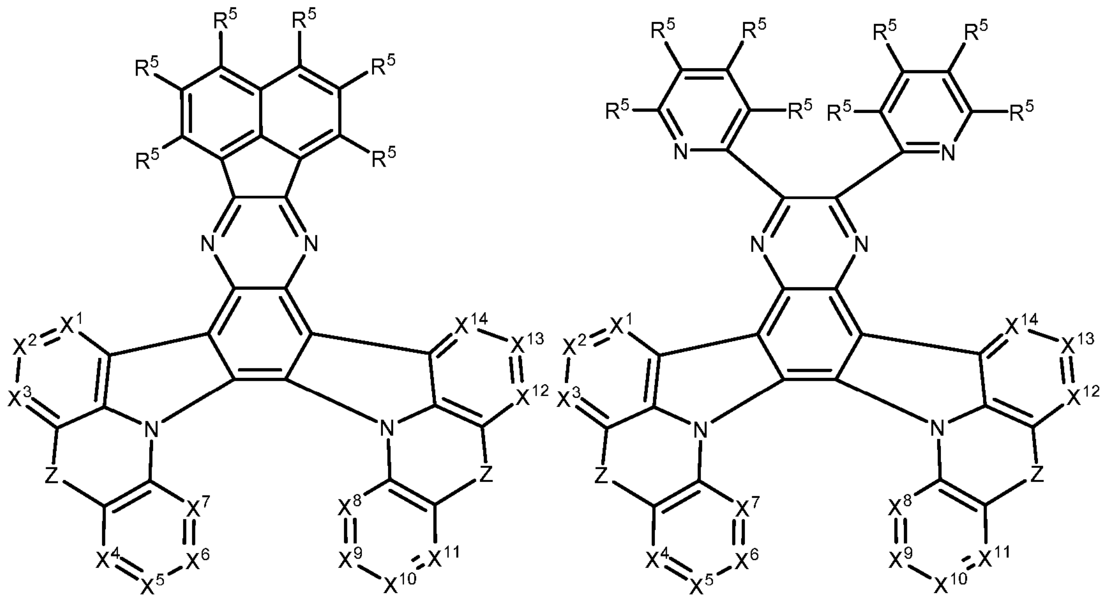

Formula IIIc Formula IIId

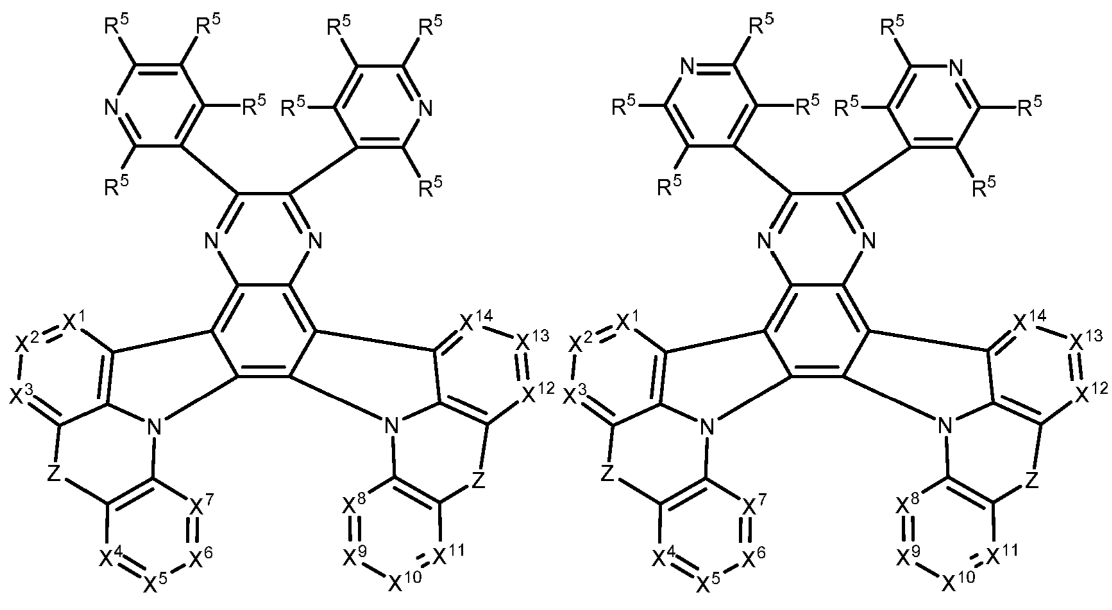

Formula IIIe Formula IIIf

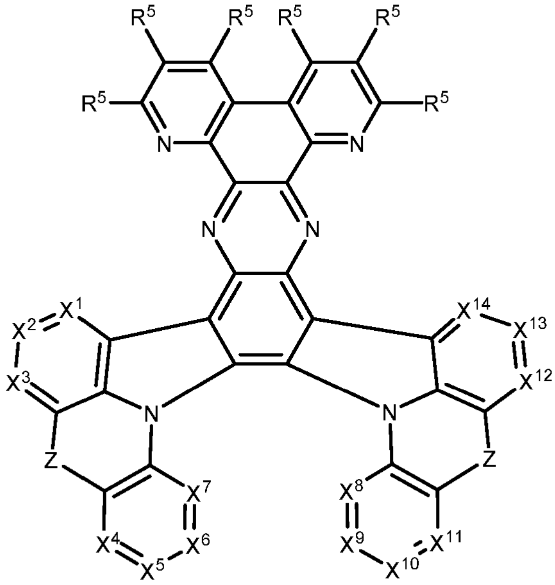

Formula IIIg

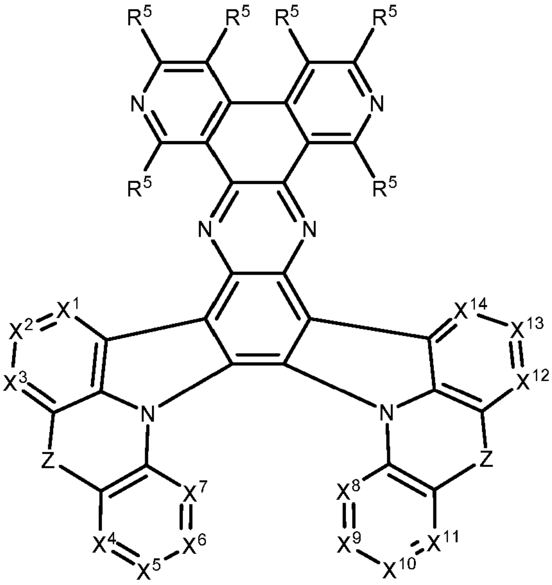

Formula IIIh

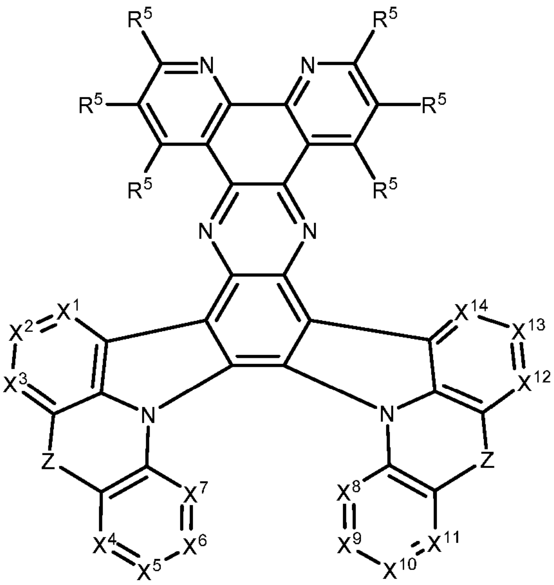

Formula IIIi

**5.** Organic molecule according to any of claims 1 to 3, wherein $R^a$ is at each occurrence independently from another selected from the group consisting of:

hydrogen,
Me,
$^{i}Pr$,
$^{t}Bu$,
CN,
$CF_3$,
Ph, which is optionally substituted with one or more substituents independently from each other selected from the group consisting of Me, $^{i}Pr$, $^{t}Bu$, CN, $CF_3$, and Ph,
pyridinyl, which is optionally substituted with one or more substituents independently from each other selected from the group consisting of Me, $^{i}Pr$, $^{t}Bu$, CN, $CF_3$, and Ph,
pyrimidinyl, which is optionally substituted with one or more substituents independently from each other selected from the group consisting of Me, $^{i}Pr$, $^{t}Bu$, CN, $CF_3$, and Ph,

carbazolyl, which is optionally substituted with one or more substituents independently from each other selected from the group consisting of Me, $^{i}$Pr, $^{t}$Bu, CN, $CF_3$, and Ph,
triazinyl, which is optionally substituted with one or more substituents independently from each other selected from the group consisting of Me, $^{i}$Pr, $^{t}$Bu, CN, $CF_3$, and Ph,
and $N(Ph)_2$.

**6.** Organic molecule according to any of claims 1 to 5 wherein $R^5$ is at each occurrence independently from another selected from the group consisting of:

hydrogen,
Me,
$^{i}$Pr,
$^{t}$Bu,
CN,
$CF_3$,
Ph, which is optionally substituted with one or more substituents independently from each other selected from the group consisting of Me, $^{i}$Pr, $^{t}$Bu, CN, $CF_3$, and Ph;
pyridinyl, which is optionally substituted with one or more substituents independently from each other selected from the group consisting of Me, $^{i}$Pr, $^{t}$Bu, CN, $CF_3$, and Ph;
pyrimidinyl, which is optionally substituted with one or more substituents independently from each other selected from the group consisting of Me, $^{i}$Pr, $^{t}$Bu, CN, $CF_3$, and Ph;
carbazolyl, which is optionally substituted with one or more substituents independently from each other selected from the group consisting of Me, $^{i}$Pr, $^{t}$Bu, CN, $CF_3$, and Ph;
triazinyl, which is optionally substituted with one or more substituents independently from each other selected from the group consisting of Me, $^{i}$Pr, $^{t}$Bu, CN, $CF_3$, and Ph;
and $N(Ph)_2$.

**7.** Use of an organic molecule according to any of claims 1 to 6 as a luminescent emitter in an optoelectronic device.

**8.** Use according to claim 7, wherein the optoelectronic device is selected from the group consisting of:

• organic light-emitting diodes (OLEDs),
• light-emitting electrochemical cells,
• OLED-sensors,
• organic diodes,
• organic solar cells,
• organic transistors,
• organic field-effect transistors,
• organic lasers, and
• down-conversion elements.

**9.** Composition, comprising:

(a) an organic molecule according to any of claims 1 to 6, in particular in the form of an emitter,
(b) a triplet-triplet annihilation (TTA) host material, which differs from the organic molecule,
(c) optionally, a TADF material, and
(d) optionally, a dye and/or a solvent.

**10.** Optoelectronic device, comprising an organic molecule according to any of claims 1 to 6 or a composition according to claim 11, in particular in the form of a device selected from the group consisting of organic light-emitting diode (OLED), light-emitting electrochemical cell, OLED-sensor, organic diode, organic solar cell, organic transistor, organic field-effect transistor, organic laser, and down-conversion element.

**11.** Optoelectronic device according to claim 10, comprising:

- a substrate,
- an anode, and
- a cathode, wherein the anode or the cathode is disposed on the substrate, and

- a light-emitting layer, which is arranged between the anode and the cathode and which comprises the organic molecule or the composition.

**12.** Method for producing an optoelectronic device, wherein an organic molecule according to any one of claims 1 to 6 or a composition according to claim 9 is used.

**13.** Method according to claim 12, comprising the processing of the organic molecule by a vacuum evaporation method or from a solution.

## Patentansprüche

**1.** Organisches Molekül mit einer Struktur der Formel Ia oder Ib:

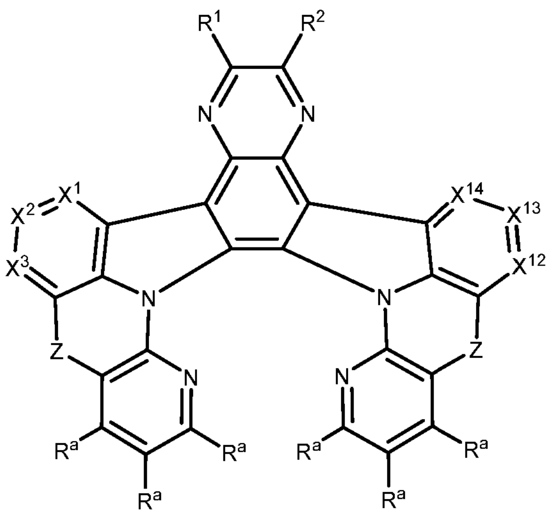

Formel Ia

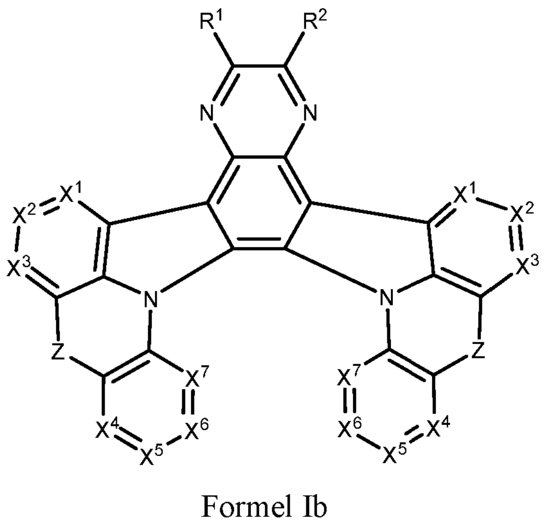

Formel Ib

wobei

$X^1$, $X^2$, $X^3$, $X^4$, $X^5$, $X^6$, $X^7$, $X^8$, $X^9$, $X^{10}$, $X^{11}$, $X^{12}$, $X^{13}$ und $X^{14}$ $CR^a$ sind;
Z eine direkte Bindung ist;
$R^1$ und $R^2$ bei jedem Auftreten unabhängig voneinander aus der Gruppe bestehend aus Wasserstoff, Deuterium, $N(R^5)_2$, $OR^5$, $Si(R^5)_3$, $B(OR^5)_2$, $OSO_2R^5$, $CF_3$, CN, F, Br, I, C1-C40-Alkyl ausgewählt sind,

welche optional mit einem oder mehreren Substituenten $R^5$ substituiert sind, und
wobei eine oder mehrere nicht benachbarte $CH_2$-Gruppen optional durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ substituiert sind;
$C_1$-$C_{40}$-Alkoxy,
das optional mit einem oder mehreren Substituenten $R^5$ substituiert ist, und
wobei eine oder mehrere nicht benachbarte $CH_2$-Gruppen optional durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ substituiert sind;
$C_1$-$C_{40}$-Thioalkoxy,
das optional mit einem oder mehreren Substituenten $R^5$ substituiert ist, und
wobei eine oder mehrere nicht benachbarte $CH_2$-Gruppen optional durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ substituiert sind;
$C_2$-$C_{40}$-Alkenyl,
das optional mit einem oder mehreren Substituenten $R^5$ substituiert ist, und
wobei eine oder mehrere nicht benachbarte CH2-Gruppen optional durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ substituiert sind;
$C_2$-$C_{40}$-Alkinyl,
das optional mit einem oder mehreren Substituenten $R^5$ substituiert ist, und
wobei eine oder mehrere nicht benachbarte $CH_2$-Gruppen optional durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ substituiert sind;
$C_6$-$C_{60}$-Aryl,
das optional mit einem oder mehreren Substituenten $R^5$ substituiert ist; und
$C_3$-$C_{57}$-Heteroaryl,
das optional mit einem oder mehreren Substituenten $R^5$ substituiert ist; und
ein mono- oder polyzyklisches, aliphatisches, aromatisches und/oder benzo-kondensiertes Ringsystem, das durch Ringschluss mit einem oder mehreren anderen Substituenten gebildet wird, ausgewählt aus der Gruppe bestehend aus $R^1$, $R^2$ und $R^5$;
$R^a$ bei jedem Auftreten unabhängig voneinander ausgewählt ist aus der Gruppe bestehend aus Wasserstoff, Deuterium,
$N(R^5)_2$,
$OR^5$,
$Si(R^5)_3$,
$B(OR^5)_2$,
$OSO_2R^5$,
$CF_3$,
CN,
F,
Br,
I,
$C_1$-$C_{40}$-Alkyl,
das optional mit einem oder mehreren Substituenten $R^5$ substituiert ist, und
wobei eine oder mehrere nicht benachbarte $CH_2$-Gruppen optional durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ substituiert sind;
$C_1$-$C_{40}$-Alkoxy,
das optional mit einem oder mehreren Substituenten $R^5$ substituiert ist, und
wobei eine oder mehrere nicht benachbarte $CH_2$-Gruppen optional durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ substituiert sind;
$C_1$-$C_{40}$-Thioalkoxy,
das optional mit einem oder mehreren Substituenten $R^5$ substituiert ist, und
wobei eine oder mehrere nicht benachbarte $CH_2$-Gruppen optional durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ substituiert sind;
$C_2$-$C_{40}$-Alkenyl,
das optional mit einem oder mehreren Substituenten $R^5$ substituiert ist, und
wobei eine oder mehrere nicht benachbarte $CH_2$-Gruppen optional durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ substituiert sind;
$C_2$-$C_{40}$-Alkinyl,
das optional mit einem oder mehreren Substituenten $R^5$ substituiert ist, und
wobei eine oder mehrere nicht benachbarte $CH_2$-Gruppen optional durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ substituiert sind;

$C_6$-$C_{60}$-Aryl,
das optional mit einem oder mehreren Substituenten $R^5$ substituiert ist, und
$C_3$-$C_{57}$-Heteroaryl,
das optional mit einem oder mehreren Substituenten $R^5$ substituiert ist;
$R^5$ bei jedem Auftreten unabhängig voneinander aus der Gruppe bestehend aus Wasserstoff, Deuterium, $N(R^6)_2$, $OR^6$, $Si(R^6)_3$, $B(OR^6)_2$, $OSO_2R^6$, $CF_3$, CN, F, Br, I ausgewählt ist,
$C_1$-$C_{40}$-Alkyl,
das optional mit einem oder mehreren Substituenten $R^6$ substituiert ist, und
wobei eine oder mehrere nicht benachbarte $CH_2$-Gruppen optional durch $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ substituiert sind;
$C_1$-$C_{40}$-Alkoxy,
das optional mit einem oder mehreren Substituenten $R^6$ substituiert ist, und
wobei eine oder mehrere nicht benachbarte $CH_2$-Gruppen optional durch $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ substituiert sind;
$C_1$-$C_{40}$-Thioalkoxy,
das optional mit einem oder mehreren Substituenten $R^6$ substituiert ist, und
wobei eine oder mehrere nicht benachbarte $CH_2$-Gruppen optional durch $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ substituiert sind;
$C_2$-$C_{40}$-Alkenyl,
das optional mit einem oder mehreren Substituenten $R^6$ substituiert ist, und
wobei eine oder mehrere nicht benachbarte $CH_2$-Gruppen optional durch $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ substituiert sind;
$C_2$-$C_{40}$-Alkinyl,
das optional mit einem oder mehreren Substituenten $R^6$ substituiert ist, und
wobei eine oder mehrere nicht benachbarte $CH_2$-Gruppen optional durch $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ substituiert sind;
$C_6$-$C_{60}$-Aryl,
das optional mit einem oder mehreren Substituenten $R^6$ substituiert ist, und
$C_3$-$C_{57}$-Heteroaryl,
das optional mit einem oder mehreren Substituenten $R^6$ substituiert ist;
$R^6$ bei jedem Auftreten unabhängig voneinander aus der Gruppe bestehend aus Wasserstoff, Deuterium, OPh, CF3, CN, F ausgewählt ist,
$C_1$-$C_5$-Alkyl,
wobei ein oder mehrere Wasserstoffatome optional unabhängig voneinander durch Deuterium, CN, $CF_3$ oder F substituiert sind;
$C_1$-$C_5$-Alkoxy,
wobei ein oder mehrere Wasserstoffatome optional unabhängig voneinander durch Deuterium, CN, $CF_3$ oder F substituiert sind;
$C_1$-$C_5$-Thioalkoxy,
wobei ein oder mehrere Wasserstoffatome optional unabhängig voneinander durch Deuterium, CN, $CF_3$ oder F substituiert sind;
$C_2$-$C_5$-Alkenyl,
wobei ein oder mehrere Wasserstoffatome optional unabhängig voneinander durch Deuterium, CN, $CF_3$ oder F substituiert sind;
$C_2$-$C_5$-Alkinyl,
wobei ein oder mehrere Wasserstoffatome optional unabhängig voneinander durch Deuterium, CN, $CF_3$ oder F substituiert sind;
$C_6$-$C_{18}$-Aryl,
das optional mit einem oder mehreren $C_1$-$C_5$-Alkylsubstituenten substituiert ist;
$C_3$-$C_{17}$-Heteroaryl,
das optional mit einem oder mehreren $C_1$-$C_5$-Alkylsubstituenten substituiert ist; $N(C_6$-$C_{18}$-aryl$)_2$;
$N(C_3$-$C_{17}$-Heteroaryl$)_2$,
und $N(C_3$-$C_{17}$-Heteroaryl$)(C_6$-$C_{18}$-Aryl$)$;
wobei jeder der Substituenten $R^1$, $R^2$, $R^a$ oder $R^5$ unabhängig voneinander optional ein mono- oder polycyclisches, aliphatisches, aromatisches und/oder benzo-kondensiertes Ringsystem mit einem oder mehreren weiteren Substituenten $R^1$, $R^2$, $R^a$ oder $R^5$ bildet.

2. Organisches Molekül nach Anspruch 1, wobei $R^1$ und $R^2$ unabhängig voneinander ausgewählt sind aus der Gruppe

bestehend aus

Phenyl, das optional mit einem oder mehreren Substituenten $R^5$ substituiert ist; und
Pyridin, das optional mit einem oder mehreren Substituenten $R^5$ substituiert ist.

**3.** Organisches Molekül nach Anspruch 1 oder 2, wobei die Substituenten $R^2$ und $R^1$ gleich sind.

**4.** Organisches Molekül nach einem oder mehreren der Ansprüche 1 bis 3, wobei das organische Molekül eine Struktur ist, die aus der Gruppe bestehend aus Formel IIIa, Formel IIIb, Formel IIIc, Formel IIId, Formel IIIe, Formel IIIf, Formel IIIg, Formel IIIh und Formel IIIi ausgewählt ist:

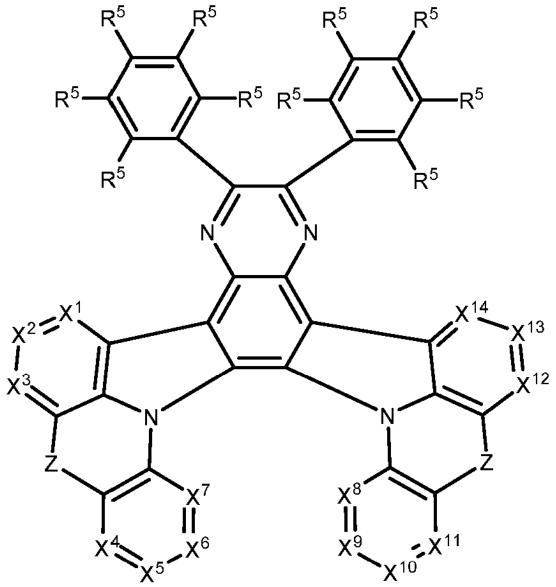

Formel IIIa

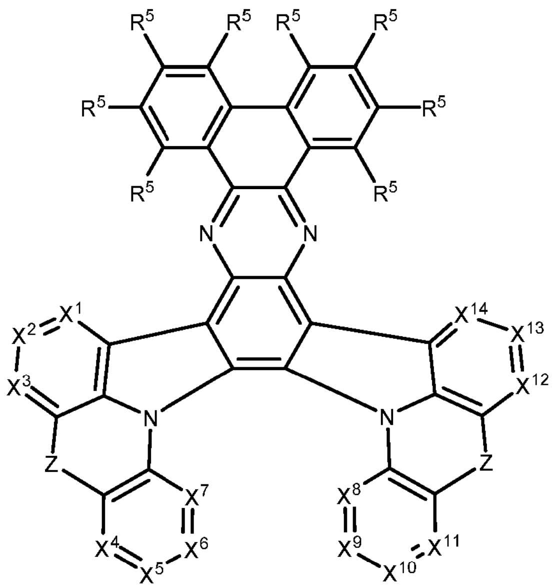

Formel IIIb

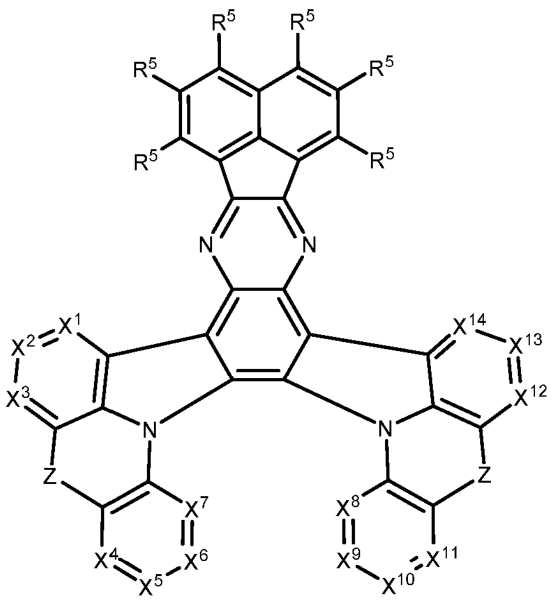

Formel IIIc

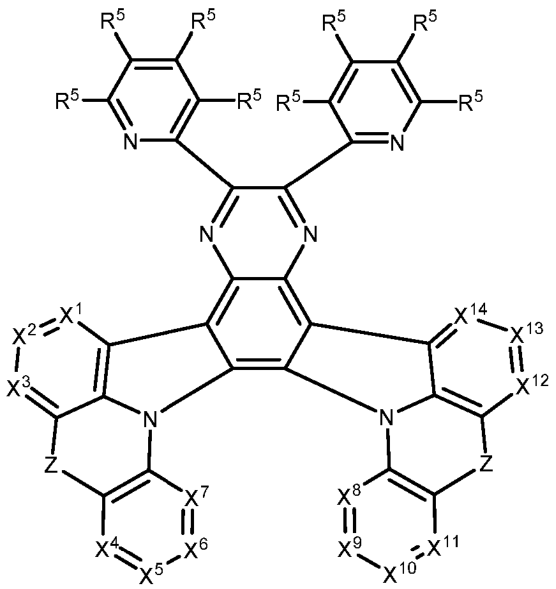

Formel IIId

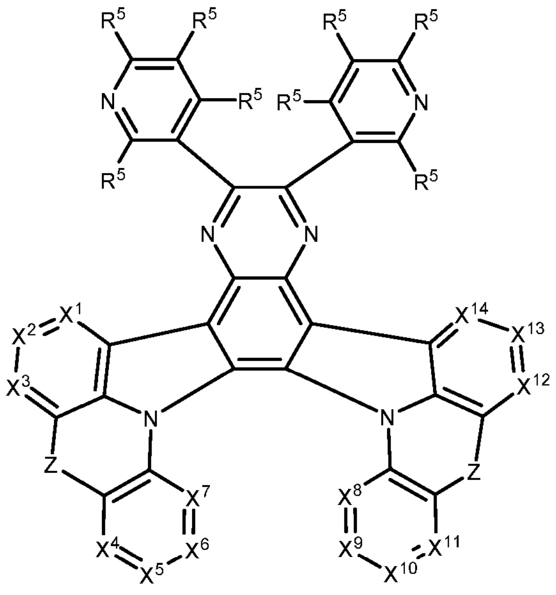

Formel IIIe

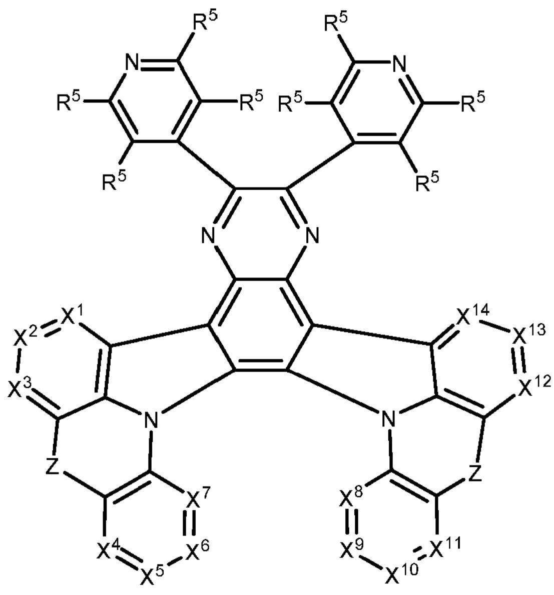

Formel IIIf

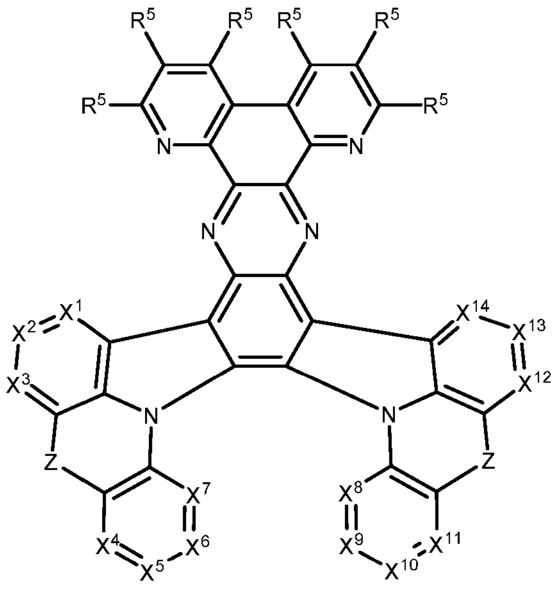

Formel IIIg

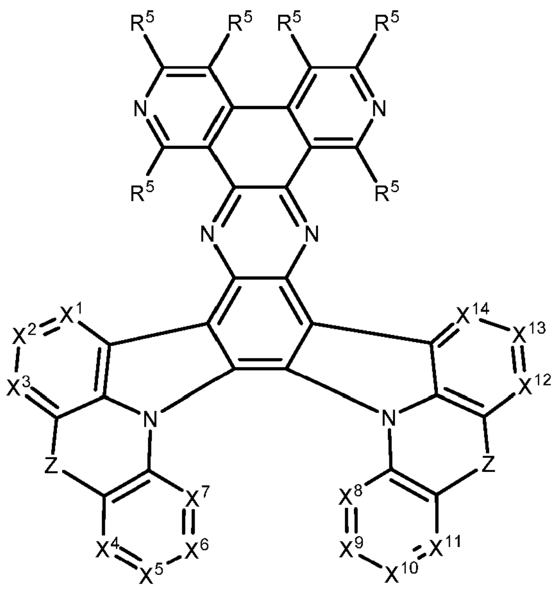

Formel IIIh

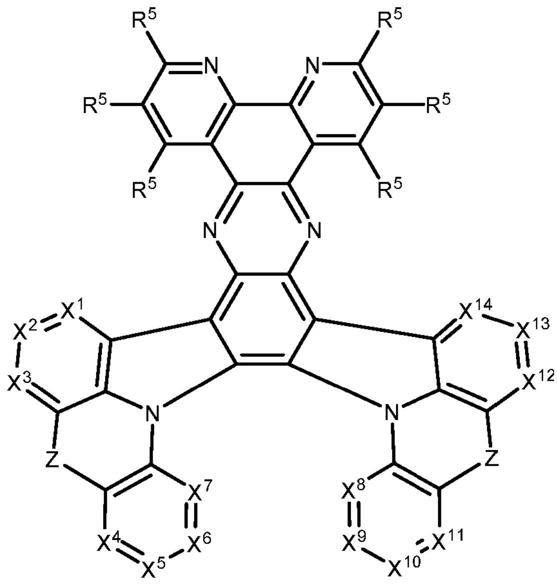

Formel IIIi

**5.** Organisches Molekül nach einem der Ansprüche 1 bis 3, wobei $R^a$ bei jedem Auftreten unabhängig voneinander ausgewählt ist aus der Gruppe bestehend aus:

Wasserstoff,
Me,
$^iPr$,
$^tBu$,
ON,
$CF_3$,
Ph, das optional mit einem oder mehreren Substituenten substituiert ist, die unabhängig voneinander aus der Gruppe bestehend aus Me, $^iPr$, $^tBu$, ON, $CF_3$ und Ph ausgewählt sind,
Pyridinyl, das optional mit einem oder mehreren Substituenten substituiert ist, die unabhängig voneinander aus der Gruppe bestehend aus Me, $^iPr$, $^tBu$, CN, $CF_3$ und Ph ausgewählt sind,
Pyrimidinyl, das optional mit einem oder mehreren Substituenten substituiert ist, die unabhängig voneinander aus der Gruppe bestehend aus Me, $^iPr$, $^tBu$, CN, $CF_3$ und Ph ausgewählt sind,
Carbazolyl, das optional mit einem oder mehreren Substituenten substituiert ist, die unabhängig voneinander aus der Gruppe bestehend aus Me, $^iPr$, $^tBu$, CN, $CF_3$ und Ph ausgewählt sind,
Triazinyl, das optional mit einem oder mehreren Substituenten substituiert ist, die unabhängig voneinander aus der Gruppe bestehend aus Me, $^iPr$, $^tBu$, CN, $CF_3$ und Ph ausgewählt sind,
und $N(Ph)_2$.

**6.** Organisches Molekül nach einem der Ansprüche 1 bis 5, wobei $R^5$ bei jedem Auftreten unabhängig voneinander ausgewählt ist aus der Gruppe bestehend aus:

Wasserstoff,
Me,
$^iPr$,
$^tBu$,
CN,
$CF_3$,
Ph, das optional mit einem oder mehreren Substituenten substituiert ist, die unabhängig voneinander aus der Gruppe bestehend aus Me, $^iPr$, $^tBu$, CN, $CF_3$ und Ph ausgewählt sind;
Pyridinyl, das optional mit einem oder mehreren Substituenten substituiert ist, die unabhängig voneinander aus der Gruppe bestehend aus Me, $^iPr$, $^tBu$, CN, $CF_3$ und Ph ausgewählt sind;
Pyrimidinyl, das optional mit einem oder mehreren Substituenten substituiert ist, die unabhängig voneinander aus der Gruppe bestehend aus Me, $^iPr$, $^tBu$, CN, $CF_3$ und Ph ausgewählt sind;

Carbazolyl, das optional mit einem oder mehreren Substituenten substituiert ist, die unabhängig voneinander aus der Gruppe bestehend aus Me, $^{i}Pr$, $^{t}Bu$, CN, $CF_3$ und Ph ausgewählt sind;
Triazinyl, das optional mit einem oder mehreren Substituenten substituiert ist, die unabhängig voneinander aus der Gruppe bestehend aus Me, $^{i}Pr$, $^{t}Bu$, CN, $CF_3$ und Ph ausgewählt sind;
und $N(Ph)_2$.

**7.** Verwendung eines organischen Moleküls nach einem der Ansprüche 1 bis 6 als Lumineszenzemitter in einem optoelektronischen Bauelement.

**8.** Verwendung nach Anspruch 7, wobei das optoelektronische Bauelement ausgewählt ist aus der Gruppe bestehend aus:

• organischen Leuchtdioden (OLEDs),
• lichtemittierenden elektrochemischen Zellen,
• OLED-Sensoren,
• organischen Dioden,
• organischen Solarzellen,
• organischen Transistoren,
• organischen Feldeffekttransistoren,
• organischen Lasern und
• Down-Conversion-Elementen.

**9.** Zusammensetzung, umfassend:

(a) ein organisches Molekül nach einem der Ansprüche 1 bis 6, insbesondere in Form eines Emitters,
(b) ein Triplet-Triplet-Annihilation (TTA)-Wirtsmaterial, das sich von dem organischen Molekül unterscheidet,
(c) optional ein TADF-Material und
(d) optional einen Farbstoff und/oder ein Lösungsmittel.

**10.** Optoelektronisches Bauelement, umfassend ein organisches Molekül nach einem der Ansprüche 1 bis 6 oder eine Zusammensetzung nach Anspruch 11, insbesondere in Form eines Bauelements, ausgewählt aus der Gruppe bestehend aus einer organischen Leuchtdiode (OLED), einer lichtemittierenden elektrochemischen Zelle, einem OLED-Sensor, einer organischen Diode, einer organischen Solarzelle, einem organischen Transistor, einem organischen Feldeffekttransistor, einem organischen Laser und einem Down-Conversion-Element.

**11.** Optoelektronisches Bauelement nach Anspruch 10, umfassend:

- ein Substrat,
- eine Anode und
- eine Kathode, wobei die Anode oder die Kathode auf dem Substrat angeordnet ist, und
- eine lichtemittierende Schicht, die zwischen der Anode und der Kathode angeordnet ist und das organische Molekül oder die Zusammensetzung umfasst.

**12.** Verfahren zur Herstellung eines optoelektronischen Bauelements, wobei ein organisches Molekül nach einem der Ansprüche 1 bis 6 oder eine Zusammensetzung nach Anspruch 9 verwendet wird.

**13.** Verfahren nach Anspruch 12, umfassend das Verarbeiten des organischen Moleküls durch ein Vakuumverdampfungsverfahren oder aus einer Lösung.

## Revendications

**1.** Molécule organique d’une structure de formule Ia ou Ib :

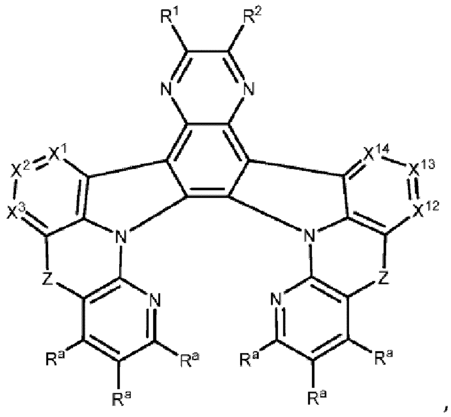

Formule Ia

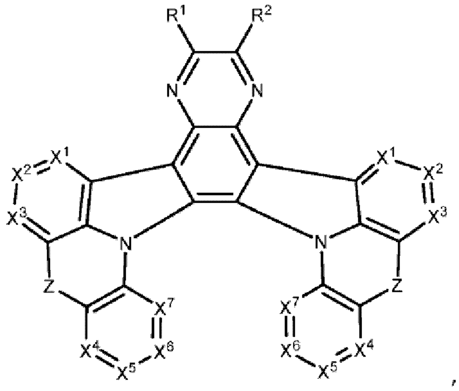

Formule Ib

dans laquelle

$X^1$, $X^2$, $X^3$, $X^4$, $X^5$, $X^6$, $X^7$, $X^8$, $X^9$, $X^{10}$, $X^{11}$, $X^{12}$, $X^{13}$, et $X^{14}$ est $CR^a$ ;
Z est une liaison directe ;
$R^1$ et $R^2$ est, à chaque occurrence indépendamment d'une autre, sélectionné parmi le groupe consistant en hydrogène, deutérium, $N(R^5)_2$, $OR^5$, $Si(R^5)_3$, $B(OR^5)_2$, $OSO_2R^5$, $CF_3$, CN, F, Br, I, alkyle en $C_1$ à $C_{40}$,
qui est facultativement substitué par un ou plusieurs substituants $R^5$ et
dans laquelle un ou plusieurs groupes $CH_2$ non adjacents sont facultativement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
alcoxy en $C_1$ à $C_{40}$,
qui est facultativement substitué par un ou plusieurs substituants $R^5$ et
dans laquelle un ou plusieurs groupes $CH_2$ non adjacents sont facultativement substitués par $R^5C=CR^5$, C≡C, $Si(R^5)_2$, $Ge(R^5)_2$, $Sn(R^5)_2$, C≡O, C=S, C=Se, $C=NR^5$, $P(=O)(R^5)$, SO, $SO_2$, $NR^5$, O, S ou $CONR^5$ ;
thioalcoxy en $C_1$ à $C_{40}$,
qui est facultativement substitué par un ou plusieurs substituants $R^5$ et
dans laquelle un ou plusieurs groupes $CH_2$ non adjacents sont facultativement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
alcényle en $C_2$ à $C_{40}$,
qui est facultativement substitué par un ou plusieurs substituants $R^5$ et
dans laquelle un ou plusieurs groupes $CH_2$ non adjacents sont facultativement substitués par $R^5C=CR^5$, C≡C, $Si(R^5)_2$, Ge $(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
alcynyle en $C_2$ à $C_{40}$,
qui est facultativement substitué par un ou plusieurs substituants $R^5$ et
dans laquelle un ou plusieurs groupes $CH_2$ non adjacents sont facultativement substitués par $R^5C=CR^5$, C≡C, $Si(R^5)_2$, Ge $(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
aryle en $C_6$ à $C_{60}$,
qui est facultativement substitué par un ou plusieurs substituants $R^5$ ; et

hétéroaryle en $C_3$ à $C_{57}$,
qui est facultativement substitué par un ou plusieurs substituants $R^5$ ; et un système de cycle mono ou polycyclique, aliphatique, aromatique et/ou benzo-fusionné formé par fermeture de cycle avec un ou plusieurs autres substituants sélectionnés parmi le groupe consistant en $R^1$, $R^2$ et $R^5$.
$R^a$ est, à chaque occurrence indépendamment d'une autre, sélectionné parmi le groupe consistant en
hydrogène,
deutérium,
$N(R^5)_2$,
$OR^5$,
$Si(R^5)_3$,
$B(OR^5)_2$,
$OSO_2R^5$,
$CF_3$,
CN,
F,
Br,
I
alkyle en $C_1$ à $C_{40}$,
qui est facultativement substitué par un ou plusieurs substituants $R^5$ et
dans laquelle un ou plusieurs groupes $CH_2$ non adjacents sont facultativement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
alcoxy en $C_1$ à $C_{40}$,
qui est facultativement substitué par un ou plusieurs substituants $R^5$ et
dans laquelle un ou plusieurs groupes $CH_2$ non adjacents sont facultativement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
thioalcoxy en $C_1$ à $C_{40}$,
qui est facultativement substitué par un ou plusieurs substituants $R^5$ et
dans laquelle un ou plusieurs groupes $CH_2$ non adjacents sont facultativement substitués par $R^5C=CR^5$, C≡C, $Si(R^5)_2$, Ge $(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
alcényle en $C_2$ à $C_{40}$,
qui est facultativement substitué par un ou plusieurs substituants $R^5$ et
dans laquelle un ou plusieurs groupes CH2 non adjacents sont facultativement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
alcynyle en $C_2$ à $C_{40}$,
qui est facultativement substitué par un ou plusieurs substituants $R^5$ et
dans laquelle un ou plusieurs groupes $CH_2$ non adjacents sont facultativement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;
aryle en $C_6$ à $C_{60}$,
qui est facultativement substitué par un ou plusieurs substituants $R^5$ ; et
hétéroaryle en $C_3$ à $C_{57}$,
qui est facultativement substitué par un ou plusieurs substituants $R^5$ ;
$R^5$ est, à chaque occurrence indépendamment d'une autre, sélectionné parmi le groupe consistant en hydrogène, deutérium, $N(R^6)_2$, $OR^6$, $Si(R^6)_3$, $B(OR^6)_2$, $OSO_2R^6$, $CF_3$, CN, F, Br, I,
alkyle en $C_1$ à $C_{40}$,
qui est facultativement substitué par un ou plusieurs substituants $R^6$ et
dans laquelle un ou plusieurs groupes $CH_2$ non adjacents sont facultativement substitués par $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$ ;
alcoxy en $C_1$ à $C_{40}$,
qui est facultativement substitué par un ou plusieurs substituants $R^6$ et
dans laquelle un ou plusieurs groupes $CH_2$ non adjacents sont facultativement substitués par $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$ ;
thioalcoxy en $C_1$ à $C_{40}$,
qui est facultativement substitué par un ou plusieurs substituants $R^6$ et
dans laquelle un ou plusieurs groupes $CH_2$ non adjacents sont facultativement substitués par $R^6C=CR^6$, C≡C, $Si(R^6)_2$, Ge $(R^6)_2$, $Sn(R^6)_2$, C=O, C=S, C=Se, $C=NR^6$, $P(=O)(R^6)$, SO, $SO_2$, $NR^6$, O, S ou $CONR^6$ ;
alcényle en $C_2$ à $C_{40}$,
qui est facultativement substitué par un ou plusieurs substituants $R^6$ et
dans laquelle un ou plusieurs groupes $CH_2$ non adjacents sont facultativement substitués par $R^6C=CR^6$, C≡C,

$Si(R^6)_2$, Ge $(R^6)_2$, $Sn(R^6)_2$, C=O, C=S, C=Se, $C=NR^6$, $P(=O)(R^6)$, SO, $SO_2$, $NR^6$, O, S ou $CONR^6$ ;
alcynyle en $C_2$ à $C_{40}$,
qui est facultativement substitué par un ou plusieurs substituants $R^6$ et
dans laquelle un ou plusieurs groupes $CH_2$ non adjacents sont facultativement substitués par $R^6C=CR^6$, C≡C, $Si(R^6)_2$, Ge $(R^6)_2$, $Sn(R^6)_2$, C=O, C=S, C=Se, $C=NR^6$, $P(=O)(R^6)$, SO, $SO_2$, $NR^6$, O, S ou $CONR^6$ ;
aryle en $C_6$ à $C_{60}$,
qui est facultativement substitué par un ou plusieurs substituants $R^6$ ; et
hétéroaryle en $C_3$ à $C_{57}$,
qui est facultativement substitué par un ou plusieurs substituants $R^6$ ;
$R^6$ est, à chaque occurrence indépendamment d'une autre, sélectionné parmi le groupe consistant en hydrogène, deutérium, OPh, $CF_3$, CN, F,
alkyle en $C_1$ à $C_5$,
dans laquelle un ou plusieurs atomes d'hydrogène sont facultativement, indépendamment les uns des autres, substitués par deutérium, CN, $CF_3$ ou F ;
alcoxy en $C_1$ à $C_5$,
dans laquelle un ou plusieurs atomes d'hydrogène sont facultativement, indépendamment les uns des autres, substitués par deutérium, CN, $CF_3$ ou F ;
thioalcoxy en $C_1$ à $C_5$,
dans laquelle un ou plusieurs atomes d'hydrogène sont facultativement, indépendamment les uns des autres, substitués par deutérium, CN, $CF_3$ ou F ;
alcényle en $C_2$ à $C_5$,
dans laquelle un ou plusieurs atomes d'hydrogène sont facultativement, indépendamment les uns des autres, substitués par deutérium, CN, $CF_3$ ou F ;
alcynyle en $C_2$ à $C_5$,
dans laquelle un ou plusieurs atomes d'hydrogène sont facultativement, indépendamment les uns des autres, substitués par deutérium, CN, $CF_3$ ou F ;
aryle en $C_6$ à $C_{18}$,
qui est facultativement substitué par un ou plusieurs substituants alkyle en $C_1$ à $C_5$ ;
hétéroaryle en $C_3$ à $C_{17}$,
qui est facultativement substitué par un ou plusieurs substituants alkyle en $C_1$ à $C_5$ ;
N (aryle en $C_6$ à $C_{18})_2$ ;
N (hétéroaryle en $C_3$ à $C_{17})_2$,
et N(hétéroaryle en $C_3$ à $C_{17}$) (aryle en $C_6$ à $C_{18}$) ;
dans laquelle l'un quelconque des substituants $R^1$, $R^2$, $R^a$, ou $R^5$, indépendamment les uns des autres, forme facultativement un système de cycle mono ou polycyclique, aliphatique, aromatique et/ou benzo-fusionné avec un ou plusieurs autres substituants $R^1$, $R^2$, $R^a$, ou $R^5$.

**2.** Molécule organique selon la revendication 1, dans laquelle $R^1$ et $R^2$ sont indépendamment sélectionnés parmi le groupe consistant en

phényle, qui est facultativement substitué par un ou plusieurs substituants $R^5$ ; et
pyridine, qui est facultativement substituée par un ou plusieurs substituants $R^5$.

**3.** Molécule organique selon la revendication 1 ou 2, dans laquelle les substituants $R^2$ et $R^1$ sont identiques.

**4.** Molécule organique selon l'une ou plusieurs des revendications 1 à 3, dans laquelle la molécule organique est une structure sélectionnée parmi le groupe consistant en la formule IIIa, la formule IIIb, la formule IIIc, formule IIId, formule IIIe, formule IIIf, formule IIIg, formule IIIh, et formule IIIi :

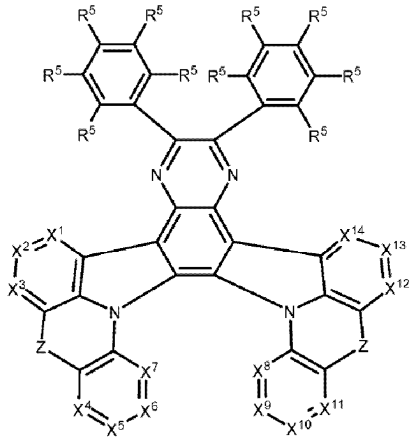

Formule IIIa

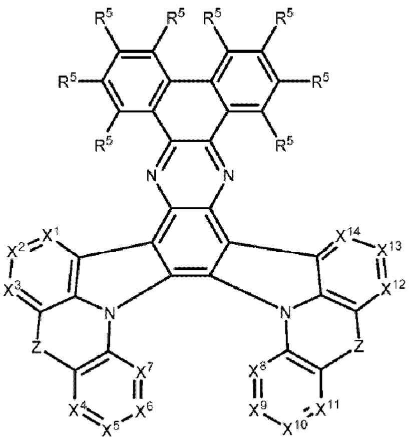

Formule IIIb

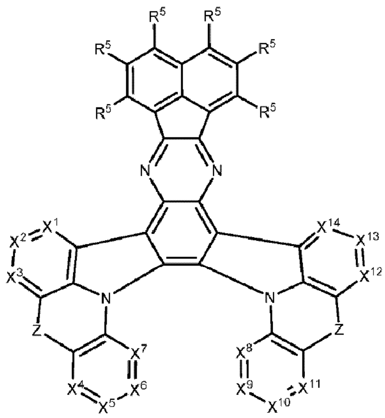

Formule IIIc

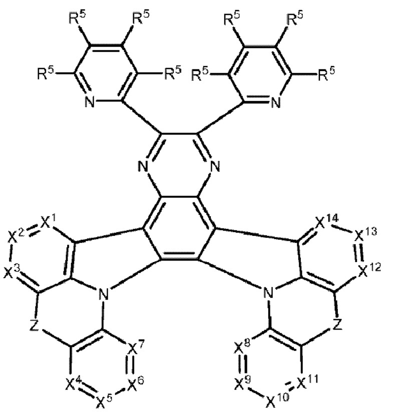

Formule IIId

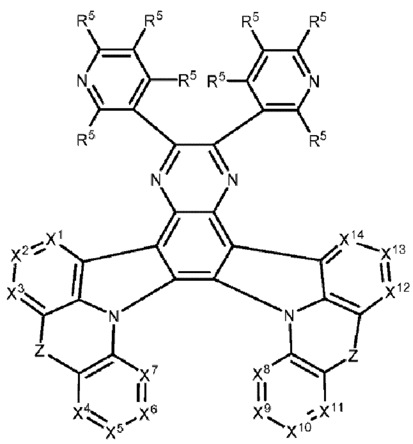

Formule IIIe

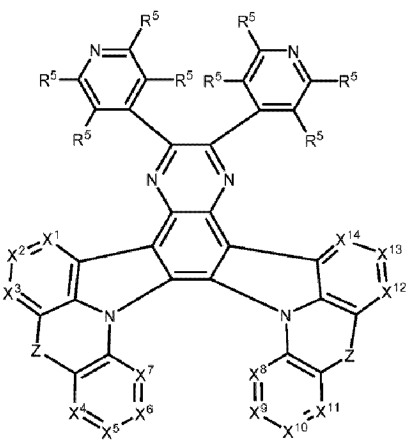

Formule IIIf

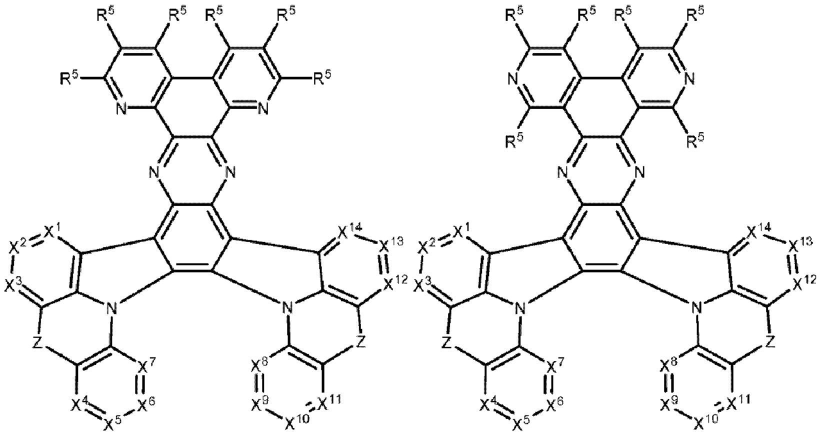

Formule IIIg

Formule IIIh

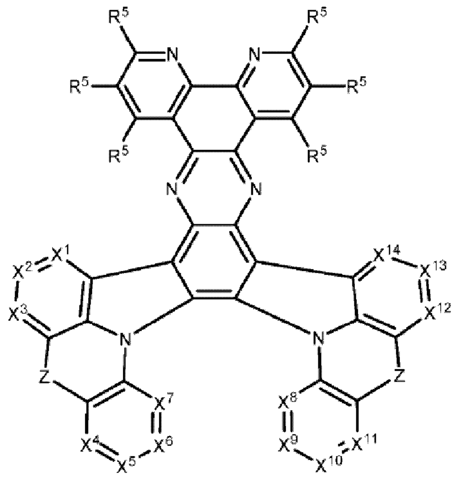

Formule IIIi

5. Molécule organique selon l'une quelconque des revendications 1 à 3, dans laquelle $R^a$ est, à chaque occurrence indépendamment d'une autre, sélectionné parmi le groupe consistant en :

hydrogène,
Me,
$^i$Pr,
$^t$Bu,
CN,
$CF_3$,
Ph, qui est facultativement substitué par un ou plusieurs substituants, indépendamment les uns des autres, sélectionnés parmi le groupe consistant en Me, $^i$Pr, $^t$Bu, CN, $CF_3$, et Ph,
pyridinyle, qui est facultativement substitué par un ou plusieurs substituants, indépendamment les uns des autres, sélectionnés parmi le groupe consistant en Me, $^i$Pr, $^t$Bu, CN, $CF_3$, et Ph,
pyrimidinyle, qui est facultativement substitué par un ou plusieurs substituants, indépendamment les uns des autres, sélectionnés parmi le groupe consistant en Me, $^i$Pr, $^t$Bu, CN, $CF_3$, et Ph,
carbazolyle, qui est facultativement substitué par un ou plusieurs substituants, indépendamment les uns des autres, sélectionnés parmi le groupe consistant en Me, $^i$Pr, $^t$Bu, CN, $CF_3$, et Ph,
triazinyle, qui est facultativement substitué par un ou plusieurs substituants, indépendamment les uns des autres, sélectionnés parmi le groupe consistant en Me, $^i$Pr, $^t$Bu, CN, $CF_3$ et Ph, et $N(Ph)_2$.

6. Molécule organique selon l'une quelconque des revendications 1 à 5 dans laquelle $R^5$ est, à chaque occurrence indépendamment d'une autre, sélectionné parmi le groupe consistant en :

hydrogène,
Me,
$^i$Pr,
$^t$Bu,
CN,
$CF_3$,
Ph, qui est facultativement substitué par un ou plusieurs substituants, indépendamment les uns des autres sélectionnés, parmi le groupe consistant en Me, $^i$Pr, $^t$Bu, CN, $CF_3$, et Ph ;
pyridinyle, qui est facultativement substitué par un ou plusieurs substituants, indépendamment les uns des autres, sélectionnés parmi le groupe consistant en Me, $^i$Pr, $^t$Bu, CN, $CF_3$, et Ph ;
pyrimidinyle, qui est facultativement substitué par un ou plusieurs substituants, indépendamment les uns des autres, sélectionnés parmi le groupe consistant en Me, $^i$Pr, $^t$Bu, CN, $CF_3$, et Ph ;
carbazolyle, qui est facultativement substitué par un ou plusieurs substituants, indépendamment les uns des autres, sélectionnés parmi le groupe consistant en Me, $^i$Pr, $^t$Bu, CN, $CF_3$, et Ph ;
triazinyle, qui est facultativement substitué par un ou plusieurs substituants, indépendamment les uns des autres, sélectionnés parmi le groupe consistant en Me, $^i$Pr, $^t$Bu, CN, $CF_3$ et Ph ; et $N(Ph)_2$.

**7.** Utilisation d'une molécule organique selon l'une quelconque des revendications 1 à 6 en tant qu'émetteur luminescent dans un dispositif optoélectronique.

**8.** Utilisation selon la revendication 7, dans laquelle le dispositif optoélectronique est sélectionné parmi le groupe consistant en :

• diodes électroluminescentes organiques (DELO),
• cellules électrochimiques électroluminescentes,
• capteurs DELO,
• diodes organiques,
• cellules solaires organiques,
• transistors organiques,
• transistors à effet de champ organiques,
• lasers organiques, et
• éléments de conversion descendante.

**9.** Composition, comportant :

(a) une molécule organique selon l'une quelconque des revendications 1 à 6, en particulier sous la forme d'un émetteur,
(b) un matériau hôte d'annihilation triplet-triplet (TTA), qui diffère de la molécule organique,
(c) facultativement, un matériau TADF, et
(c) facultativement, un colorant et/ou un solvant.

**10.** Dispositif optoélectronique, comportant une molécule organique selon l'une quelconque des revendications 1 à 6 ou une composition selon la revendication 11, en particulier sous la forme d'un dispositif sélectionné parmi le groupe consistant en une diode électroluminescente organique (DELO), une cellule électrochimique électroluminescente, un capteur DELO, une diode organique, une cellule solaire organique, un transistor organique, un transistor à effet de champ organique, un laser organique et un élément de conversion descendante.

**11.** Dispositif optoélectronique selon la revendication 10, comportant :

- un substrat,
- une anode, et
- une cathode, dans lequel l'anode ou la cathode est disposée sur le substrat, et
- une couche électroluminescente, qui est agencée entre l'anode et la cathode et qui comporte la molécule organique ou la composition.

**12.** Procédé de production d'un dispositif optoélectronique, dans lequel une molécule organique selon l'une quelconque des revendications 1 à 6 ou une composition selon la revendication 9 est utilisée.

**13.** Procédé selon la revendication 12, comportant le traitement de la molécule organique par un procédé d'évaporation sous vide ou à partir d'une solution.

Figure 1

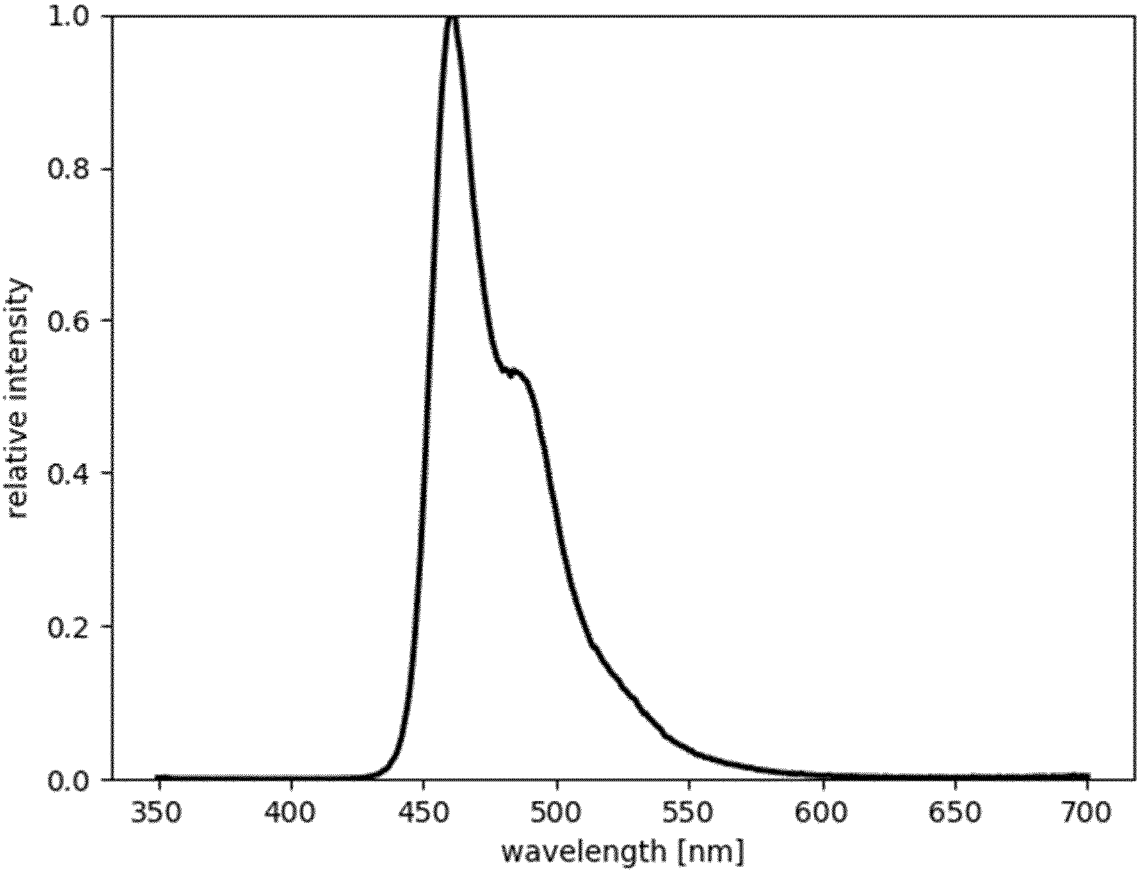
1.0
0.8
0.6
0.4
0.2
0.0
relative intensity
350
400
450
500
550
600
650
700
wavelength [nm]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 108727394 A
- KR 20180008283 A